# EUROPEAN PATENT APPLICATION

(11) **EP 4 501 317 A1**
(43) Date of publication of application: **05.02.2025**
(21) Application number: 23780180.8
(22) Date of filing: 24.03.2023
(51) Int. Cl.: A61K 9/127, A61K 9/51, A61K 31/7088, A61K 47/10, A61K 47/14, A61K 47/20, A61K 47/24, A61K 47/28, A61K 48/00, A61P 43/00

(54) **METHOD FOR PRODUCING NUCLEIC ACID-ENCAPSULATED LIPID NANOPARTICLES, METHOD FOR PRODUCING PHARMACEUTICAL COMPOSITION CONTAINING SAID LIPID NANOPARTICLES, AND METHOD FOR INTRODUCING NUCLEIC ACID INTO CELL OR TARGET CELL**

(30) Priority: 28.03.2022 JP 2022051913
(71) Applicant: NOF Corporation, Shibuya-ku Tokyo 150-6019 (JP); National University Corporation Chiba University, Chiba-shi, Chiba 263-8522 (JP)
(72) Inventor: TANGE, Kota, Kawasaki-shi, Kanagawa 210-0865 (JP); NAKAI, Yuta, Kawasaki-shi, Kanagawa 210-0865 (JP); AKITA, Hidetaka, Chiba-shi, Chiba 260-8675 (JP); TANAKA, Hiroki, Chiba-shi, Chiba 260-8675 (JP); SAKURAI, Yu, Chiba-shi, Chiba 260-8675 (JP); YAMAKAWA, Takuma, Chiba-shi, Chiba 260-8675 (JP); SATO, Yuka, Chiba-shi, Chiba 260-8675 (JP)
(74) Representative: Weickmann & Weickmann PartmbB
(86) International application number: PCT/JP2023/011843
(87) International publication number: WO 2023/190170

(57) **Abstract**

The present invention provides a method for producing nucleic acid-encapsulated lipid nanoparticles, which can encapsulate any nucleic acid with high efficiency and with ease. A method for producing nucleic acid-encapsulated lipid nanoparticles, including the following steps:
a) a step of preparing a suspension of lipid nanoparticles not containing a nucleic acid, by mixing an alcohol solution containing ionic lipid, sterol and PEG lipid with an acidic buffer having a buffering action at pH 1 to 6.5, and
b) a step of mixing, without lyophilization, the suspension of the lipid nanoparticles obtained in step a with an aqueous solution containing a nucleic acid and optionally containing 0 to 25 v/v% alcohol, and optionally incubating the mixture at 0 to 95°C for 0 to 60 min to obtain nucleic acid-encapsulated lipid nanoparticles.

## Description

### [Technical Field]

The present invention relates to a method for producing nucleic acid-encapsulated lipid nanoparticles, including a step of preparing lipid nanoparticles not containing a nucleic acid and thereafter adding a nucleic acid, a method for producing a pharmaceutical composition including same, and a method for introducing the nucleic acid into a cell or target cell.

### [Background Art]

For practicalization of nucleic acid therapy using oligonucleic acids such as siRNA, and gene therapy using mRNA, pDNA, and the like, an effective and safe nucleic acid delivery carrier is demanded. While virus vectors are nucleic acid delivery carriers with good expression efficiency, the development of non-viral nucleic acid delivery carriers that can be used more safely is ongoing.

Since cationic liposomes using cationic lipids with quaternary amine are positively charged, they can form a complex (lipoplex) by electrostatic interaction with negatively-charged nucleic acids, and can deliver nucleic acids into cells. Utilizing the electrostatic interaction of quaternary amine with nucleic acid, it is also possible to prepare a lyophilized composition of cationic liposomes not containing nucleic acids and form a lipoplex by rehydration with an aqueous solution of nucleic acid. Thus, it has been shown that use thereof as a gene transfer reagent is available (see, for example, Patent Literatures 1 and 2).

However, it is difficult to control the particle size of lipoplex produced by such method, and cytotoxicity derived from positively-charged cationic lipids becomes a problem.

Therefore, lipid nanoparticles (LNP) using ionic lipids having a tertiary amine -which is positively charged under acidic conditions and has no electric charge under near neutral conditions- in the molecule were developed, and have become non-viral nucleic acid delivery carriers most generally used at present (see, for example, Non Patent Literature 1).

As lipid nanoparticles using an ionic lipid having a tertiary amine in the molecule, an example also exists in which a degradable group is added to the ionic lipid (see, for example, Patent Literature 3).

As described, various non-viral carriers have been developed. However, since nucleic acids are generally unstable compounds, there are still problems with their stability as pharmaceutical preparations.

### [Citation List]

### [Patent Literature]

[Patent Literature 1]
   JP 4919397 B
[Patent Literature 2]
   JP 4598908 B
[Patent Literature 3]
   JP 6093710 B
[Patent Literature 4]
   WO 2017/218704
[Patent Literature 5]
   WO 2021/060440

### [Non Patent Literature]

[Non Patent Literature 1]
   Gene Therapy 6:271-281, 1999
[Non Patent Literature 2]
   Biol. Pharm. Bull. 41, 1291-1294(2018)

### [Summary of Invention]

### [Technical Problem]

As one of the methods for improving the stability as a pharmaceutical preparation, attempts have been made to lyophilize lipid nanoparticles encapsulating nucleic acid and rehydrate them at the time of use to reconstitute the lipid nanoparticles (Patent Literature 4 and Non Patent Literature 2) .

Although these methods are useful as a method for enhancing the storage stability of lipid nanoparticles encapsulating a specific nucleic acid, there is a problem as a method for more easily encapsulating any nucleic acid in lipid nanoparticles.

As a method for more easily encapsulating any nucleic acid in lipid nanoparticles, a method of preparing a lyophilized composition not containing a nucleic acid and then rehydrating same with an aqueous solution of nucleic acid, like the methods described in Patent Literatures 1 and 2, can be mentioned.

However, lipid nanoparticles obtained using ionic lipid having tertiary amine in the molecule do not electrostatically interact with nucleic acid because the surface charge after preparation is weakly negative to neutral. Thus, the method of preparing a lyophilized composition not containing a nucleic acid and then rehydrating same with an aqueous solution of nucleic acid, disclosed in Patent Literatures 1 and 2, cannot prepare nucleic acid-encapsulated lipid nanoparticles.

In this connection, Patent Literature 5 shows that any nucleic acid can be encapsulated in lipid nanoparticles with high efficiency and ease by preparing lipid nanoparticles not containing a nucleic acid in an acidic buffer, adding a cryoprotectant, lyophilizing the nanoparticles, and then rehydrating the nanoparticles with an aqueous solution containing the nucleic acid.

However, the above-mentioned technique requires a lyophilization step, and there is room for improvement as regards simplicity.

In view of the above-mentioned problems, the present invention aims to provide a method for producing nucleic acid-encapsulated lipid nanoparticles, which can encapsulate any nucleic acid with high efficiency and with ease and which could not be achieved by the prior art, a method for producing a pharmaceutical composition including same, and a method for introducing a nucleic acid into a cell or target cell.

### [Solution to Problem]

The present inventors have conducted intensive studies in view of the above-mentioned problems and found that any nucleic acid can be encapsulated with high efficiency and easily by preparing lipid nanoparticles not containing nucleic acid in an acidic buffer and adding an aqueous solution containing nucleic acid. Furthermore, they conducted experiments to transfer gene into cells by using the lipid nanoparticles prepared by this method, and unexpectedly found that the gene transfer efficiency is improved compared to conventional techniques, which resulted in the completion of the present invention.

Accordingly, the present invention encompasses the following.
[1] A method for producing nucleic acid-encapsulated lipid nanoparticles, comprising the following steps:
   a) a step of preparing a suspension of lipid nanoparticles not containing a nucleic acid, by mixing an alcohol solution comprising ionic lipid, sterol and PEG lipid with an acidic buffer having a buffering action at pH 1 to 6.5, and
   b) a step of mixing, without lyophilization, the suspension of the lipid nanoparticles obtained in step a with an aqueous solution comprising a nucleic acid and optionally containing 0 to 25 v/v% alcohol, and optionally incubating the mixture at 0 to 95°C for 0 to 60 min to obtain nucleic acid-encapsulated lipid nanoparticles.
[2] The production method of [1], comprising the following step c after step b:
   c) a step of exchanging an external aqueous phase of the obtained nucleic acid-encapsulated lipid nanoparticles with a neutral buffer by dialysis, ultrafiltration or dilution.
[3] The production method of [1] or [2], wherein step a further comprises a step of freezing the lipid nanoparticles not containing a nucleic acid at -80 to 0°C and then thawing the lipid nanoparticles at 0 to 95°C.
[4] The production method of any one of [1] to [3], wherein step a further comprises, after preparation of the suspension of the lipid nanoparticles, exchanging the external aqueous phase with another acidic buffer having a buffering action at pH 1 to 6.5 by dialysis, ultrafiltration or dilution.
[5] The production method of any one of [1] to [4], wherein the alcohol solution further comprises phospholipid in step a.
[6] The production method of any one of [1] to [5], wherein the ionic lipid is a compound represented by the formula (1): 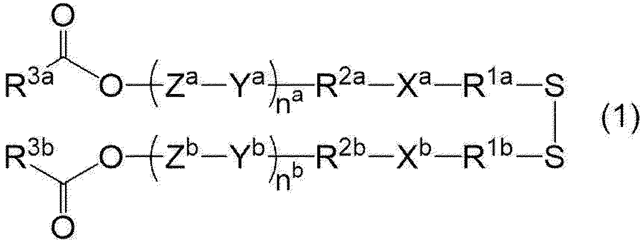 (in the formula (1),
   R^{1a} and R^{1b} are each independently an alkylene group having 1 - 6 carbon atoms,
   X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
   R^{2a} and R^{2b} are each independently an alkylene group or an oxydialkylene group each having not more than 8 carbon atoms,
   Y^{a} and Y^{b} are each independently an ester bond, an amide bond, a carbamate bond, an ether bond or a urea bond,
   Z^{a} and Z^{b} are each independently a divalent group derived from an aromatic compound having 3 - 16 carbon atoms and at least one aromatic ring, and optionally having a hetero atom,
   n^{a} and n^{b} are each independently 0 or 1, and
   R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (3):

      R⁹-O-CO-(CH₂) a- (3)
   (in the formula (3),
   R⁹ is an aliphatic hydrocarbon group having 2 - 20 carbon atoms, and a is an integer of 2 to 10)).
[7] The production method of any one of [1] to [5], wherein the ionic lipid is a compound represented by the formula (2): 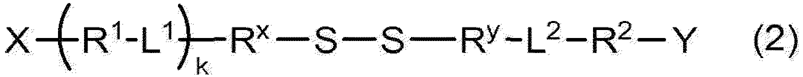 wherein
   X is a nitrogen-containing aliphatic group containing one or more tertiary nitrogens,
   R¹ is an aliphatic hydrocarbon group having not more than 8 carbon atoms,
   L¹ is an ester bond, an amide bond, a carbamate bond, an N-alkylcarbamate bond, a carbonate bond or a urea bond,
   k is 0 or 1,
   R^{x} and R^{y} are each independently an alkylene group having 2 - 5 carbon atoms,
   L² is an ester bond, an amide bond, a carbamate bond, a carbonate bond, an ether bond or a urea bond,
   R² is an alkylene group having not more than 8 carbon atoms, or absent, and
   Y is a group which (i) contains one or more divalent groups derived from an aromatic compound optionally having a hetero atom, (ii) contains a group containing at least one selected from the group consisting of an ester bond and a carbonate bond on the aromatic ring of the aforementioned divalent group, and (iii) contains at least one selected from the group consisting of an aliphatic hydrocarbon group having 10 - 37 carbon atoms, a liposoluble vitamin residue, and a residue of a sterol derivative.
[8] A method for introducing a nucleic acid into a cell, comprising a step of contacting nucleic acid-encapsulated lipid nanoparticles produced by the method of any one of [1] to [7] with the cell in vitro.
[9] A method for introducing a nucleic acid into a target cell, comprising a step of administering nucleic acid-encapsulated lipid nanoparticles produced by the method of any one of [1] to [7] to a living organism.
[10] A method for producing a pharmaceutical composition, comprising the method of any one of [1] to [7].

### [Advantageous Effects of Invention]

In the method for producing nucleic acid-encapsulated lipid nanoparticles of the present invention, lipid nanoparticles not containing nucleic acids are prepared, and then an aqueous solution of nucleic acid is added without lyophilization, and therefore, lipid nanoparticles encapsulating any nucleic acid can be produced with high efficiency and ease. Furthermore, the nucleic acid-encapsulated lipid nanoparticles prepared using the production method of the present invention have a higher nucleic acid transfer efficiency than conventional techniques, are advantageous for gene transfer in cells and living organism, and are particularly useful as pharmaceutical compositions.

### [Brief Description of Drawings]

[Fig. 1]

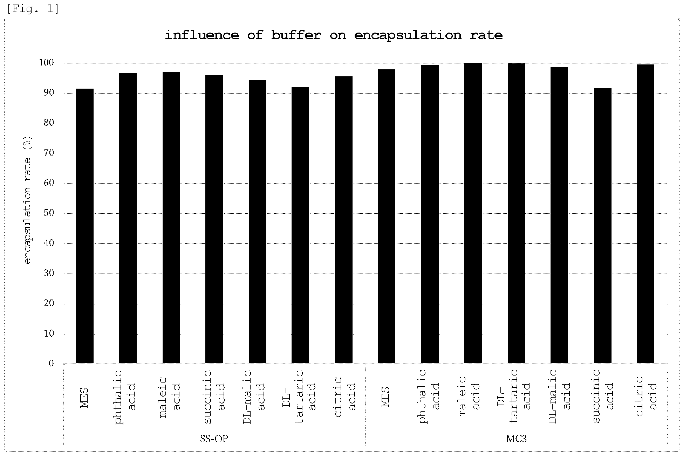

   Fig. 1 is a graph evaluating the influence of a buffer on the encapsulation rate.
[Fig. 2]

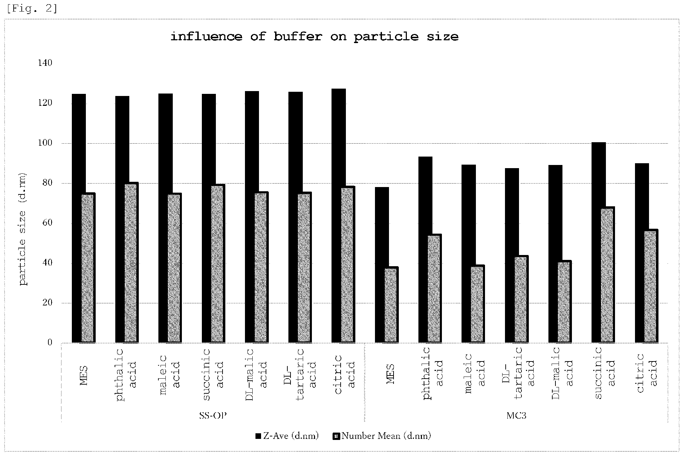

   Fig. 2 is a graph evaluating the influence of a buffer on the particle size.
[Fig. 3]

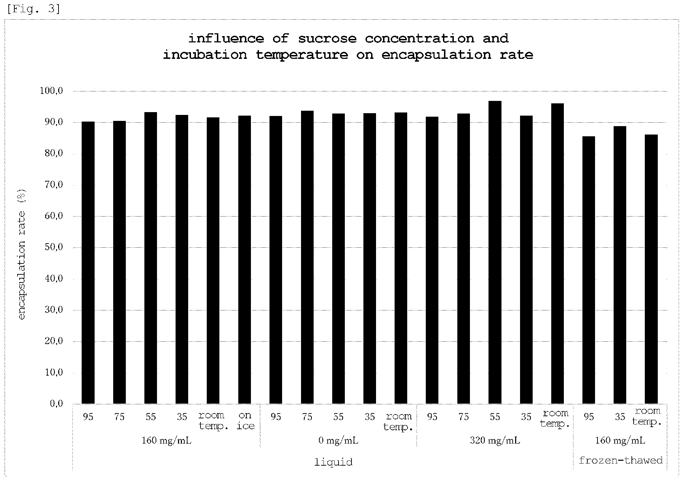

   Fig. 3 is a graph evaluating the influence of sucrose concentration and incubation temperature on the encapsulation rate. The incubation temperature is in °C.
[Fig. 4]

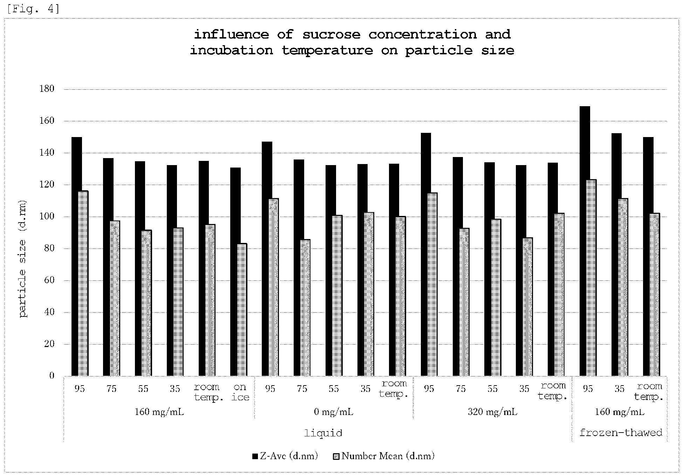

   Fig. 4 is a graph evaluating the influence of sucrose concentration and incubation temperature on the particle size. The incubation temperature is in °C.
[Fig. 5]

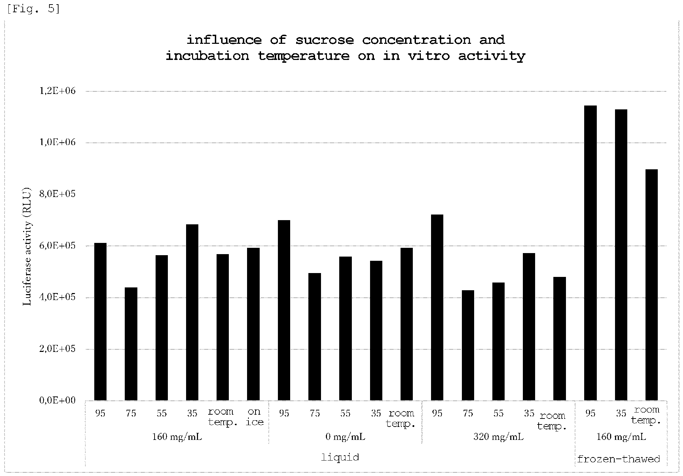

   Fig. 5 is a graph evaluating the influence of sucrose concentration and incubation temperature on the in vitro activity. The incubation temperature is in °C.
[Fig. 6]

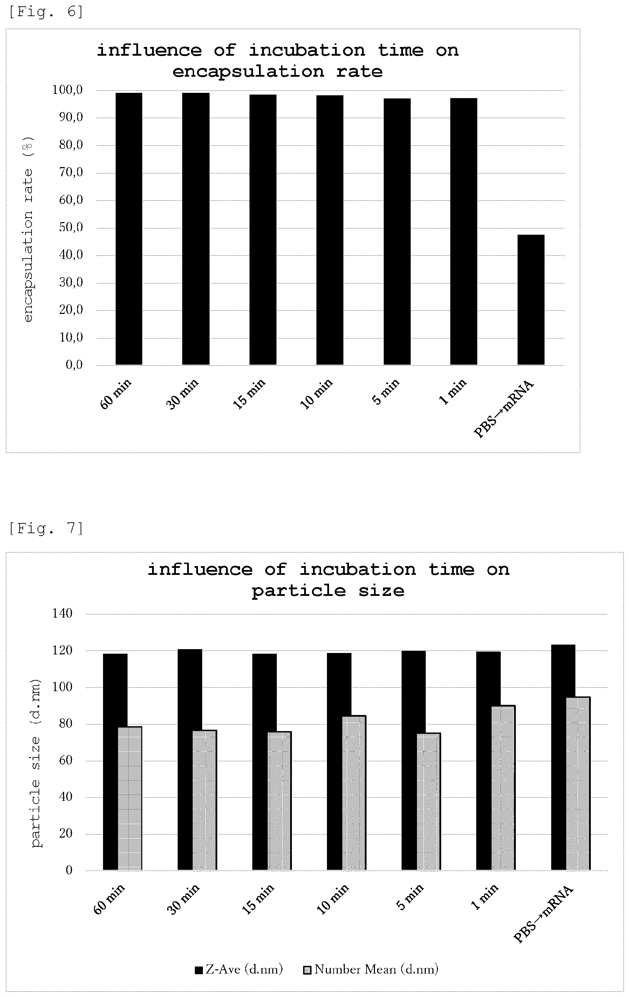

   Fig. 6 is a graph evaluating the influence of incubation time on the encapsulation rate.
[Fig. 7]
   Fig. 7 is a graph evaluating the influence of incubation time on the particle size.
[Fig. 8]

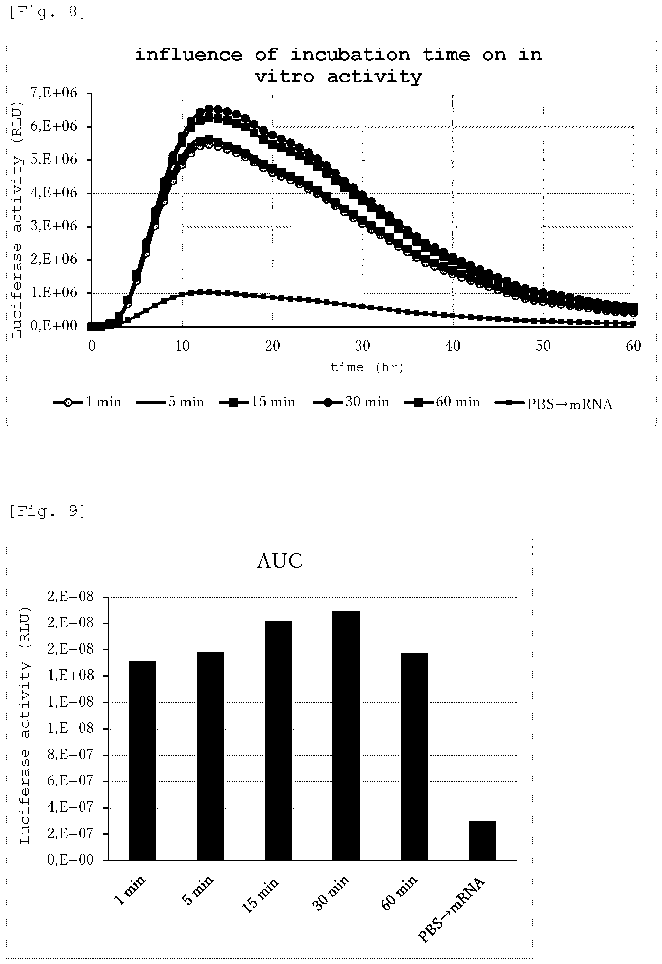

   Fig. 8 is a graph evaluating the influence of incubation time on the in vitro activity.
[Fig. 9]
   Fig. 9 is a graph showing the area under the curve (AUC) of Fig. 8.
[Fig. 10]

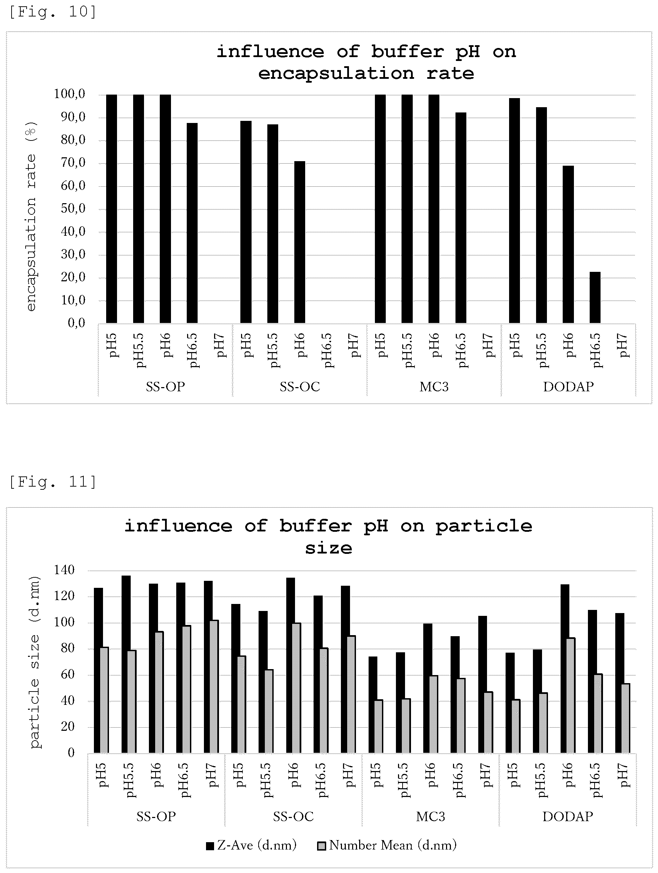

   Fig. 10 is a graph evaluating the influence of the pH of buffer on the encapsulation rate.
[Fig. 11]
   Fig. 11 is a graph evaluating the influence of the pH of buffer on the particle size.
[Fig. 12]

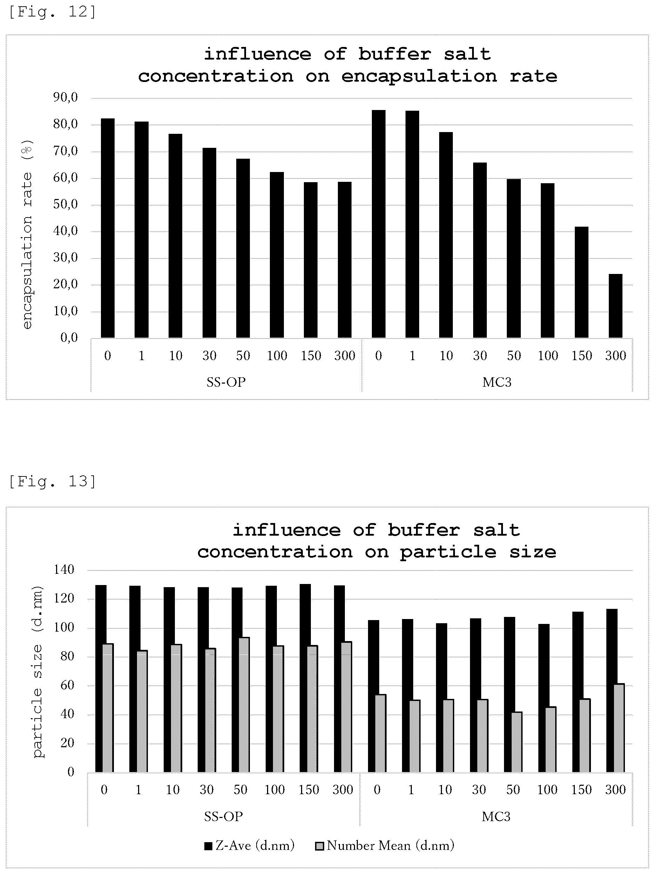

   Fig. 12 is a graph evaluating the influence of the salt concentration of buffer on the encapsulation rate.
[Fig. 13]
   Fig. 13 is a graph evaluating the influence of the salt concentration of buffer on the particle size.
[Fig. 14]

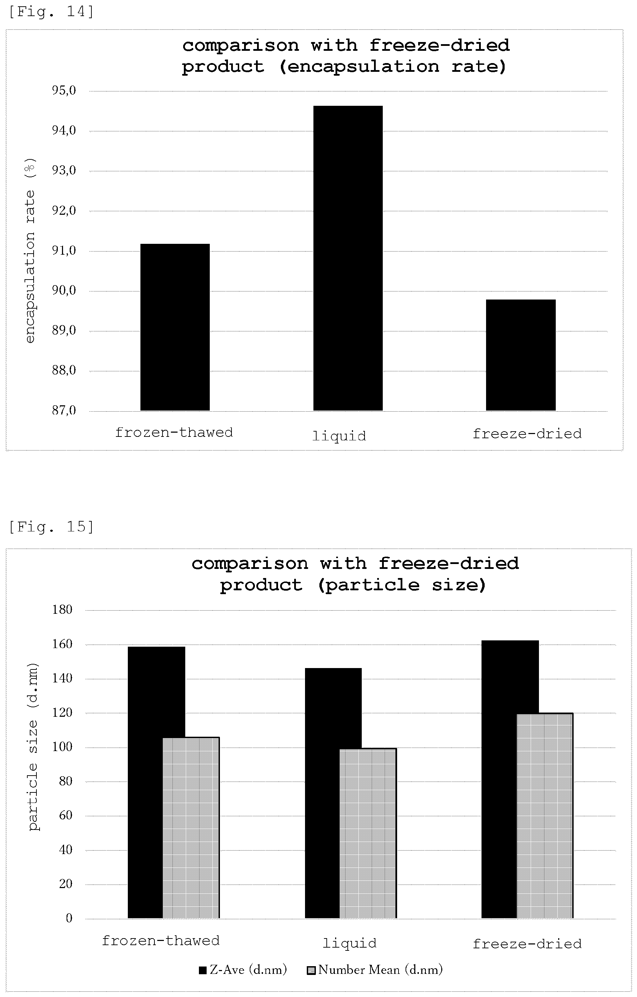

   Fig. 14 is a graph comparing the encapsulation rate between the nucleic acid-encapsulated nanoparticles of the present invention and a freeze-dried product.
[Fig. 15]
   Fig. 15 is a graph comparing the particle size between the nucleic acid-encapsulated nanoparticles of the present invention and a freeze-dried product.
[Fig. 16]

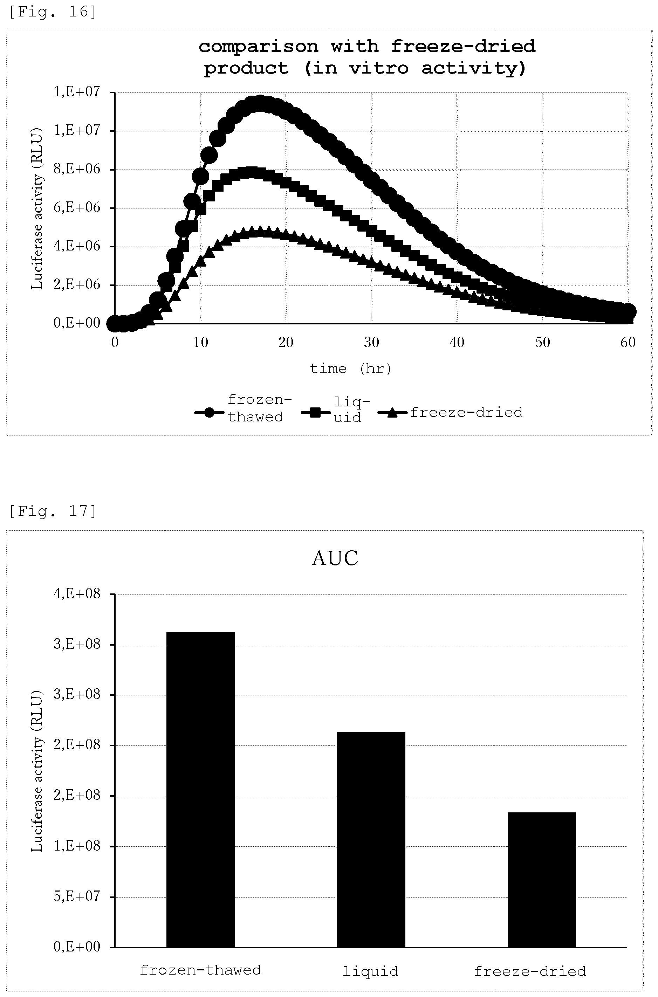

   Fig. 16 is a graph comparing the in vitro activity between the nucleic acid-encapsulated nanoparticles of the present invention and a freeze-dried product.
[Fig. 17]
   Fig. 17 is a graph showing the area under the curve (AUC) of Fig. 16.
[Fig. 18]

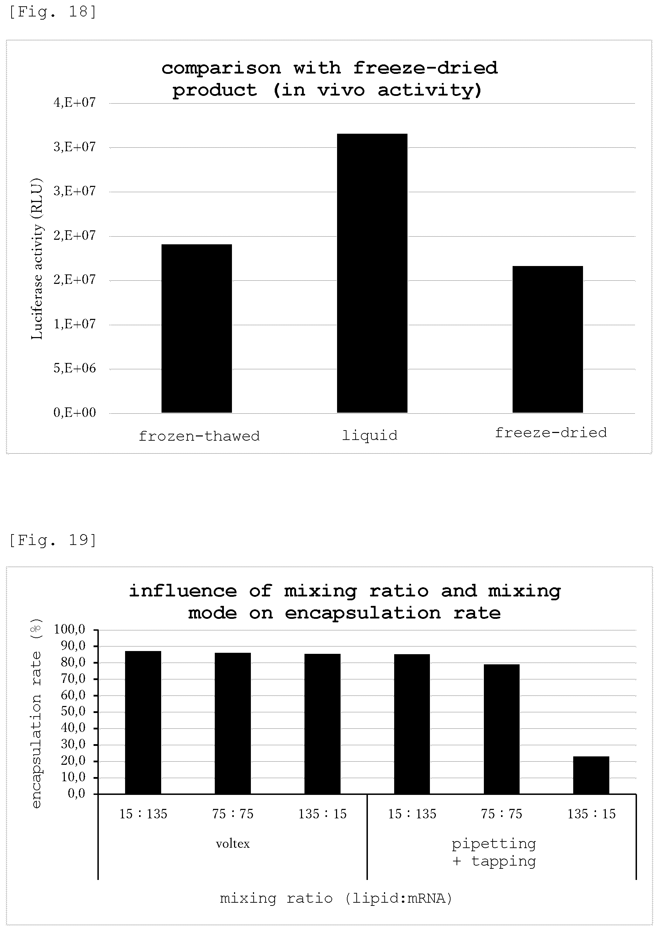

   Fig. 18 is a graph comparing the in vivo activity between the nucleic acid-encapsulated nanoparticles of the present invention and a freeze-dried product.
[Fig. 19]
   Fig. 19 is a graph evaluating the influence of the mixing ratio and mixing mode on the encapsulation rate.
[Fig. 20]

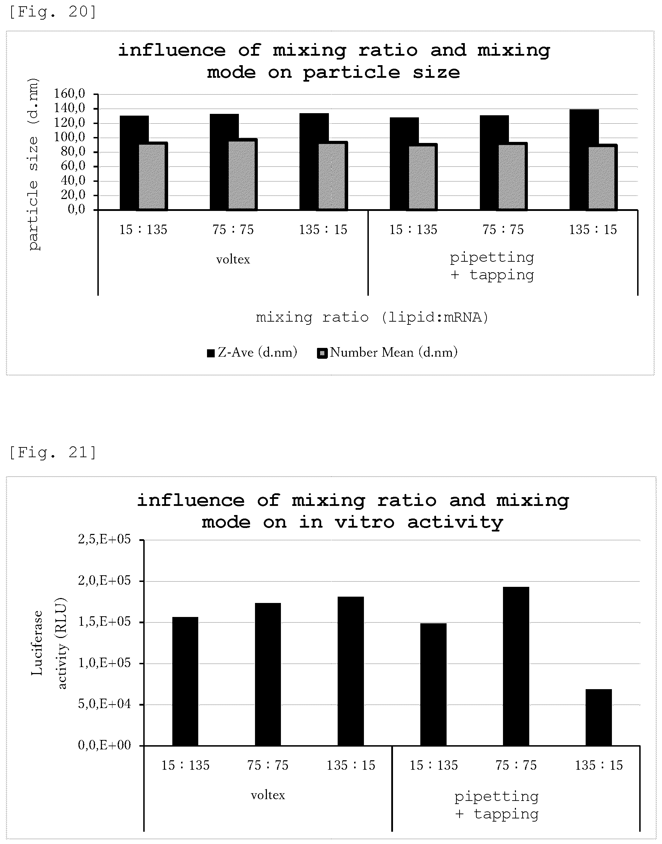

   Fig. 20 is a graph evaluating the influence of the mixing ratio and mixing mode on the particle size.
[Fig. 21]
   Fig. 20 is a graph evaluating the influence of the mixing ratio and mixing mode on the in vitro activity.
[Fig. 22]

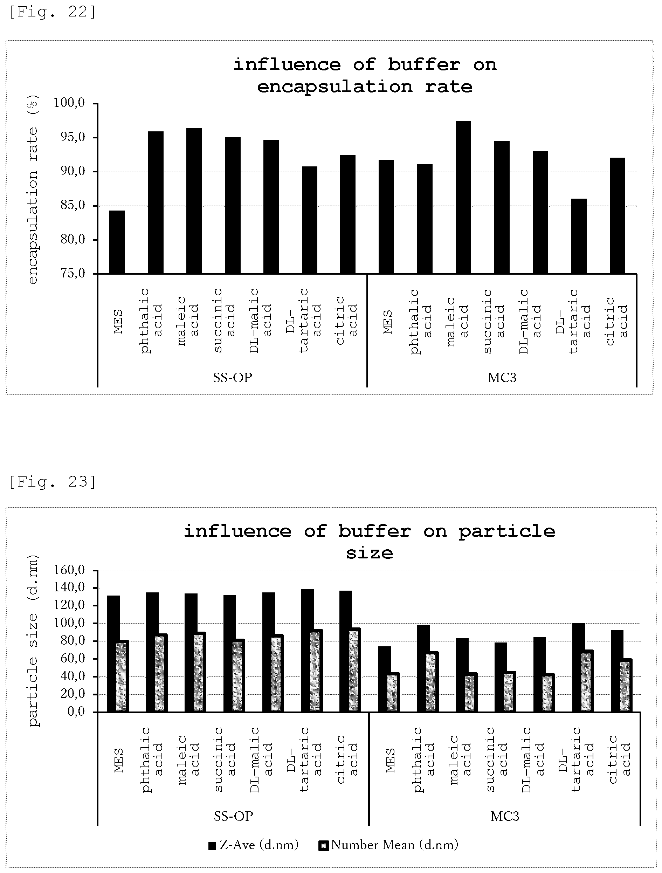

   Fig. 22 is a graph evaluating the influence of a buffer on the encapsulation rate.
[Fig. 23]
   Fig. 23 is a graph evaluating the influence of a buffer on the particle size.
[Fig. 24]

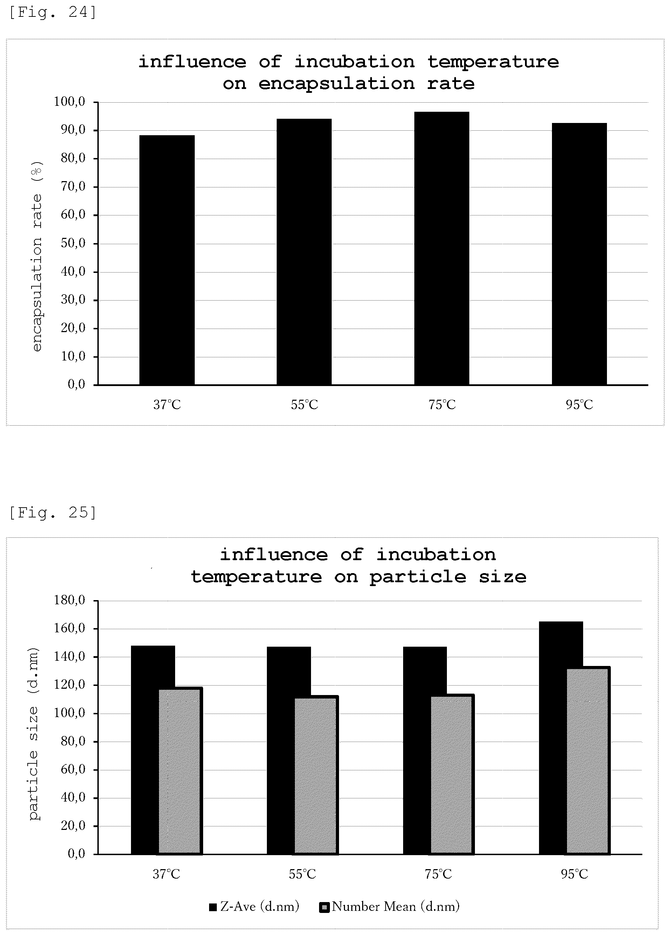

   Fig. 24 is a graph evaluating the influence of incubation temperature on the encapsulation rate.
[Fig. 25]
   Fig. 25 is a graph evaluating the influence of incubation temperature on the in vitro activity.
[Fig. 26]

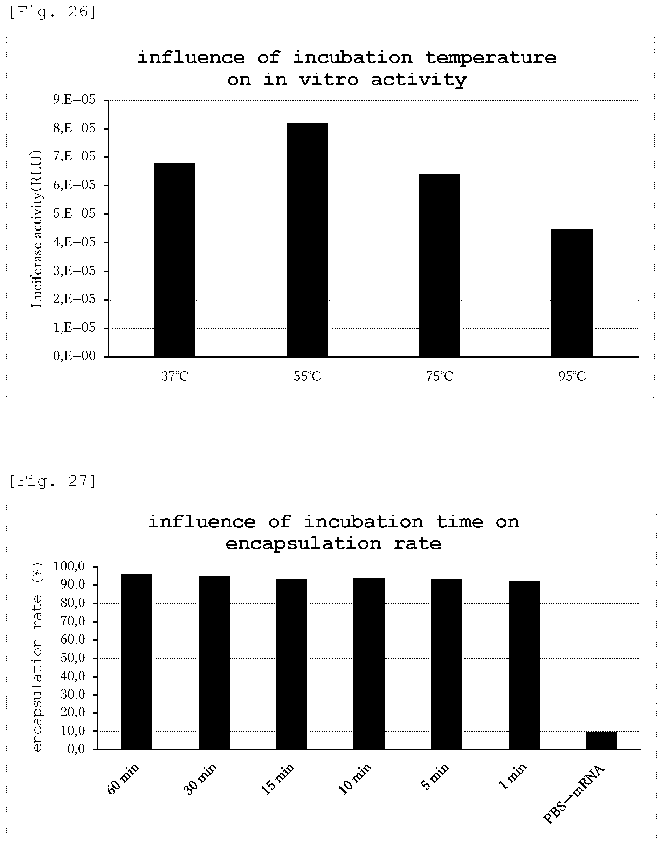

   Fig. 26 is a graph evaluating the influence of incubation temperature on the in vitro activity.
[Fig. 27]
   Fig. 27 is a graph evaluating the influence of incubation time on the encapsulation rate.
[Fig. 28]

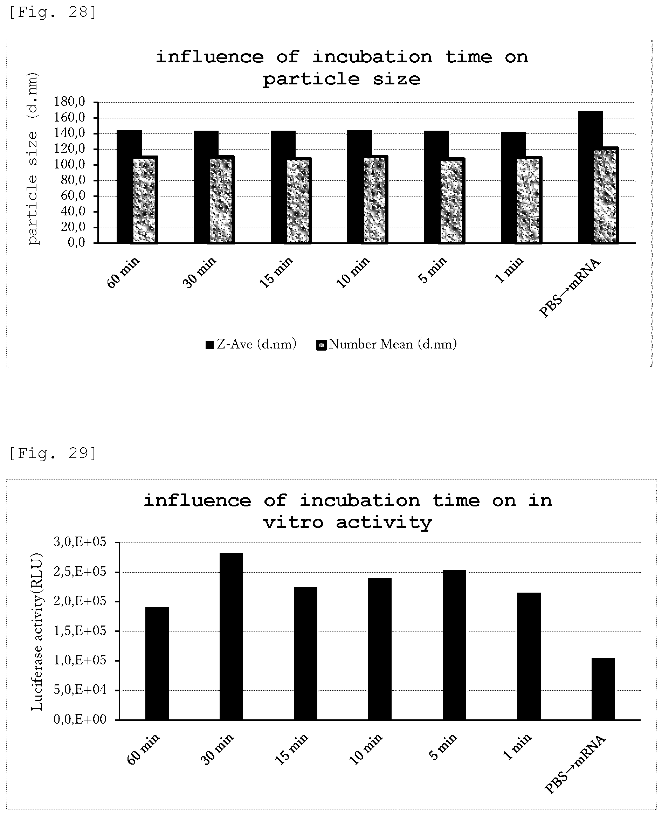

   Fig. 28 is a graph evaluating the influence of incubation time on the particle size.
[Fig. 29]
   Fig. 29 is a graph evaluating the influence of incubation time on the in vitro activity.
[Fig. 30]

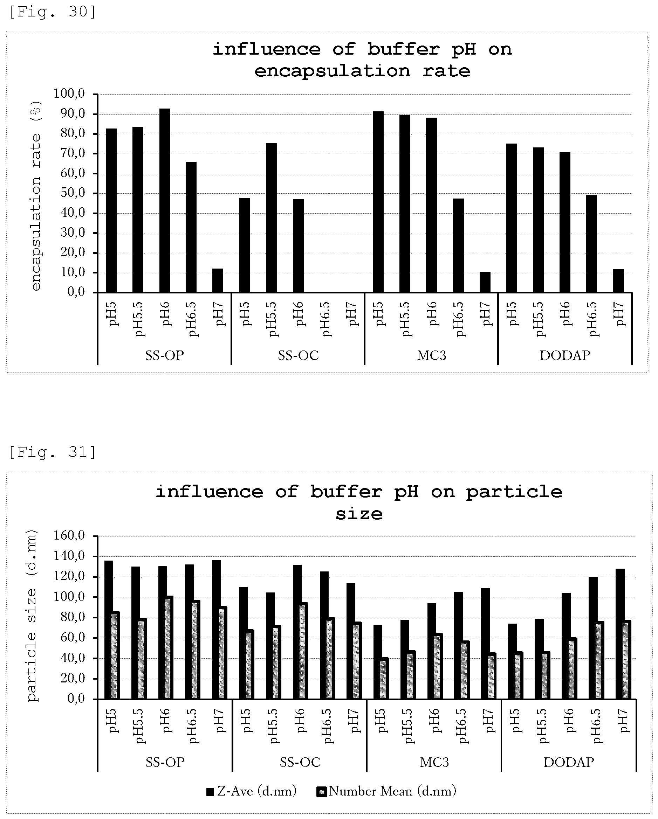

   Fig. 30 is a graph evaluating the influence of the pH of buffer on the encapsulation rate.
[Fig. 31]
   Fig. 31 is a graph evaluating the influence of the pH of buffer on the particle size.
[Fig. 32]

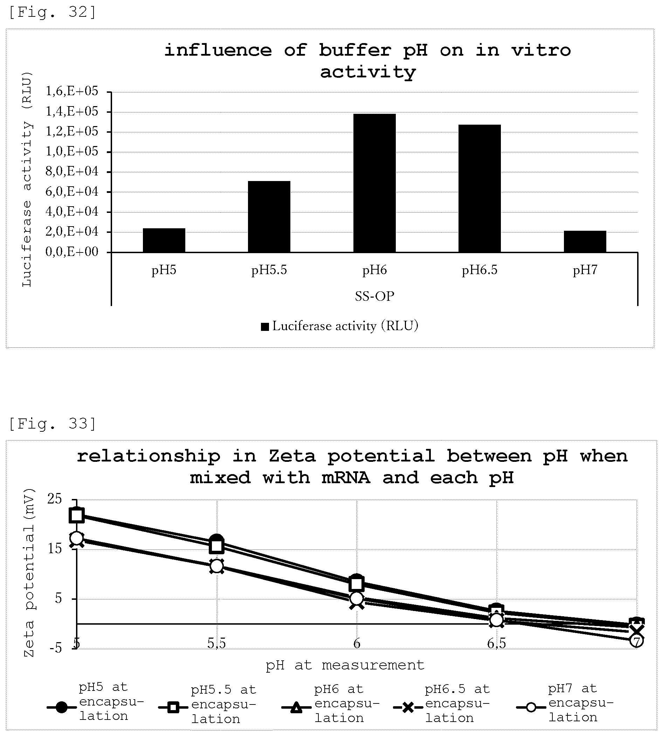

   Fig. 32 is a graph evaluating the influence of the pH of buffer on the in vitro activity.
[Fig. 33]
   Fig. 33 is a graph showing the relationship in the Zeta potential between the pH when mixed with mRNA and each pH.
[Fig. 34]

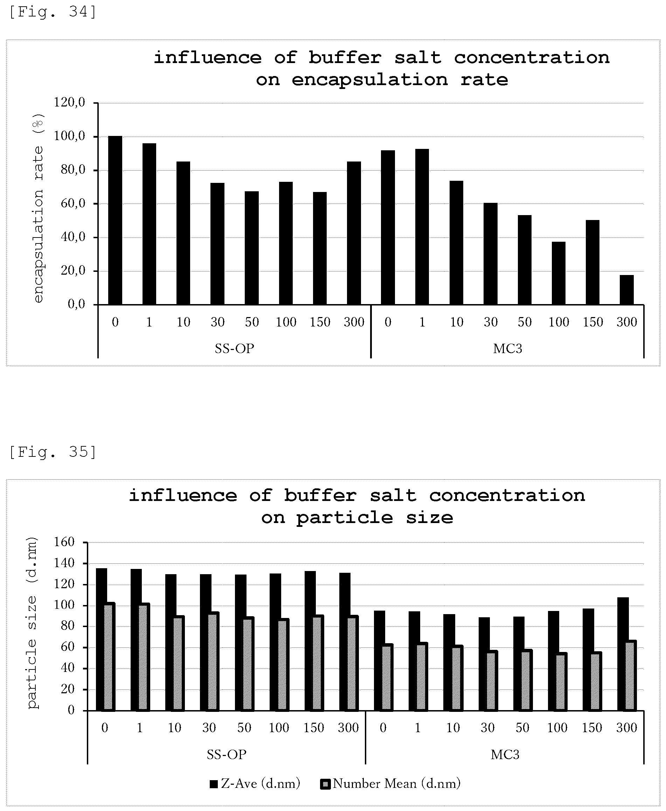

   Fig. 34 is a graph evaluating the influence of the salt concentration of buffer on the encapsulation rate.
[Fig. 35]
   Fig. 35 is a graph evaluating the influence of the salt concentration of buffer on the particle size.
[Fig. 36]

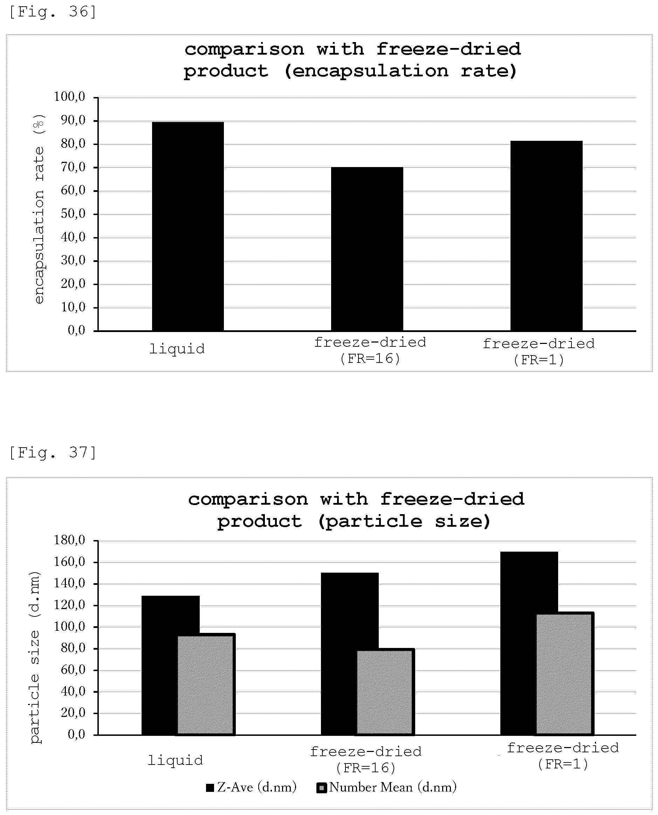

   Fig. 36 is a graph comparing the encapsulation rate between the nucleic acid-encapsulated nanoparticles of the present invention and a freeze-dried product.
[Fig. 37]
   Fig. 37 is a graph comparing the particle size between the nucleic acid-encapsulated nanoparticles of the present invention and a freeze-dried product.
[Fig. 38]

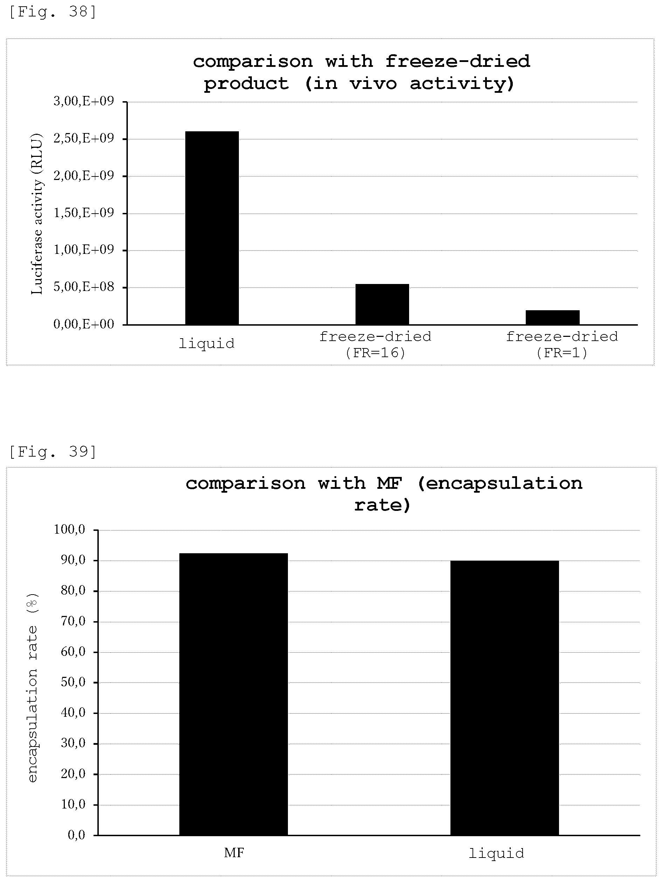

   Fig. 38 is a graph comparing the in vivo activity between the nucleic acid-encapsulated nanoparticles of the present invention and a freeze-dried product.
[Fig. 39]
   Fig. 39 is a graph comparing the encapsulation rate of nucleic acid-encapsulated nanoparticles of the present invention and nucleic acid-encapsulated nanoparticles (MF) produced using a microfluidic channel.
[Fig. 40]

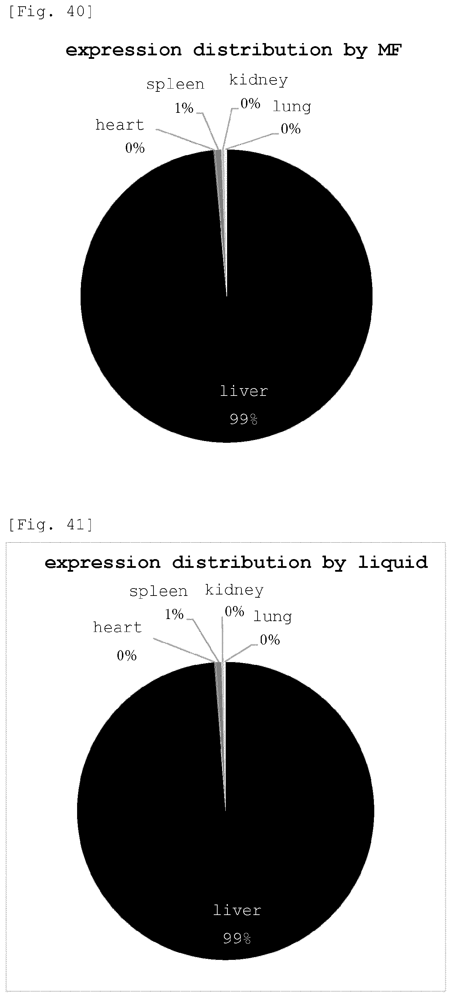

   Fig. 40 is a graph showing the expression distribution by nucleic acid-encapsulated nanoparticles (MF) produced using a microfluidic channel.
[Fig. 41]
   Fig. 41 is a graph showing the expression distribution by nucleic acid-encapsulated nanoparticles of the present invention.
[Fig. 42]

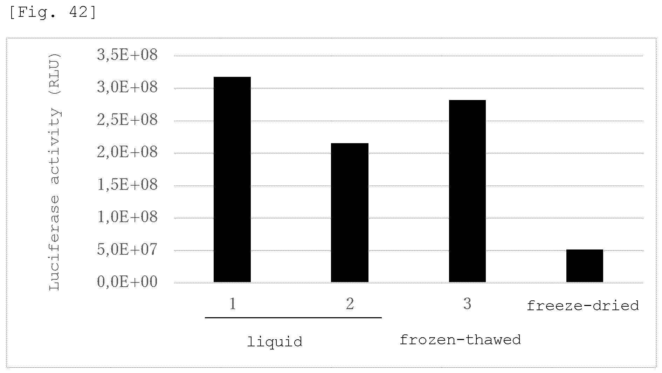

   Fig. 42 is a graph comparing the in vitro activity between the nucleic acid-encapsulated nanoparticles of the present invention and a freeze-dried product.

### [Description of Embodiments]

While the embodiments of the present invention are explained in the following, the present invention is not limited thereto.

The present invention relates to a method for producing nucleic acid-encapsulated lipid nanoparticles by mixing a lipid solution containing ionic lipid, sterol and PEG lipid, but not containing nucleic acid, with an acidic buffer having a buffering action at pH 1 to 6.5 to prepare lipid nanoparticles not containing a nucleic acid, and then adding an aqueous solution of nucleic acid.

A lipid nanoparticle means a particle having a membrane structure wherein the hydrophilic groups of amphiphilic lipid are arranged in the interface, facing the aqueous phase side. The "amphiphilic lipid" means a lipid having both a hydrophilic group showing hydrophilicity, and a hydrophobic group showing hydrophobicity. Examples of the amphiphilic lipid include ionic lipid, phospholipid, PEG lipid, and the like.

The lipid nanoparticles used in the present invention contain ionic lipid, a sterol, and PEG lipid as substances constituting a membrane, and may further contain a phospholipid. The particle size of the lipid nanoparticles is not particularly limited, and is preferably 10 nm to 500 nm, more preferably 30 nm to 300 nm. The particle size can be measured by using a particle size distribution measuring device such as Zetasizer Nano (Malvern) or the like. The particle size of the lipid nanoparticles can be appropriately adjusted by the method for producing the lipid nanoparticles. In the present invention, the particle size means an average particle size (number average) measured by a dynamic light scattering method.

In the present invention, the "total lipid" means the total amount of lipid. Examples of the lipid include ionic lipids, sterols, PEG lipids, and phospholipids.

In the present invention, "not containing nucleic acid" or "nucleic acid-free" means that nucleic acid is substantially not contained, and that the nucleic acid content is below the detection limit.

In the present invention, the "nucleic acid-encapsulated lipid nanoparticle" means a lipid nanoparticle in which a nucleic acid is encapsulated inside the lipid nanoparticle.

### Ionic lipid

The ionic lipid that can be used in the present invention may be any as long as it is composed of a tertiary amino group and a hydrophobic group and can constitute lipid nanoparticles.

Specific examples of the ionic lipid include 1,2-dioleoyloxy-3-dimethylaminopropane (DODAP), 1,2-dioleyloxy-3-dimethylaminopropane (DODMA), 1,2-dilinoleyloxy-3-dimethylaminopropane (DLinDMA), 2,2-dilinoleyl-4-(2-dimethylaminoethyl)-[1,3]-dioxolane (DLin-KC2-DMA), heptatriaconta-6,9,28,31-tetraen-19-yl-4-(dimethylamino)butanoate (DLin-MC3-DMA or MC3), heptadecan-9-yl 8-((2-hydroxyethyl) (8-(nonyloxy)-8-oxooctyl)amino)octanoate (Lipid 5), heptadecan-9-yl 8-((2-hydroxyethyl) (6-oxo-6-(undecyloxy)hexyl)amino)octanoate (Lipid 8), and compounds of the following formula (1) or the formula (2). The compounds of the following formula (1) or the formula (2), DODMA, MC3, Lipid 5, Lipid 8 are preferred, and the compound of the formula (1) or the formula (2) is most preferred.

A compound represented by the formula (1) 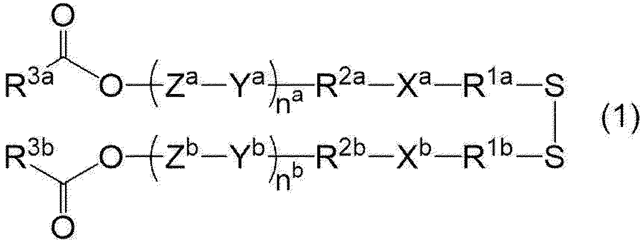 (in the formula (1),
R^{1a} and R^{1b} are each independently an alkylene group having 1 - 6 carbon atoms,
X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 - 16 carbon atoms and at least one aromatic ring, and optionally having a hetero atom,
n^{a} and n^{b} are each independently 0 or 1, and
R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (3):

   R⁹-O-CO-(CH₂)a- (3)
(in the formula (3),
R⁹ is an aliphatic hydrocarbon group having 2 - 20 carbon atoms, and
a is an integer of 2 to 10)).

R^{1a} and R^{1b} are each independently an alkylene group having 1 - 6 carbon atoms, and may be linear or branched, preferably linear. The carbon number of the alkylene group is preferably 1 - 4, more preferably 1 - 2. Specific examples of the alkylene group having 1 - 6 carbon atoms include methylene group, ethylene group, trimethylene group, isopropylene group, tetramethylene group, isobutylene group, pentamethylene group, neopentylene group and the like. Preferably, R^{1a} and R^{1b} are each independently a methylene group, an ethylene group, a trimethylene group, an isopropylene group or a tetramethylene group, most preferably an ethylene group.

R^{1a} may be the same as or different from R^{1b}, and R^{1a} is preferably the same group as R^{1b}.

X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups, preferably each independently a cyclic alkylene tertiary amino group having 2 - 5 carbon atoms and 1 - 2 tertiary amino groups.

The alkyl group having 1 - 6 carbon atoms in the non-cyclic alkyl tertiary amino group having 1 - 6 carbon atoms and one tertiary amino group may be linear, branched or cyclic. The alkyl group preferably has a carbon number of 1 to 3. Specific examples of the alkyl group having 1 - 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like, preferably methyl group, ethyl group, propyl group or isopropyl group, most preferably methyl group.

A preferable specific structure of the non-cyclic alkyl tertiary amino group having 1 - 6 carbon atoms and one tertiary amino group is represented by X¹.

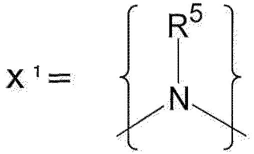

R⁵ in X¹ is an alkyl group having 1 - 6 carbon atoms, which may be linear, branched or cyclic. The alkyl group preferably has a carbon number of 1 - 3. Specific examples of the alkyl group having 1 - 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like. R⁵ is preferably methyl group, ethyl group, propyl group or isopropyl group, most preferably methyl group.

The carbon number of the cyclic alkylene tertiary amino group having 2 - 5 carbon atoms and 1 - 2 tertiary amino groups is preferably 4 - 5. The cyclic alkylene tertiary amino group having 2 - 5 carbon atoms and 1 - 2 tertiary amino groups is specifically aziridylene group, azetidylene group, pyrrolidylene group, piperidylene group, imidazolidylene group, or piperazylene group, preferably pyrrolidylene group, piperidylene group, or piperazylene group, most preferably piperidylene group.

A preferable specific structure of the cyclic alkylene tertiary amino group having 2 - 5 carbon atoms and one tertiary amino group is represented by X².

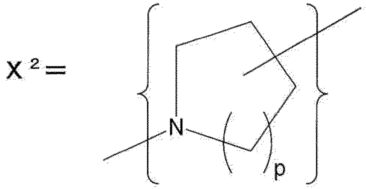

The p of X² is 1 or 2. When p is 1, X² is a pyrrolidylene group, and when p is 2, X² is a piperidylene group. Preferably, p is 2.

A preferable specific structure of the cyclic alkylene tertiary amino group having 2 - 5 carbon atoms and two tertiary amino groups is represented by X³.

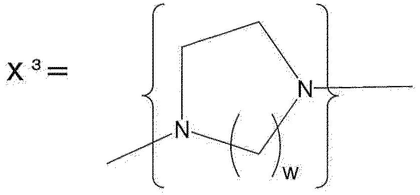

The w of X³ is 1 or 2. When w is 1, X³ is an imidazolidylene group, and when w is 2, X³ is a piperazylene group.

X^{a} may be the same as or different from X^{b}, and X^{a} is preferably the same group as X^{b}.

R^{2a} and R^{2b} are each independently an alkylene group or an oxydialkylene group each having not more than 8 carbon atoms, preferably each independently an alkylene group having not more than 8 carbon atoms.

The alkylene group having not more than 8 carbon atoms may be linear or branched, preferably linear. The number of carbons contained in the alkylene group is preferably not more than 6, most preferably not more than 4. Specific examples of the alkylene group having not more than 8 carbon atoms include methylene group, ethylene group, trimethylene group, isopropylene group, tetramethylene group, isobutylene group, pentamethylene group, hexamethylene group, heptamethylene group, octamethylene group and the like, preferably methylene group, ethylene group, trimethylene group, and tetramethylene group, most preferably ethylene group.

In the present specification, the "oxydialkylene group having not more than 8 carbon atoms" means a group containing alkylene groups via an ether bond (alkylene-O-alkylene, in other words, "alkyleneoxyalkylene group"), wherein the total carbon number of the two alkylene groups present is 8 or below. The two alkylene groups present may be the same or different, preferably the same. Specific examples of the oxydialkylene group having not more than 8 carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group) and the like. Preferably, it is oxydimethylene group, oxydiethylene group, oxydi(trimethylene) group, most preferably oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 - 16 carbon atoms and at least one aromatic ring, and optionally having a hetero atom. The number of carbons contained in the aromatic compound is preferably 6 to 12, most preferably 6 or 7. The aromatic ring contained in the aromatic compound is preferably one.

As the kind of the aromatic ring contained in the aromatic compound having 3 - 16 carbon atoms, benzene ring, naphthalene ring, and anthracene ring can be mentioned for aromatic hydrocarbocycle, and imidazole ring, pyrazole ring, oxazole ring, isoxazole ring, thiazole ring, isothiazole ring, triazine ring, pyrrole ring, furanthiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring and the like can be mentioned for aromatic heterocycle. It is preferably benzene ring, naphthalene ring, or anthracene ring, most preferably benzene ring.

The aromatic ring may have a substituent. Examples of the substituent include acyl group having 2 - 4 carbon atoms, alkoxycarbonyl group having 2 - 4 carbon atoms, carbamoyl group having 2 - 4 carbon atoms, acyloxy group having 2 - 4 carbon atoms, acylamino group having 2 - 4 carbon atoms, alkoxycarbonylamino group having 2 - 4 carbon atoms, fluorine atom, chlorine atom, bromine atom, iodine atom, alkylsulfanyl group having 1 - 4 carbon atoms, alkylsulfonyl group having 1 - 4 carbon atoms, arylsulfonyl group having 6 - 10 carbon atoms, nitro group, trifluoromethyl group, cyano group, alkyl group having 1 - 4 carbon atoms, ureido group having 1 - 4 carbon atoms, alkoxy group having 1 - 4 carbon atoms, aryl group having 6 - 10 carbon atoms, aryloxy group having 6 - 10 carbon atoms, and the like. Preferable examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group and the like.

A preferable specific structure of Z^{a} and Z^{b} is Z¹.

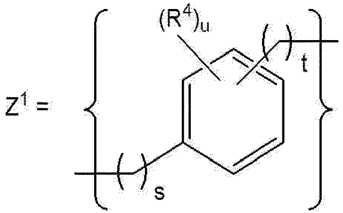

wherein s is an integer of 0 - 3, t is an integer of 0 - 3, u is an integer of 0 - 4, and R⁴ in the number of u are each independently a substituent.

The s for Z¹ is preferably an integer of 0 or 1, more preferably 0.

The t for Z¹ is preferably an integer of 0 to 2, more preferably 1.

The u for Z¹ is preferably an integer of 0 to 2, more preferably an integer of 0 or 1.

The R⁴ for Z¹ is a substituent of an aromatic ring (benzene ring) contained in the aromatic compound having 3 - 16 carbon atoms which does not inhibit the reaction in the synthesis process of an ionic lipid. Examples of the substituent include acyl group having 2 - 4 carbon atoms, alkoxycarbonyl group having 2 - 4 carbon atoms, carbamoyl group having 2 - 4 carbon atoms, acyloxy group having 2 - 4 carbon atoms, acylamino group having 2 - 4 carbon atoms, alkoxycarbonylamino group having 2 - 4 carbon atoms, fluorine atom, chlorine atom, bromine atom, iodine atom, alkylsulfanyl group having 1 - 4 carbon atoms, alkylsulfonyl group having 1 - 4 carbon atoms, arylsulfonyl group having 6 - 10 carbon atoms, nitro group, trifluoromethyl group, cyano group, alkyl group having 1 - 4 carbon atoms, ureido group having 1 - 4 carbon atoms, alkoxy group having 1 - 4 carbon atoms, aryl group having 6 - 10 carbon atoms, aryloxy group having 6 - 10 carbon atoms, and the like. Preferable examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group and the like. When R⁴ is present in plurality, each R⁴ may be the same or different.

Z^{a} may be the same as or different from Z^{b}, and Z^{a} is preferably the same group as Z^{b}.

n^{a} and n^{b} are each independently 0 or 1.

n^{a} may be the same as or different from n^{b}, and n^{a} is preferably the same as n^{b}.

R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (3):

R⁹-O-CO-(CH₂)a- (3)

(in the formula (3),
R⁹ is an aliphatic hydrocarbon group having 2 - 20 carbon atoms, and
a is an integer of 2 to 10), preferably each independently a residue derived from a reaction product of a liposoluble vitamin having a hydroxyl group, and succinic anhydride or glutaric anhydride, or an aliphatic hydrocarbon group having 12 - 22 carbon atoms, most preferably each independently an aliphatic hydrocarbon group having 12 - 22 carbon atoms.

The residue derived from a reaction product of a liposoluble vitamin having a hydroxyl group, and succinic anhydride or glutaric anhydride is a group having a structure in which the hydroxyl group of a liposoluble vitamin having a hydroxyl group is replaced by *-O-CO-CH₂-CH₂- or *-O-CO-CH₂-CH₂-CH₂-. * is the bonding position with the liposoluble vitamin. The residue derived from a reaction product of a sterol derivative having a hydroxyl group, and succinic anhydride or glutaric anhydride is a group having a structure in which the hydroxyl group of a sterol derivative having a hydroxyl group is replaced by *-O-CO-CH₂-CH₂- or *-O-CO-CH₂-CH₂-CH₂-. * is the bonding position with the sterol derivative.

The liposoluble vitamin having a hydroxyl group is, for example, retinol, ergosterol, 7-dehydrocholesterol, calciferol, cholecalciferol, dihydroergocalciferol, dihydrotachysterol, tocopherol, tocotrienol and the like. Preferred example of the liposoluble vitamin having a hydroxyl group is tocopherol.

Examples of the sterol derivative having a hydroxyl group include cholesterol, cholestanol, stigmasterol, β-sitosterol, lanosterol, and ergosterol and the like, preferably cholesterol or cholestanol.

The aliphatic hydrocarbon group having 1 - 40 carbon atoms may be linear or branched. The aliphatic hydrocarbon group may be saturated or unsaturated. In the case of an unsaturated aliphatic hydrocarbon group, the aliphatic hydrocarbon group generally contains 1 - 6, preferably 1 - 3, more preferably 1 - 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 12 - 22, more preferably 13 - 19, most preferably 13 - 17. While the aliphatic hydrocarbon group includes an alkyl group, an alkenyl group, an alkynyl group and the like, it is preferably an alkyl group or an alkenyl group. Specific examples of the aliphatic hydrocarbon group having 1 - 40 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, tetracontyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group and the like. The aliphatic hydrocarbon group having 1 - 40 carbon atoms is preferably tridecyl group, pentadecyl group, heptadecyl group, nonadecyl group, heptadecenyl group, heptadecadienyl group, or 1-hexylnonyl group, particularly preferably tridecyl group, heptadecyl group, heptadecenyl group, or heptadecadienyl group.

In one embodiment of the present invention, the aliphatic hydrocarbon group having 1 - 40 (preferably 12 - 22) carbon atoms for R^{3a} or R^{3b} is derived from fatty acid. In this case, the carbonyl carbon derived from fatty acid is contained in - CO-O- in the formula (1). A specific example of the aliphatic hydrocarbon group is a heptadecadienyl group when linoleic acid is used as the fatty acid, and heptadecenyl group when oleic acid is used as the fatty acid.

The alkyl group having a cyclopropane ring and having 3 - 40 carbon atoms for R^{3a} or R^{3b} means an alkyl group having at least one cyclopropane ring in the alkyl chain and having 3 - 40 carbon atoms. The number of carbon atoms in the alkyl group is 3 to 40, and does not include the number of carbon atoms in the cyclopropane ring. The number of cyclopropane rings in the alkyl group is preferably one. The alkyl group having a cyclopropane ring and having 3 - 40 carbon atoms for R^{3a} or R^{3b} is preferably a group represented by the formula (4): 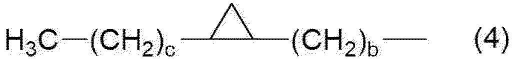 (in the formula (4), b and c are each independently an integer and the total of b and c is 2 to 39). Preferably, b is an integer of 1 to 20, c is an integer of 1 to 19. b is more preferably an integer of 2 to 18, further preferably an integer of 3 to 17, further more preferably an integer of 4 to 12. c is more preferably an integer of 3 to 15, further preferably an integer of 3 to 11, further more preferably an integer of 3 to 9. Examples of the group represented by the formula (4) include 7-(2-octylcyclopropyl)heptyl and the like.

In the formula (3), the aliphatic hydrocarbon group having 2 - 20 carbon atoms for R⁹ may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 or 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 8 to 20, more preferably 9 to 19, further preferably 13 to 19, most preferably 13 to 17. While the aliphatic hydrocarbon group includes an alkyl group, an alkenyl group, an alkynyl group and the like, it is preferably an alkyl group or an alkenyl group, more preferably alkyl group. Specific examples of the aliphatic hydrocarbon group having 2 to 20 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, dodecenyl group, tridecenyl group, tetradecenyl group, pentadecenyl group, hexadecenyl group, heptadecenyl group, octadecyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, icosatrienyl group, icosatetraenyl group, icosapentaenyl group, isostearyl group, 1-hexylheptyl group, 1-ethylnonyl group, 1-butylnonyl group, 1-hexylnonyl group, 1-octylnonyl group, 1-octylundecyl group, 3-octylundecyl group, and the like. The aliphatic hydrocarbon group having 2 - 20 carbon atoms is preferably tridecyl group, pentadecyl group, heptadecyl group, nonadecyl group, heptadecenyl group, heptadecadienyl group, or 1-hexylnonyl group, particularly preferably tridecyl group, heptadecyl group, heptadecenyl group, or heptadecadienyl group.

In the formula (3), a is preferably an integer of 3 to 9, more preferably an integer of 3 to 7, further preferably an integer of 5 to 7, and most preferably 5 or 7.

R^{3a} may be the same as or different from R^{3b}, and R^{3a} is preferably the same group as R^{3b}.

In one embodiment of the present invention, R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferable examples of the ionic lipid (sometimes to be abbreviated as "ionic lipid (1)" in the present specification) represented by the formula (1) include the following ionic lipids.

### [Ionic lipid (1-1)]

Ionic lipid (1) wherein
R^{1a} and R^{1b} are each independently an alkylene group having 1 - 6 carbon atoms (e.g., methylene group, ethylene group);
X^{a} and X^{b} are each independently a non-cyclic alkyl tertiary amino group having 1 - 6 carbon atoms and one tertiary amino group (e.g., -N(CH₃)-), or a cyclic alkylene tertiary amino group having 2 - 5 carbon atoms and 1 - 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 - 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 - 22 carbon atoms (e.g., heptadecenyl group, heptadecadienyl group, 1-hexylnonyl group).

### [Ionic lipid (1-2)]

Ionic lipid (1) wherein
R^{1a} and R^{1b} are each independently an alkylene group having 1 - 6 carbon atoms (e.g., methylene group, ethylene group);
X^{a} and X^{b} are each independently a non-cyclic alkyl tertiary amino group having 1 - 6 carbon atoms and one tertiary amino group (e.g., -N(CH₃)-), or a cyclic alkylene tertiary amino group having 2 - 5 carbon atoms and 1 - 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 - 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a residue derived from a reaction product of a liposoluble vitamin having a hydroxyl group (e.g., tocopherol), and succinic anhydride or glutaric anhydride, or an aliphatic hydrocarbon group having 12 - 22 carbon atoms (e.g., heptadecenyl group, heptadecadienyl group, 1-hexylnonyl group).

### [Ionic lipid (1-3)]

Ionic lipid (1) wherein
R^{1a} and R^{1b} are each independently an alkylene group having 1 - 2 carbon atoms (i.e., methylene group or ethylene group);
X^{a} and X^{b} are each independently X¹:

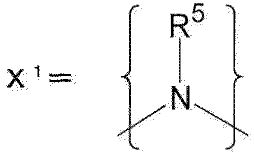

wherein R⁵ is an alkyl group having 1 - 3 carbon atoms (e.g., methyl group), or X²:

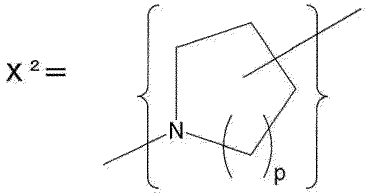

wherein p is 1 or 2;
R^{2a} and R^{2b} are each independently alkylene group having not more than 4 carbon atoms (e.g., methylene group, ethylene group, trimethylene group);
Y^{a} and Y^{b} are each independently an ester bond or an amide bond;
Z^{a} and Z^{b} are each independently Z¹: 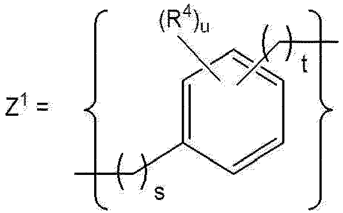
   wherein s is an integer of 0 - 1, t is an integer of 0 - 2, u is an integer of 0 - 2 (preferably 0), and R⁴ in the number of u are each independently a substituent; and
   n^{a} and n^{b} are each independently 0 or 1; and
   R^{3a} and R^{3b} are each independently a residue derived from a reaction product of a liposoluble vitamin having a hydroxyl group (e.g., tocopherol) and succinic anhydride, or an aliphatic hydrocarbon group having 13 - 17 carbon atoms (e.g., heptadecenyl group, heptadecadienyl group, 1-hexylnonyl group).

Specific examples of the ionic lipid (1) include the following O-Ph-P3C1, O-Ph-P4C1, O-Ph-P4C2, O-Bn-P4C2, E-Ph-P4C2, L-Ph-P4C2, HD-Ph-P4C2, O-Ph-amide-P4C2, O-Ph-C3M, B-2, B-2-5, TS-P4C2, L-P4C2, and O-P4C2.

Specific examples of ionic lipid (1) are Lipid 1 to Lipid 20 described in WO2021/195529A2.

**[Table 1-1]**

| name of ionic lipid | structure |
|---|---|
| O-Ph-P3C1 | 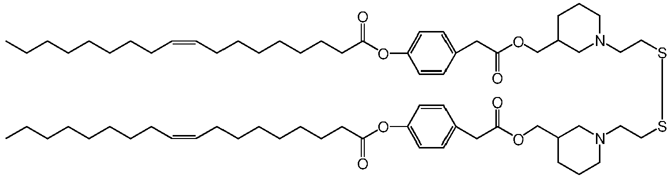 |
| O-Ph-P4C1 | 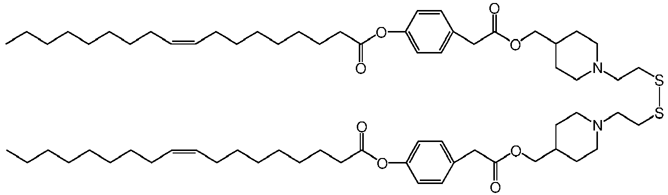 |
| O-Ph-P4C2 (or SS-OP) | 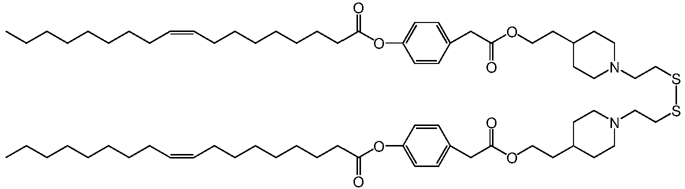 |
| O-Bn-P4C2 | 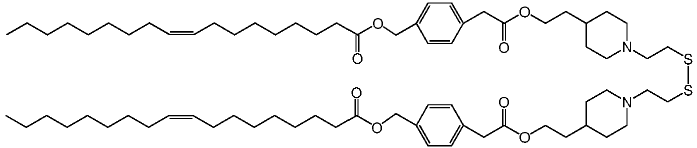 |
| E-Ph-P4C2 (or SS-EP) | 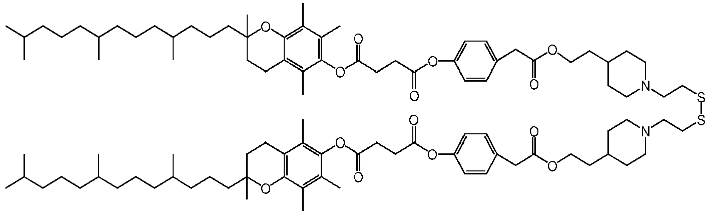 |

**[Table 1-2]**

| name of ionic lipid | structure |
|---|---|
| L-Ph-P4C2 | 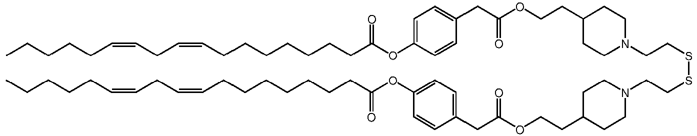 |
| HD-Ph-P4C2 | 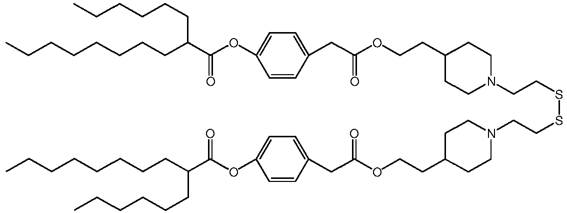 |
| O-Ph-amide-P4C2 | 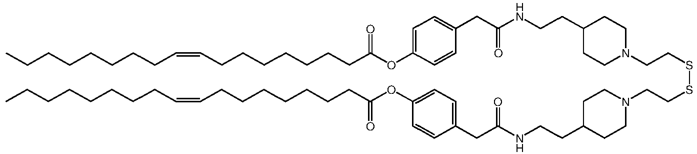 |
| O-Ph-C3M | 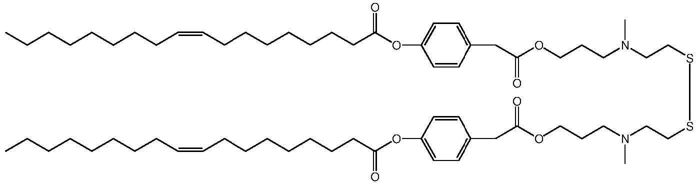 |

**[Table 1-3]**

| name of ionic lipid | structure |
|---|---|
| B-2 | 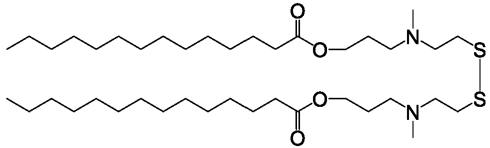 |
| B-2-5 | 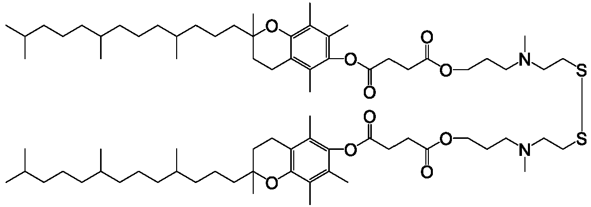 |
| TS-P4C2 | 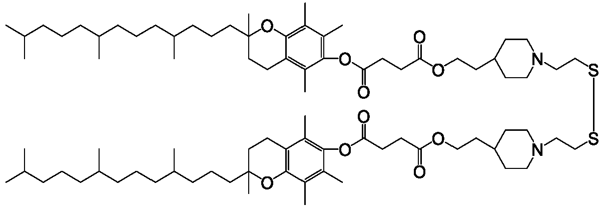 |
| L-P4C2 | 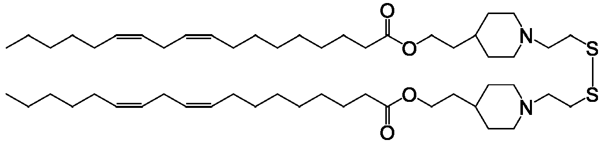 |
| O-P4C2 (or SS-OC) | 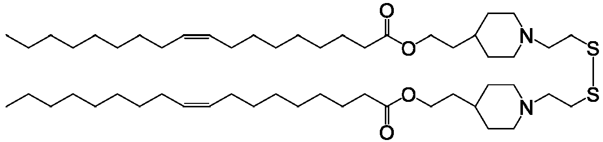 |

Ionic lipid (1) can be produced by a known method (e.g., methods described in WO2019/188867A1 (US2021/0023008A1), US9708628B2, WO2021/195529A2).

A compound represented by the formula (2)

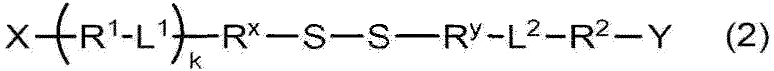

wherein
X is a nitrogen-containing aliphatic group containing one or more tertiary nitrogens,
R¹ is an aliphatic hydrocarbon group having not more than 8 carbon atoms,
L¹ is an ester bond, an amide bond, a carbamate bond, an N-alkylcarbamate bond, a carbonate bond or a urea bond,
k is 0 or 1,
R^{x} and R^{y} are each independently an alkylene group having 2 - 5 carbon atoms,
L² is an ester bond, an amide bond, a carbamate bond, a carbonate bond, an ether bond or a urea bond,
R² is an alkylene group having not more than 8 carbon atoms, or absent, and
Y is a group which (i) contains one or more divalent groups derived from an aromatic compound optionally having a hetero atom, (ii) contains a group containing at least one selected from the group consisting of an ester bond and a carbonate bond on the aromatic ring of the aforementioned divalent group, and (iii) contains at least one selected from the group consisting of an aliphatic hydrocarbon group having 10 - 37 carbon atoms, a liposoluble vitamin residue, and a residue of a sterol derivative.

One embodiment of preferred X can be represented by the following formula (5).

R^{ex}-N(R^{β})- (5)

wherein
R^{α} is an aliphatic hydrocarbon group, and
Rβ is an aliphatic hydrocarbon group, an aliphatic group containing one or more hetero atoms other than nitrogen, or an aliphatic group containing one or more tertiary amines, or
R^{α} and R^{β} may be bonded to form a 3- to 8-membered nitrogen-containing alicyclic ring.

More preferred example of X is a dialkylamino group (wherein the two alkyl groups of the dialkylamino group each independently have 1 to 8 carbon atoms), a 3- to 6-membered cyclic amino group optionally having a hetero atom, or -N(R^{a})-Rb,
R^{a} is an alkyl group having 1 - 8 carbon atoms,
R^{b} is - (CH2) q-O-R^{c},
R^{c} is a hydrogen or an alkyl group having 1 - 8 carbon atoms, and
q is an integer of 2 to 4.

The number of carbon atoms in the two alkyl groups in the dialkylamino group is preferably each independently 1 to 5, more preferably each independently 1 to 4. The alkyl group may be linear, branched, or cyclic.

Specifically, methyl group, ethyl group, propyl group, isopropyl group, cyclopropyl group, n-butyl group, sec-butyl group, isobutyl group, tert-butyl group, cyclobutyl group, pentyl group, isopentyl group, neopentyl group, tert-pentyl group, 1,2-dimethylpropyl group, 2-methylbutyl group, cyclopentyl group and the like can be mentioned. It is preferably each independently methyl group, ethyl group, propyl group or isopropyl group, more preferably each independently methyl group or ethyl group.

The 3- to 6-membered cyclic amino group optionally having a hetero atom means a group in which the substituents of the amino group are bonded to form a ring, the number of atoms forming the ring is 3 to 6, and which may have a hetero atom such as oxygen. The 3- to 6-membered cyclic amino group optionally having a hetero atom is preferably a 5- or 6-membered cyclic amino group optionally having a hetero atom, more preferably a 6-membered cyclic amino group optionally having a hetero atom. As the cyclic amino group, an amino group in which the ring is formed only of a nitrogen atom and a methylene group (-CH₂-) is preferred, and may contain an oxygen atom therein. It is specifically a 1-pyrrolidinyl group, a 1-piperidyl group, or a morpholino group (4-morpholinyl group), preferably a 1-piperidyl group or a morpholino group.

The aliphatic hydrocarbon group having not more than 8 carbon atoms for R¹ is preferably an alkylene group, an alkenylene group, or an alkynylene group, and more preferably an alkylene group or an alkenylene group.

The alkylene group having not more than 8 carbon atoms may be linear or branched chain. The carbon number of the above-mentioned alkylene group is preferably not more than 6, more preferably not more than 4. The alkylene group having not more than 8 carbon atoms is preferably methylene group, ethylene group, trimethylene group, tetramethylene group, isopropylene group (-CH(CH₃)CH₂-, -CH₂CH(CH₃)-), isobutylene group (-C(CH₃)₂CH₂-, -CH₂C(CH₃)₂-), pentamethylene group, or hexamethylene group, more preferably methylene group, ethylene group, trimethylene group, isopropylene group (-CH(CH₃)CH₂-, - CH₂CH(CH₃)-), tetramethylene group, or isobutylene group (-C (CH₃)₂CH₂-, -CH₂C(CH₃)₂-).

The alkenylene group having not more than 8 carbon atoms may be linear or branched chain. The carbon number of the above-mentioned alkenylene group is preferably not more than 6, more preferably not more than 4. The alkenylene group having not more than 8 carbon atoms is preferably propenylene group (-CH₂CH=CH-, -CH=CHCH₂-), butenylene group, isopropenylene group, isobutenylene group, pentenylene group, hexaylene group, more preferably propenylene group (-CH₂CH=CH-, -CH=CHCH₂-), butenylene group, isopropenylene group, or isobutenylene group.

L¹ is an ester bond, an amide bond, a carbamate bond, an N-alkylcarbamate bond, a carbonate bond or a urea bond, preferably an ester bond, an amide bond, a carbamate bond, an N-methylcarbamate bond or a carbonate bond.

In the present specification, N-alkylcarbamate bond is - NR¹⁰-CO-O- or -O-CO-NR¹⁰-, and R¹⁰ is an alkyl group having 1 - 8 carbon atoms. The alkyl group having 1 - 8 carbon atoms may be linear, branched chain or cyclic. The carbon number of the above-mentioned alkyl group is preferably 1 to 6, more preferably 1 to 4. Specifically, methyl group, ethyl group, propyl group, isopropyl group, cyclopropyl group, n-butyl group, sec-butyl group, isobutyl group, tert-butyl group, cyclobutyl group, pentyl group, isopentyl group, neopentyl group, tert-pentyl group, 1,2-dimethylpropyl group, 2-methylbutyl group, cyclopentyl group and the like can be mentioned. It is preferably a methyl group, an ethyl group, a propyl group or an isopropyl group, more preferably a methyl group.

k is 0 or 1. Here, when k is 0, it means that R¹-L¹ does not exist, that is, X and R^{x} are directly bonded. The same applies when l, m, n, and the like are 0.

The alkylene group having 2 - 5 carbon atoms for R^{x} or R^{y} may be linear or branched chain, preferably linear. The carbon number of the above-mentioned alkylene group is preferably 2 to 4, more preferably 2.

It is specifically an ethylene group, a trimethylene group, a tetramethylene group, an isopropylene group (-CH(CH₃)CH₂-, -CH₂CH(CH₃)-), or an isobutylene group (-C (CH₃)₂CH₂-, -CH₂C(CH₃)₂-) , preferably an ethylene group or a trimethylene group, more preferably an ethylene group.

L² is an ester bond, an amide bond, a carbamate bond, a carbonate bond, an ether bond or a urea bond, preferably an ester bond or an amide bond, more preferably an ester bond.

R² is an alkylene group having not more than 8 carbon atoms, or absent. That R² is absent means that L² and Y are directly bonded.

The alkylene group having not more than 8 carbon atoms for R² may be linear or branched chain, preferably linear. The carbon number of the above-mentioned alkylene group is preferably not more than 6, more preferably not more than 4. The alkylene group having not more than 8 carbon atoms is preferably methylene group, ethylene group, trimethylene group, tetramethylene group, isopropylene group (-CH(CH₃)CH₂-, - CH₂CH (CH₃)-), isobutylene group (-C(CH₃)₂CH₂-, -CH₂C(CH₃)₂-), pentamethylene group, hexamethylene group, more preferably methylene group, ethylene group, trimethylene group, isopropylene group (-CH(CH₃)CH₂-, -CH₂CH(CH₃)-), tetramethylene group, or isobutylene group (-C(CH₃)₂CH₂-, -CH₂C(CH₃)₂-).

The alkyl group having 1 - 8 carbon atoms for R^{a} or R^{c} may be linear or branched chain, preferably linear. The carbon number of the above-mentioned alkyl group is preferably 1 to 6, more preferably 1 to 4. Specific examples of the alkyl group having 1 - 8 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group and the like. It is preferably methyl group, ethyl group, propyl group or isopropyl group, more preferably methyl group.

One embodiment of preferred Y can be represented by the following formula (6).

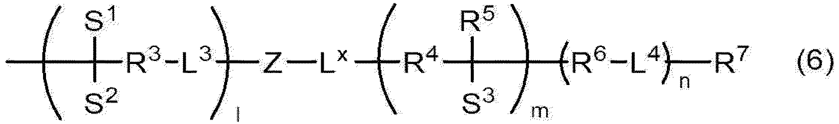

wherein
R³ is an alkylene group having not more than 8 carbon atoms,
L³ is an ester bond or a carbonate bond,
S¹ is a hydrogen, an alkyl group having 1 - 8 carbon atoms, -R^{e}-L^{a}-R⁷', or -R^{e}-L^{a}-Z'-L^{b}-R⁷',
S² is -R^{e}' -L^{a}-R⁷" or -R^{e}'-L^{a}-Z"-L^{b}-R⁷",
R^{e} and R^{e}' are each independently an alkylene group having not more than 8 carbon atoms,
L^{a} is an ester bond or a carbonate bond,
L^{b} is an ester bond or a carbonate bond,
l is 0 or 1,
Z, Z' and Z" are each independently a divalent group derived from an aromatic compound having 3 - 16 carbon atoms and at least one aromatic ring, and optionally having a hetero atom,
L^{x} is an ester bond or a carbonate bond,
R⁴ is an alkylene group having not more than 8 carbon atoms, or absent,
R⁵ is a hydrogen or an alkyl group having 1 - 8 carbon atoms,
S³ is -R⁶' -L⁴' -R⁷‴,
m is 0 or 1,
R⁶ and R⁶' are each independently an alkylene group having not more than 8 carbon atoms, or absent,
L⁴ and L⁴' are each independently an ester bond or a carbonate bond,
R⁷, R⁷' , R⁷" and R⁷‴ are each independently an aliphatic hydrocarbon group having 10 - 37 carbon atoms, or - (CH₂)p-C(=O)-R^{f},
R^{f} is a residue of a liposoluble vitamin having a hydroxyl group or a residue of a sterol derivative having a hydroxyl group, and p is 2 or 3, and
n is 0 or 1.

R⁴ is an alkylene group having not more than 8 carbon atoms, or absent. That R⁴ is absent means that the carbon atom to which R⁵ and S³ are bonded and L^{x} are directly bonded.

R⁶ and R⁶' are each independently an alkylene group having not more than 8 carbon atoms, or absent. That R⁶ is absent means that when m=0, L^{x} and L⁴ are directly bonded and when m=1, the carbon atom to which R⁵ and S³ are bonded and L⁴ are directly bonded. That R⁶' is absent means that the carbon atom to which R⁴ and R⁵ are bonded and L⁴' are directly bonded.

The alkylene group having not more than 8 carbon atoms for R³, R⁴, R⁶, R⁶' , R^{e} or R^{e}' may be linear or branched chain, preferably linear. The carbon number of the above-mentioned alkylene group is preferably not more than 6, more preferably not more than 4. The alkylene group having not more than 8 carbon atoms is preferably methylene group, ethylene group, trimethylene group, tetramethylene group, isopropylene group (-CH(CH₃)CH₂-, -CH₂CH(CH₃)-), isobutylene group (-C(CH₃)₂CH₂-, - CH₂C(CH₃)₂-), pentamethylene group, or hexamethylene group, more preferably methylene group, ethylene group, trimethylene group, isopropylene group (-CH(CH₃)CH₂-, -CH₂CH(CH₃)-), tetramethylene group, or isobutylene group (-C(CH₃)₂CH₂-, -CH₂C(CH₃)₂-).

The alkyl group having 1 - 8 carbon atoms for R⁵ or S¹ may be linear or branched, preferably linear. The carbon number of the above-mentioned alkyl group is preferably 1 - 6, more preferably 1 - 4. Specific examples of the alkyl group having 1 - 8 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, and the like. Preferably, it is a methyl group, an ethyl group, a propyl group or an isopropyl group, more preferably a methyl group.

L^{x}, L³, L⁴, L⁴' , L^{a} and L^{b} are each an ester bond or a carbonate bond, preferably an ester bond.

Z, Z' and Z" are each independently a divalent group derived from an aromatic compound having 3 - 16 carbon atoms and at least one aromatic ring, and optionally having a hetero atom. Here, the divalent group means a divalent group having a structure obtained by removing two hydrogen atoms from the above-mentioned aromatic compound. The number of carbon atoms of the above-mentioned aromatic compound is preferably 6 to 12, more preferably 6 or 7. The number of aromatic rings of the above-mentioned aromatic compound is preferably 1. Z, Z' and Z'' may be the same or different, and Z, Z' and Z" are preferably the same.

The aromatic ring of the above-mentioned aromatic compound may be either an aromatic hydrocarbon ring or an aromatic hetero ring. Examples of the aromatic hydrocarbon ring include a benzene ring, a naphthalene ring, and an anthracene ring. Examples of the aromatic hetero ring include an imidazole ring, a pyrazole ring, an oxazole ring, an isoxazole ring, a thiazole ring, an isothiazole ring, a triazine ring, a pyrrole ring, a furanthiophene ring, a pyrimidine ring, a pyridazine ring, a pyrazine ring, a pyridine ring, a purine ring, a pteridine ring, a benzimidazole ring, an indole ring, a benzofuran ring, a quinazoline ring, a phthalazine ring, a quinoline ring, an isoquinoline ring, a coumarin ring, a chromone ring, a benzodiazepine ring, a phenoxazine ring, a phenothiazine ring, and an acridine ring. The aromatic ring of the aromatic compound is preferably a benzene ring, a naphthalene ring, or an anthracene ring, more preferably a benzene ring.

The aromatic ring of the above-mentioned aromatic compound may have a substituent. Examples of the substituent include an acyl group having 2 - 4 carbon atoms, an alkoxycarbonyl group having 2 - 4 carbon atoms, a carbamoyl group having 2 - 4 carbon atoms, an acyloxy group having 2 - 18 carbon atoms, an acylamino group having 2 - 4 carbon atoms, an alkoxycarbonylamino group having 2 - 4 carbon atoms, a fluorine atom, a chlorine atom, a bromine atom, an iodine atom, an alkylsulfanyl group having 1 - 4 carbon atoms, an alkylsulfonyl group having 1 - 4 carbon atoms, an arylsulfonyl group having 6 - 10 carbon atoms, a nitro group, a trifluoromethyl group, a cyano group, an alkyl group having 1 - 4 carbon atoms, a ureido group having 1 - 4 carbon atoms, an alkoxy group having 1 - 4 carbon atoms, an aryl group having 6 - 10 carbon atoms, and an aryloxy group having 6 - 10 carbon atoms. Preferred examples of the above-mentioned substituent include an acetyl group, a methoxycarbonyl group, a methylcarbamoyl group, an acetoxy group, an acetamido group, a methoxycarbonylamino group, a fluorine atom, a chlorine atom, a bromine atom, an iodine atom, a methylsulfanyl group, a phenylsulfonyl group, a nitro group, a trifluoromethyl group, a cyano group, a methyl group, an ethyl group, a propyl group, an isopropyl group, a tert-butyl group, a ureido group, a methoxy group, an ethoxy group, a propoxy group, an isopropoxy group, a tert-butoxy group, a phenyl group, a phenoxy group, and the like.

Z, Z' and Z'' are preferably each independently the formula (7):

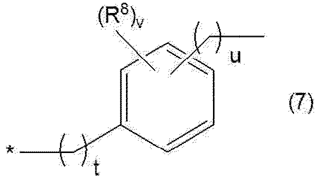

wherein
t is an integer of 0 to 3,
u is an integer of 0 to 3,
v is an integer of 0 to 4, and
R⁸ in the number of v are each independently a substituent,
* is the bonding position with L³ or L^{a},
t is preferably 0 or 1, more preferably 1.
u is preferably an integer of 0 to 2, more preferably 0.
v is preferably an integer of 0 to 2, more preferably 0 or 1, further preferably 0.

Examples of R⁸ include acyl group having 2 - 4 carbon atoms, alkoxycarbonyl group having 2 - 4 carbon atoms, carbamoyl group having 2 - 4 carbon atoms, acyloxy group having 2 - 18 carbon atoms, acylamino group having 2 - 4 carbon atoms, alkoxycarbonylamino group having 2 - 4 carbon atoms, fluorine atom, chlorine atom, bromine atom, iodine atom, alkylsulfanyl group having 1 - 4 carbon atoms, alkylsulfonyl group having 1 - 4 carbon atoms, arylsulfonyl group having 6 - 10 carbon atoms, nitro group, trifluoromethyl group, cyano group, alkyl group having 1 - 4 carbon atoms, ureido group having 1 - 4 carbon atoms, alkoxy group having 1 - 4 carbon atoms, aryl group having 6 - 10 carbon atoms, aryloxy group having 6 - 10 carbon atoms, and the like. Preferred examples of R⁸ include acetyl group, methoxycarbonyl group, methylcarbamoyl group, acetoxy group, propanoyloxy group, butanoyloxy group, pentanoyloxy group, hexanoyloxy group, heptanoyloxy group, octanoyloxy group, nonanoyloxy group, decanoyloxy group, undecanoyloxy group, dodecanoyloxy group, tridecanoyloxy group, tetradecanoyloxy group, pentadecanoyloxy group, hexadecanoyloxy group, heptadecanoyloxy group, octadecanoyloxy group, yloxy group, octadecenoyloxy group, octadecadienoyloxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like. When R⁸ is present in plurality, each R⁸ may be the same or different.

R⁷, R⁷' , R⁷" and R⁷‴ are each independently an aliphatic hydrocarbon group having 10 - 37 carbon atoms or - (CH₂)p-C(=O)-R^{f}, R^{f} is a residue of a liposoluble vitamin having a hydroxyl group or a residue of a sterol derivative having a hydroxyl group, and p is 2 or 3. R⁷, R⁷', R⁷" and R⁷‴ each may be the same or different.

In the present specification, the residue of a liposoluble vitamin having a hydroxyl group is a monovalent group having a structure obtained by removing a hydrogen atom from the hydroxyl group of liposoluble vitamin. In addition, the residue of a sterol derivative having a hydroxyl group is a monovalent group having a structure obtained by removing a hydrogen atom from the hydroxyl group of a sterol derivative.

The aliphatic hydrocarbon group having 10 - 37 carbon atoms may be linear or branched chain. The carbon number of the above-mentioned aliphatic hydrocarbon group is preferably 12 to 37, more preferably 13 to 37, further preferably 15 to 37.

The above-mentioned aliphatic hydrocarbon group may be saturated or unsaturated. When the above-mentioned aliphatic hydrocarbon group is an unsaturated aliphatic hydrocarbon group, the number of the unsaturated bond is preferably 1 to 6, more preferably 1 to 3, further preferably 1 or 2. While the unsaturated bond may be a carbon-carbon double bond or a carbon-carbon triple bond, it is preferably a carbon-carbon double bond. The above-mentioned aliphatic hydrocarbon group is preferably an alkyl group or an alkenyl group.

Examples of the aliphatic hydrocarbon group having 10 - 37 carbon atoms include decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group, henicosenyl group, heneicosenyl group, docosenyl group, dodecadienyl group, tridecadienyl group, tetradecadienyl group, pentadecadienyl group, hexadecadienyl group heptadecadienyl group, octadecadienyl group, nonadecadienyl group, icosadienyl group, henicosadienyl group, heneicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group, 1-dodecyltridecyl group, 1-tetradecylpentadecyl group, 1-hexadecylheptadecyl group, 1-octadecylnonadecyl group and the like.

The aliphatic hydrocarbon group having 10 - 37 carbon atoms is preferably undecyl group, dodecyl group, tridecyl group, tetradecyl group, pentadecyl group, hexadecyl group, heptadecyl group, octadecyl group, nonadecyl group, icosyl group, henicosyl group, docosyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group, henicosenyl group, heneicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, heneicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group, 1-dodecyltridecyl group, 1-tetradecylpentadecyl group, 1-hexadecylheptadecyl group, or 1-octadecylnonadecyl group, particularly preferably pentadecyl group, hexadecyl group, heptadecyl group, octadecyl group, nonadecyl group, icosyl group, henicosyl group, docosyl group, heptadecenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, heneicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group, 1-dodecyltridecyl group, 1-tetradecylpentadecyl group, 1-hexadecylheptadecyl group, or 1-octadecylnonadecyl group.

The liposoluble vitamin having a hydroxyl group is, for example, retinol, ergosterol, 7-dehydrocholesterol, calciferol, cholecalciferol, dihydroergocalciferol, dihydrotachysterol, tocopherol, tocotrienol and the like. Preferred example of the liposoluble vitamin having a hydroxyl group is tocopherol.

Examples of the sterol derivative having a hydroxyl group include cholesterol, cholestanol, stigmasterol, β-sitosterol, lanosterol, and ergosterol and the like. The sterol derivative having a hydroxyl group is preferably cholesterol or cholestanol.

R^{f} is preferably a residue of a liposoluble vitamin having a hydroxyl group. p is preferably 2.

l, m, n are each 0 or 1. Preferred combinations of l, m, n include
l=0, m=0, n=0
l=0, m=0, n=1
l=0, m=1, n=1
l=1, m=0, n=0,
particularly preferably
l=0, m=0, n=0
l=0, m=1, n=1
l=1, m=0, n=0.

Preferred examples of the ionic lipid represented by the formula (2) (sometimes to be abbreviated as "ionic lipid (2)" in the present specification) include the following ionic lipid.

Ionic lipid (2) wherein
X is a dialkylamino group (carbon numbers of two alkyl groups of the aforementioned dialkylamino group are each independently 1 to 8), a 3- to 6-membered cyclic amino group optionally having a hetero atom, or -N(R^{a})-R^{b},
R^{a} is alkyl group having 1 - 8 carbon atoms,
R^{b} is - (CH2) q-O-R^{c},
R^{c} is a hydrogen or an alkyl group having 1 - 8 carbon atoms,
q is an integer of 2 to 4;
R¹ is an alkylene group having not more than 6 carbon atoms, or an alkenylene group having not more than 6 carbon atoms;
L¹ is an ester bond, an amide bond, a carbamate bond or a carbonate bond
k is 0 or 1;
R^{x} and R^{y} are each independently an alkylene group having 2 - 4 carbon atoms group;
L² is an ester bond or an amide bond;
R² is an alkylene group having not more than 6 carbon atoms, or absent;
Y is a group represented by the formula (Y):

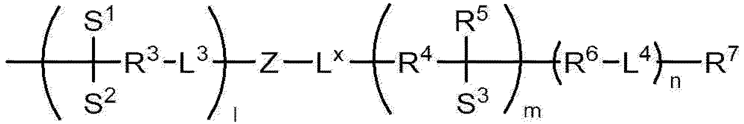

   wherein
   R³ is an alkylene group having not more than 6 carbon atoms,
   L³ is an ester bond,
   S¹ is a hydrogen, an alkyl group having 1 - 8 carbon atoms, -R^{e}-L^{a}-R⁷', or -R^{e}-L^{a}-Z'-L^{b}-R⁷',
   S² is -R^{e}'-L^{a}-R⁷" or -R^{e}' -L^{a}-Z"-L^{b}-R^{7"},
   R^{e} and R^{e}' are each independently an alkylene group having not more than 6 carbon atoms,
   L^{a} is an ester bond,
   L^{b} is an ester bond,
   l is 0 or 1,
   L^{x} is an ester bond or a carbonate bond,
   R⁴ is an alkylene group having not more than 6 carbon atoms, or absent,
   R⁵ is a hydrogen or an alkyl group having 1 - 8 carbon atoms,
   S³ is -R⁶' -L⁴' R⁷‴,
   m is 0 or 1,
   R⁶ and R⁶' are each independently an alkylene group having not more than 6 carbon atoms, or absent,
   L⁴ and L⁴' are each an ester bond,
   R⁷, R⁷', R⁷" and R⁷‴ are each independently an aliphatic hydrocarbon group having 10 - 37 carbon atoms, or - (CH₂)p-C(=O)-R^{f},
   R^{f} is a residue of a liposoluble vitamin having a hydroxyl group or a residue of a sterol derivative having a hydroxyl group, and p is 2 or 3, and
   n is 0 or 1;
   Z, Z' and Z" are each independently a group represented by the formula (Z):

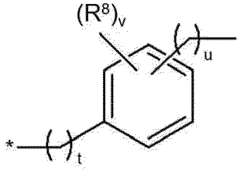

      wherein
      t is an integer of 0 or 1,
      u is an integer of 0 to 2,
      v is an integer of 0 to 2, and
      R⁸ in the number of v are each independently an acyl group having 2 - 4 carbon atoms, an alkoxycarbonyl group having 2 - 4 carbon atoms, a carbamoyl group having 2 - 4 carbon atoms, an acyloxy group having 2 - 18 carbon atoms, an acylamino group having 2 - 4 carbon atoms, an alkoxycarbonylamino group having 2 - 4 carbon atoms, a fluorine atom, a chlorine atom, a bromine atom, an iodine atom, an alkylsulfanyl group having 1 - 4 carbon atoms, an alkylsulfonyl group having 1 - 4 carbon atoms, an arylsulfonyl group having 6 - 10 carbon atoms, a nitro group, a trifluoromethyl group, a cyano group, an alkyl group having 1 - 4 carbon atoms, a ureido group having 1 - 4 carbon atoms, an alkoxy group having 1 - 4 carbon atoms, an aryl group having 6 - 10 carbon atoms, or an aryloxy group having 6 - 10 carbon atoms.

Preferred specific examples of the ionic lipid (2) include the compounds described in Production Example 1 - Production Example 46 below, but the present invention is not limited thereto. The compounds described in Production Example 1 - Production Example 46 are referred to as compound 1 - compound 46, respectively.

The ionic lipid (2) is preferably at least one selected from the group consisting of these, more preferably at least one selected from the group consisting of compounds 5, 7, 8, 9, 10, 11, 15, 19, 21, 22, 23, 24, 25, 28, 29, 30, 31, 32, 34, 35, 36, and 42, even more preferably at least one selected from the group consisting of compounds 5, 7, 8, 9, 10, 11, 15, 21, 22, 24, 31, 32, 34, 35, and 36, most preferably compound 31 or compound 34.

**[Table 2-1]**

| name | structure |
|---|---|
| compound 1 | 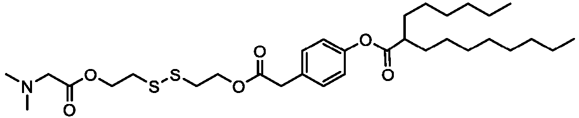 |
| compound 2 | 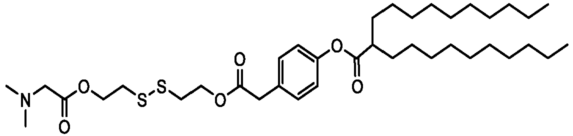 |
| compound 3 | 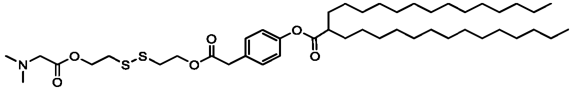 |
| compound 4 | 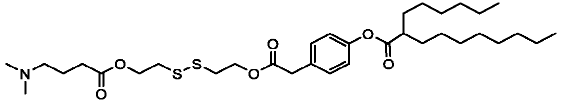 |
| compound 5 | 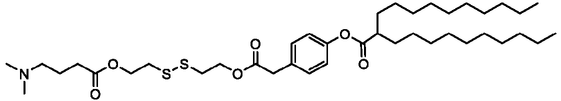 |
| compound 6 | 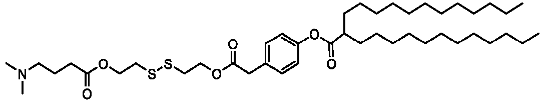 |
| compound 7 | 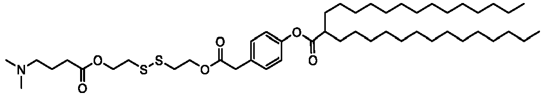 |
| compound 8 | 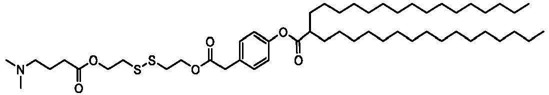 |
| compound 9 | 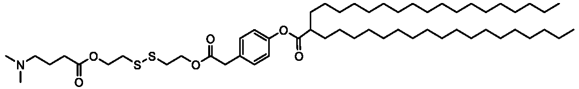 |
| compound 10 | 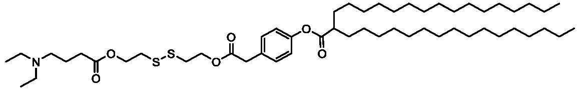 |

**[Table 2-2]**

| name | structure |
|---|---|
| compound 11 | 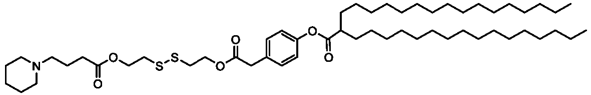 |
| compound 12 | 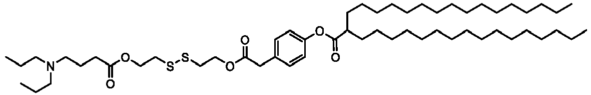 |
| compound 13 | 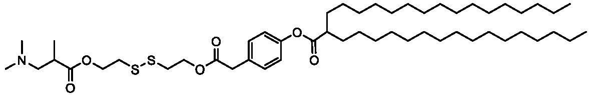 |
| compound 14 | 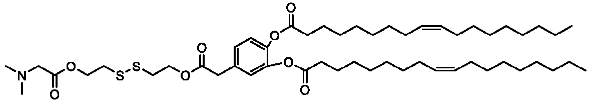 |
| compound 15 | 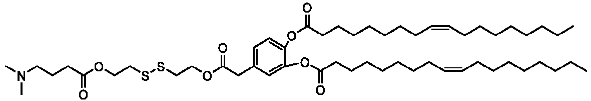 |
| compound 16 | 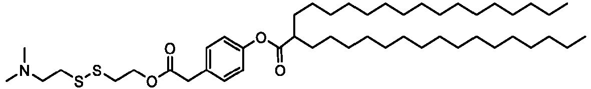 |
| compound 17 | 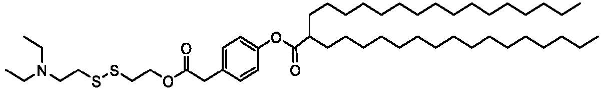 |
| compound 18 | 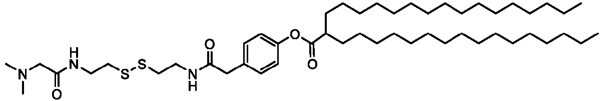 |
| compound 19 | 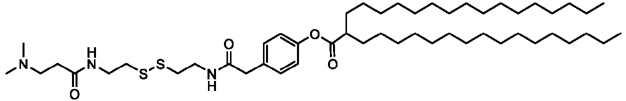 |
| compound 20 | 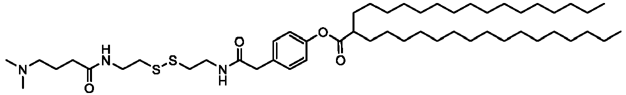 |
| compound 21 | 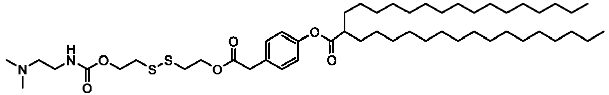 |

**[Table 2-3]**

| name | structure |
|---|---|
| compound 22 | 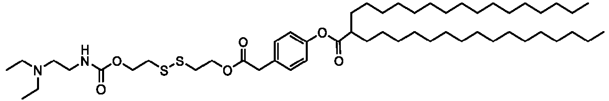 |
| compound 23 | 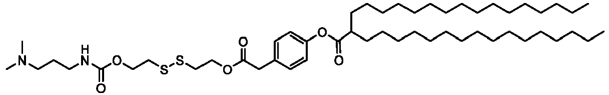 |
| compound 24 | 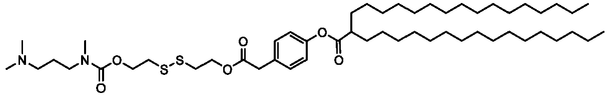 |
| compound 25 | 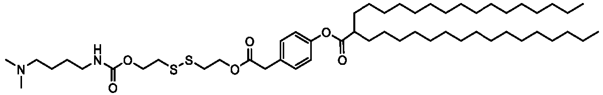 |
| compound 26 | 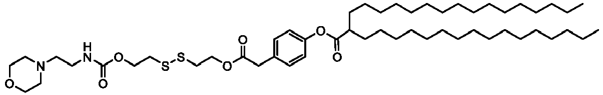 |
| compound 27 | 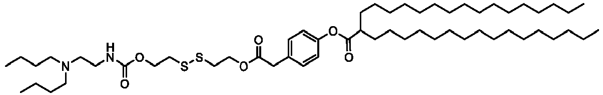 |
| compound 28 | 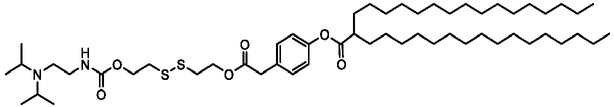 |
| compound 29 | 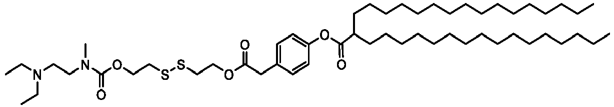 |
| compound 30 | 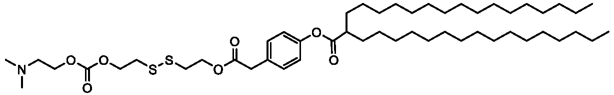 |
| compound 31 | 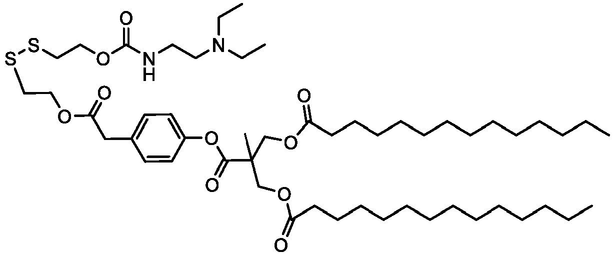 |

**[Table 2-4]**

| name | structure |
|---|---|
| compound 32 | 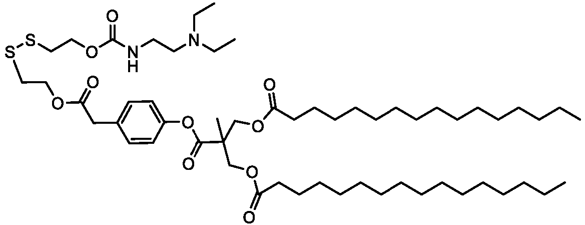 |
| compound 33 | 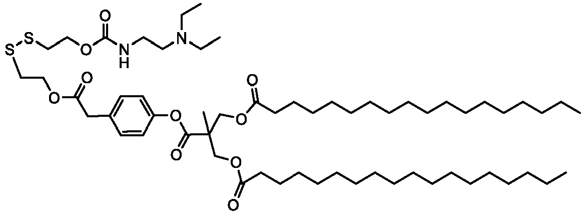 |
| compound 34 (LN-81) | 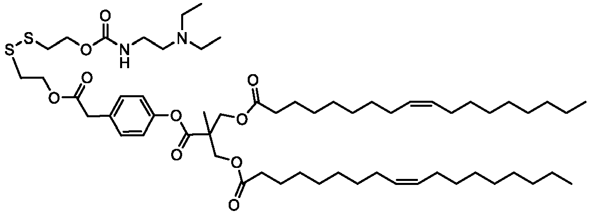 |
| compound 35 | 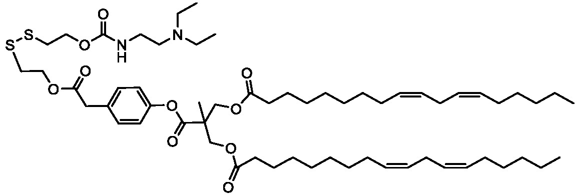 |
| compound 36 | 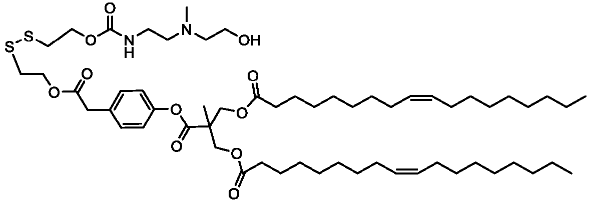 |

**[Table 2-5]**

| name | structure |
|---|---|
| compound 37 | 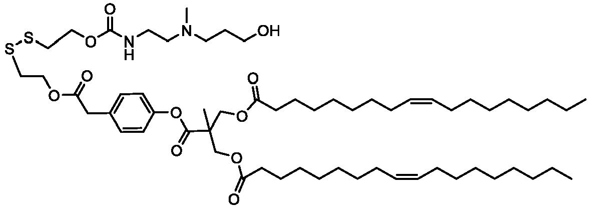 |
| compound 38 | 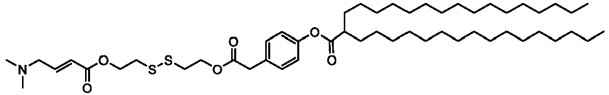 |
| compound 39 | 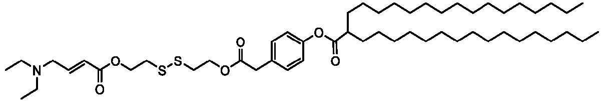 |
| compound 40 | 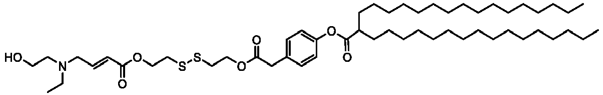 |
| compound 41 | 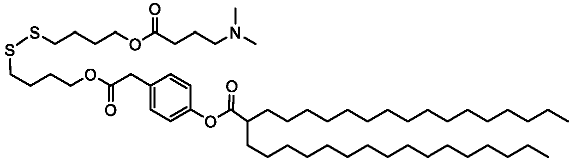 |
| compound 42 | 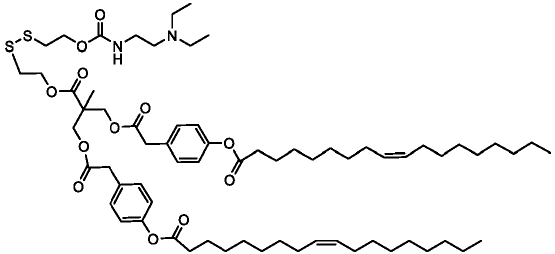 |
| compound 43 | 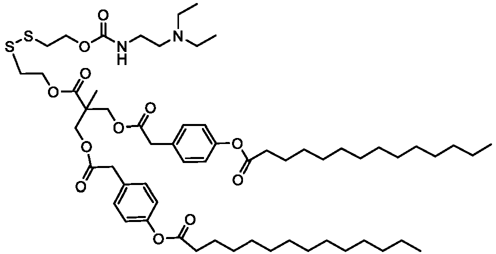 |

**[Table 2-6]**

| name | structure |
|---|---|
| compound 44 | 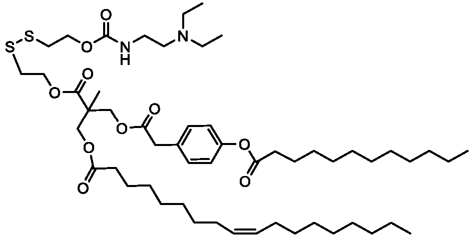 |
| compound 45 | 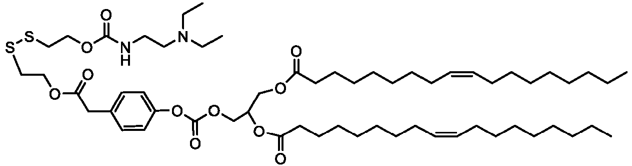 |
| compound 46 | 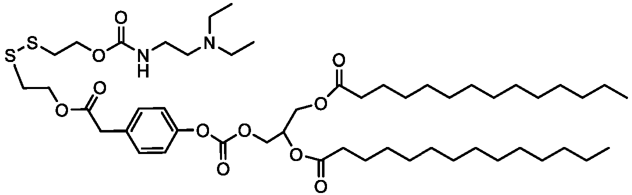 |

The production methods of ionic lipid (2) are described below.

The ionic lipid (2) has an -S-S-(disulfide) bond. Thus, the method for producing ionic lipid (2) include
(A) a method including producing a thiol represented by X- (R¹-L¹)ₖ-R^{X}-SH and a thiol represented by HS-R^{y}-L²-R²-Y, and then oxidizing and coupling same, and
(B) a method including sequentially synthetizing the necessary parts from a starting compound containing an -S-S-bond to finally obtain ionic lipid (2) and the like. The production method of ionic lipid (2) is preferably method (B).

The method (B) is described below, but the method for producing ionic lipid (2) is not limited thereto.

The raw materials, reagents, and compounds used and obtained in each step of the following production methods may each form a salt. Examples of such salt include those similar to the salts in the compounds of the aforementioned present invention.

When the compound obtained in each step is a free compound, it can be converted into the desired salt by a known method. Conversely, when the compound obtained in each step is a salt, it can be converted into the free form or another type of desired salt by a known method.

The compound obtained in each step can be used in the next reaction either as a reaction solution or after being obtained as a crude product. Alternatively, the compound obtained in each step can be appropriately purified by a general purification method, for example, extraction from reaction mixture, recrystallization, adsorption, reprecipitation, column chromatography, ion exchange chromatography and the like.

When the raw materials and reagent compounds for each step are commercially available, the commercially available products can be used as is.

In the reactions of each step, the reaction time is generally 1 min to 48 hr, preferably 10 min to 22 hr, unless otherwise specified.

In the reactions of each step, the reaction temperature is generally -78°C to 300°C, preferably -78°C to 150°C.

In the reactions of each step, the reagent is used in an amount of 0.5 equivalents to 20 equivalents, preferably 0.8 equivalents to 8 equivalents 0.5 to 20 equivalents, preferably 0.8 to 8 equivalents, relative to the substrate, unless otherwise specified. When a reagent is used as a catalyst, the reagent is used in an amount of 0.001 equivalents - 1 equivalents, preferably 0.01 equivalents - 0.4 equivalents, relative to the substrate. When a reagent also serves as a reaction solvent, the reagent is used in an amount equal to the solvent.

In the reactions of each step, unless otherwise specified, the reactions are performed without solvent or by dissolving or suspending in a suitable solvent. Specific examples of solvents include those described in the Examples, methanol, ethanol, isopropanol, tert-butyl alcohol, tetrahydrofuran, toluene, cyclohexane, hexane, heptane, N,N-dimethylformamide, N-methylpyrrolidone, chloroform, dichloromethane, acetonitrile, dimethylsulfoxide, ethyl acetate, acetone, water, and the like. Two or more of the above solvents may be mixed in an appropriate ratio.

When a base is used in the reaction of each step, examples thereof include the bases described in the Examples, sodium hydroxide, potassium hydroxide, sodium carbonate, calcium carbonate, sodium hydrogen carbonate, triethylamine, N,N-diisopropylethylamine, pyridine, 4-dimethylaminopyridine, 1,8-diazabicyclo[5.4.0]-7-undecene (DBU), imidazole, piperidine, sodium ethoxide, potassium tert-butoxide, sodium tert-butoxide, lithium hydride and the like.

When a acid or acidic catalyst is used in the reaction of each step, examples thereof include the acids and acidic catalysts described in the Examples, hydrochloric acid, sulfuric acid, nitric acid, phosphoric acid, acetic acid, trifluoroacetic acid, methanesulfonic acid, p-toluenesulfonic acid, pyridinium p-toluenesulfonate, boron trifluoride diethyl ether complex, and the like.

In each step, the protection or deprotection reaction of the functional group is performed using known reagents and methods (e.g., reagents and methods described in GREENE'S PROTECTIVE GROUPS IN ORGANIC SYNTHESIS, 4th Edition, WILEY-INTERSCIENCEM) or in accordance with the methods described in the Examples.

Protective groups for hydroxyl groups of alcohols and phenolic hydroxyl groups include, for example, ether-type protective groups such as tetrahydropyranyl ether, methoxymethyl ether, benzyl ether, p-methoxybenzyl ether, t-butyldimethylsilyl ether, t-butyldiphenylsilyl ether and the like; carboxylate-type protective groups such as acetate and the like; sulfonate-type protective groups such as methanesulfonate and the like; and carbonate-type protective groups such as t-butyl carbonate and the like.

Protective groups can be removed by known methods such as using acid, base, ultraviolet light, hydrazine, phenylhydrazine, sodium N-methyldithiocarbamate, tetrabutylammonium fluoride, palladium acetate, trialkylsilyl halide, reduction method, and the like.

When a carbonation reaction or carbamation reaction is performed in each step, reagents to be used include N,N'disuccinimidyl carbonate (DSC), 4-nitrophenyl chloroformate (pNPCl), and bases (basic salts, organic bases, and the like). When carbonation is performed, an additive such as 4-dimethylaminopyridine (DMAP) and the like may be further added.

In each step, when a mesylation reaction is performed, the reagents to be used are methanesulfonyl chloride and a base (basic salts, organic bases, and the like).

In each step, when an esterification reaction, an amidation reaction, or a urea reaction is performed, the reagents to be used include activated carboxylic acids such as acyl halides such as acid chlorides, acid anhydrides, and activated esters. Examples of activator for carboxylic acid include carbodiimide-based condensing agents such as 1-ethyl-3-(3-dimethylaminopropyl)carbodiimide hydrochloride (EDC), triazine-based condensing agents such as 4-(4,6-dimethoxy-1,3,5-triazin-2-yl)-4-methylmorpholinium chloride n-hydrate (DMT-MM), O-(7-azabenzotriazol-1-yl)-N,N,N' ,N'tetramethyluronium hexafluorophosphate (HATU), and combinations thereof. When a carbodiimide-based condensing agent is used, additives such as 1-hydroxybenzotriazole (HOBt), N-hydroxysuccinimide (HOSu), 4-dimethylaminopyridine (DMAP) and the like may be further added to the reaction.

In each step, when nucleophilic substitution reactions such as amination and the like are performed, a nucleophile (such as amines) and a base (such as basic salts or organic bases) are used as reagents. Additives such as potassium iodide (KI), tetrabutylammonium iodide (TBAI) and the like may also be added to the reaction.

Ionic lipid (2) can be produced, for example, by the following production methods. In addition, salts of the ionic lipid (2) can be obtained by appropriate mixing with an inorganic acid or an organic acid.

### Production method A: k=1, l=0, m=0, n=0, R^{x} and R^{y} are both ethylene groups

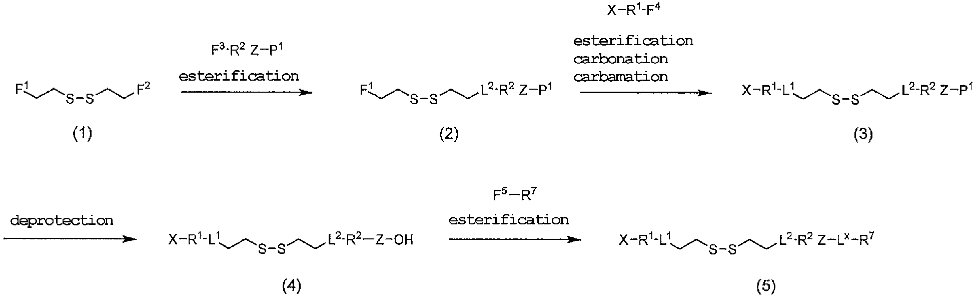

### Production method B: k=1, l=0, m=1, n=1, R^{x} and R^{y} are both ethylene groups

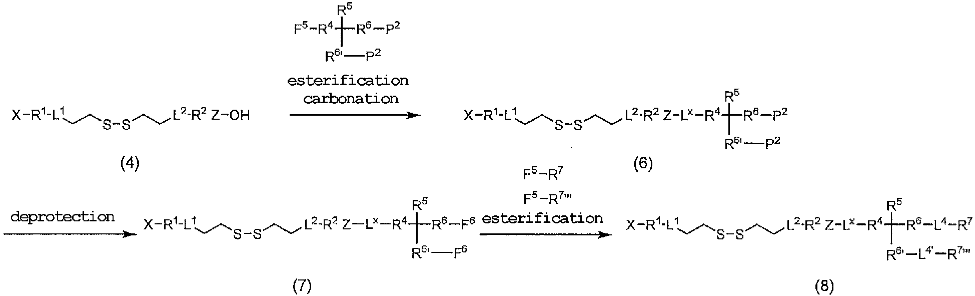

### Production method C: k=1, l=1, m=0, n=0, R^{x} and R^{y} are both ethylene groups, S¹ is hydrogen or alkyl group

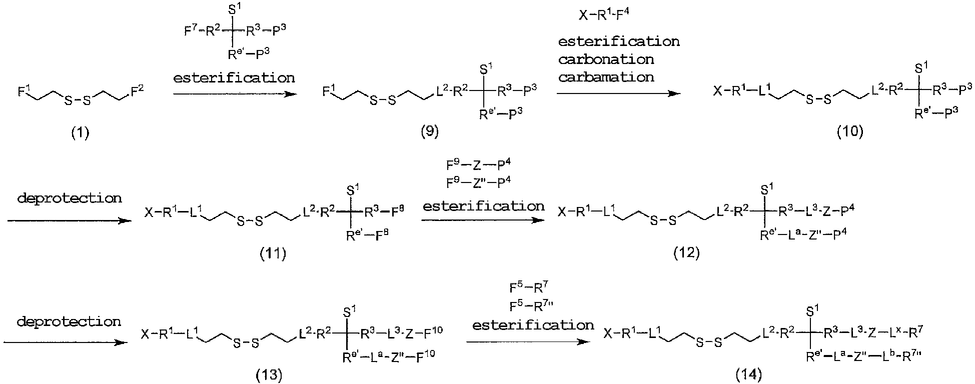

### Production method D: k=0, l=0, m=0, n=0, R^{x} and R^{y} are both ethylene groups

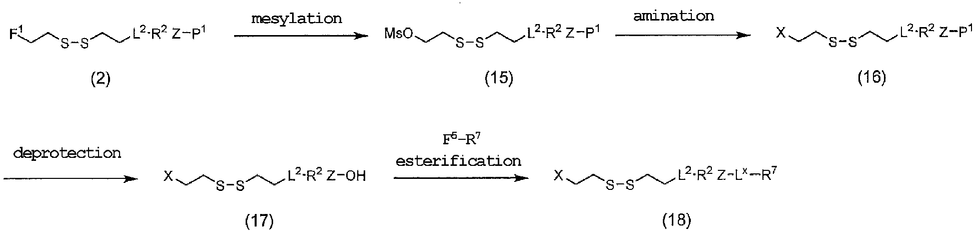

In the above-mentioned formula, F¹ to F¹⁰ each independently represent a reactive functional group, and P¹ to P⁴ each independently represent a protecting group.

Specific production methods are described in Production Examples 1 to 46 below. A person skilled in the art can produce the desired ionic lipid (2) by appropriately selecting raw materials and performing reactions in accordance with the methods described in Production Examples 1 to 46.

### Phospholipid

Phospholipids can be used as a lipid membrane constituting component of lipid nanoparticles.

Examples of the phospholipid include 1,2-diacyl-sn-glycero-3-phosphocholine (PC), 1,2-diacyl-sn-glycero-3-phosphatidylethanolamine (PE), 1,2-diacyl-sn-glycero-3-phosphatidylserine (PS), 1,2-diacyl-sn-glycero-3-phosphatidylglycerol (PG), 1,2-diacyl-sn-glycero-3-phosphatidic acid (PA), and lyso forms of these, specifically, 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl-sn-glycero-3-phosphocholine (DLoPC), 1,2-dierucoyl-sn-glycero-3-phosphocholine (DEPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), 1-stearoyl-2-oleoyl-sn-glycero-3-phosphocholine (SOPC), and these PCs appropriately converted to PE, PS, PG, or PA can be used.

Phospholipid to be used in the present invention is preferably PC or PE, further preferably DOPC, DSPC, DEPC, POPC, DOPE (1,2-dioleoyl-sn-glycero-3-phosphoethanolamine), or POPE (1-palmitoyl-2-oleoyl-sn-glycero-3-phosphoethanolamine), particularly preferably DOPC, DSPC, DEPC.

### Sterol

Sterol can be used as a component that regulates fluidity of the lipid membrane of lipid nanoparticles. Examples of the sterol include cholesterol, lanosterol, phytosterol, zymosterol, zymostenol, desmosterol, stigmastanol, dihydrolanosterol, and 7-dehydrocholesterol, preferably cholesterol, lanosterol, and phytosterol, further preferably cholesterol.

### PEG lipid

PEG lipids are used as stabilizers that coat the surface of lipid nanoparticles with hydrophilic polyethylene glycol (PEG) and suppress the aggregation of particles, or used to suppress the interaction between biological components and particles when administered to a living organism.

The PEG region can have any molecular weight. In some embodiments, the PEG region has a molecular weight of 200 - 10,000 Da and may be linear or branched.

Examples of the PEG lipid include PEG-phospholipid, PEG-ceramide, PEG-diacylglycerol, and PEG-cholesterol, preferably diacylglycerol PEG with a PEG molecular weight of 1,000 - 10,000, further preferably dimyristoylglycerol PEG or distearoylglycerol PEG with a PEG molecular weight of 1,000 - 10,000.

### Acidic buffer

A buffer having a buffering action in the acidic region can be used. Specifically, HCl/KCl buffer, p-toluenesulfonic acid/sodium p-toluenesulfonate buffer, tartaric acid/NaOH buffer, citric acid/NaOH buffer, phthalic acid HK/HCl buffer, glycine/HCl buffer, trans-aconitic acid/NaOH buffer, formic acid/sodium formate buffer, citric acid/sodium citrate buffer, 3,3-dimethylglutaric acid/NaOH buffer, 3,3-dimethylglutaric acid/NaOH/0.1M NaCl buffer, phenylacetic acid/sodium phenylacetate buffer, acetic acid/sodium acetate buffer, succinic acid/NaOH buffer, phthalic acid HK/NaOH buffer, sodium cacodylate/HCl buffer, maleic acid HNa/NaOH buffer, maleic acid/Tris/NaOH buffer, phosphate buffer, KH₂PO₄/NaOH buffer, imidazole/HCl buffer, s-collidine (2,4,6-trimethylpyridine)/HCl buffer, triethanolamine HCl/NaOH buffer, sodium 5,5-diethylbarbiturate/HCl buffer, N-methylmorpholine/HCl buffer, sodium pyrophosphate/HCl buffer, MES buffer, malic acid buffer, ADA buffer, PIPES buffer, ACES buffer, HEPES, BES, Bis-Tris buffer, Bis-Tris propane buffer, anhydrous sodium carbonate buffer, glycylglycine buffer, MOPS, MOPSO, and TES can be mentioned.

It is preferably an acid buffer having a buffering action at pH 1 to 6.5, more preferably pH 3 to 6.5. Examples thereof include tartaric acid/NaOH buffer, citric acid/NaOH buffer, phthalic acid HK/HCl buffer, glycine/HCl buffer, trans-aconitic acid/NaOH buffer, formic acid/sodium formate buffer, citric acid/sodium citrate buffer, 3,3-dimethylglutaric acid/NaOH buffer, 3,3-dimethylglutaric acid/NaOH/0.1M NaCl buffer, phenylacetic acid/sodium phenylacetate buffer, acetic acid/sodium acetate buffer, succinic acid/NaOH buffer, phthalic acid HK/NaOH buffer, sodium cacodylate/HCl buffer, maleic acid HNa/NaOH buffer, maleic acid/Tris/NaOH buffer, phosphate buffer, KH₂PO₄/NaOH buffer, MES buffer, malic acid buffer, phthalic acid buffer, maleic acid buffer, succinic acid buffer, tartaric acid buffer, citrate buffer, Bis-Tris buffer, glycylglycine buffer and the like, further preferably malic acid buffer or MES buffer.

### Preparation method of lipid nanoparticles

In the method of the present invention, a suspension of lipid nanoparticles not containing nucleic acid is prepared by mixing an alcohol solution containing an ionic lipid, sterol and PEG lipid with an acidic buffer having a buffering action at pH 1 to 6.5. Any operation to induce organization can be used to prepare lipid nanoparticles. Examples of the alcohol include ethanol, tert-butanol and the like.

Examples of the "operation to induce organization" for preparing lipid nanoparticles include methods known per se such as an alcohol dilution method using a micro flow path or vortex, a simple hydration method, sonication, heating, vortex, an ether injecting method, a French press method, a cholic acid method, a Ca²⁺ fusion method, a freeze-thaw method, a reversedphase evaporation method and the like, preferably an alcohol dilution method using a micro flow path or vortex, further preferably an alcohol dilution method using a micro flow path. Preparation of particles by the alcohol dilution method using a micro flow path can be performed using, for example, NanoAssemblr (Precision NanoSystems). The buffer in the external aqueous phase of the prepared lipid nanoparticles can be replaced by an operation such as ultrafiltration, dialysis, or dilution. In a preferred embodiment, after preparation of the suspension of the lipid nanoparticles, the external aqueous phase can be exchanged for another acidic buffer having a buffering action at pH 1 to 6.5 (preferably pH 3 to 6.5) by an operation such as ultrafiltration, dialysis, dilution, and the like. Also, sugars such as monosaccharide, sugar alcohol, disaccharide, oligosaccharide, polysaccharides and the like can be added to the lipid nanoparticle suspension. As the sugar, disaccharides are preferred, and sucrose is more preferred. The concentration of the sugar is preferably 0 to 320 mg/mL, more preferably 0 to 160 mg/mL, as a final concentration.

### Storage conditions for lipid nanoparticles

Lipid nanoparticles not containing nucleic acid may be stored as a liquid in a temperature range of 0°C to 50°C, or may be stored frozen in a temperature range of -80°C to 0°C.

When stored as a liquid, the storage temperature is preferably 0°C to 30°C, further preferably 0°C to 10°C, most preferably 0°C to 5°C, from the aspect of the stability of the components.

When stored frozen, the storage temperature is preferably -80°C to -10°C, further preferably -80°C to -20°C, from the aspect of the stability of the components.

When stored frozen, the nanoparticles are thawed at 0°C to 100°C before use. From the aspect of the stability of the components, the thawing temperature is preferably 0°C to 95°C, more preferably 0°C to 50°C, further preferably 0°C to 30°C, most preferably 0°C to 10°C.

Freezing and thawing are preferably performed under atmospheric pressure.

### Nucleic acid addition step

The nucleic acid addition step is a step of preparing nucleic acid-encapsulated lipid nanoparticles by mixing the lipid nanoparticles not containing a nucleic acid prepared in the previous step with an aqueous solution containing nucleic acid without lyophilizing them.

Note that "without lyophilization" means that the nucleic acid addition step is performed immediately after the preparation step of lipid nanoparticles not containing nucleic acid. The preparation step of lipid nanoparticles not containing nucleic acid includes storing the lipid nanoparticles not containing nucleic acid as a liquid, or freezing and storing the lipid nanoparticles not containing nucleic acid, and then thawing them.

As for the amount of nucleic acid, an aqueous solution containing nucleic acid such that the ratio of total lipid and nucleic acid contained in the lipid nanoparticles is, for example, total lipid/nucleic acid = 1 - 1000 nmol/µg, preferably total lipid/nucleic acid = 50 - 500 nmol/µg, is added, and nucleic acid-encapsulated lipid nanoparticles can be prepared by mixing them by pipetting or vortex. In addition, alcohol can be added in order to increase the nucleic acid encapsulation rate, and methanol, ethanol, n-butanol and t-butanol, preferably ethanol, can be used as the alcohol. The concentration of alcohol in the aqueous solution containing nucleic acid is preferably 0 to 50 v/v%, more preferably 0 to 30 v/v%, further preferably 0 to 25 v/v%. In addition, in order to increase the efficiency of nucleic acid encapsulation in the nucleic acid addition step, an aqueous solution containing nucleic acid or further alcohol may be added to the lipid nanoparticles not containing nucleic acid in a liquid or frozen state, followed by incubation. The incubation conditions are, for example, 0 to 100°C for 0 to 120 min, preferably 0 to 95°C for 0 to 60 min.

As the aqueous solution containing nucleic acid, an acidic buffer containing nucleic acid is preferably used. As the acidic buffer, a buffer having a buffering action in the acidic range can be used, and is preferably an acidic buffer having a buffering action at pH 1 to 6.5, more preferably pH 3 to 6.5. Specifically, the same acidic buffer as those exemplified above as the acidic buffers used in the preparation of lipid nanoparticles can be used, and examples thereof include MES buffer, malic acid buffer, phthalic acid buffer, maleic acid buffer, succinic acid buffer, tartaric acid buffer, citrate buffer, and the like.

### Step of exchanging external aqueous phase with neutral buffer

Nucleic acid-encapsulated lipid nanoparticles that can be used as a nucleic acid-introducing agent can be prepared by exchanging the external aqueous phase of the nucleic acid-encapsulated lipid nanoparticles obtained in the nucleic acid addition step with a neutral buffer.

Examples of the method of exchanging the external aqueous phase with a neutral buffer include methods using dialysis, ultrafiltration or dilution.

Examples of the neutral buffer include phosphate buffered saline (PBS), Tris-HCl buffer, ADA, PIPES, PIPES sesquisodium, ACES, MOPS, BES, MOPS, TES, HEPES, TAPSO, POPSO, HEPSO and the like. The pH of the neutral buffer is 6 to 8.

A nucleic acid can be introduced into a cell in vivo and/or in vitro by contacting nucleic acid-encapsulated lipid nanoparticles prepared by the method of the present invention with the cell. Therefore, the present invention provides methods for introducing the nucleic acid into the cells in vivo and/or ex vivo.

The method of the present invention can introduce any nucleic acid into a cell. Examples of the kind of nucleic acid include, but are not limited to, DNA, RNA, chimera nucleic acid of RNA, DNA/RNA hybrid and the like. While any nucleic acid having 1 to 3 chains can be used, it is preferably a single strand or double strand. The nucleic acid may be other type of nucleotide such as N-glycoside of purine or pyrimidine base or other oligomer having a non-nucleotide backbone (e.g., commercially available peptide nucleic acid (PNA) etc.), other oligomer containing a special bond (said oligomer comprising base pairing or a nucleotide having a configuration permitting attachment of base, which are found in DNA and RNA) and the like. Furthermore, it may be a nucleic acid added with known modification, for example, a nucleic acid with a label known in the field, a nucleic acid with a cap, a methylated nucleic acid, one or more natural nucleotides substituted by an analog, a nucleic acid with intramolecular nucleotidyl modification, for example, a nucleic acid with non-charge bond (e.g., methylphosphonate, phosphotriester, phosphoramidate, carbamate and the like), a nucleic acid with a charged bond or sulfurcontaining bond (e.g., phosphorothioate, phosphorodithioate and the like), for example, a nucleic acid with a side chain group such as protein (nuclease, nuclease inhibitor, toxin, antibody, signal peptide, poly-L-lysine and the like), sugar (e.g., monosaccharide and the like) and the like, a nucleic acid with an intercalating compound (e.g., acridine, psoralen and the like), a nucleic acid with a chelate compound (e.g., metal, radioactive metal, boron, oxidative metal and the like), a nucleic acid containing an alkylating agent, or a nucleic acid with a modified bond (e.g., α anomer-type nucleic acid and the like).

The type of the DNA that can be used in the present invention is not particularly limited, and can be selected as appropriate according to the purpose of use. For example, plasmid DNA, cDNA, antisense DNA, chromosomal DNA, PAC, BAC, CpG oligosaccharide, and the like can be mentioned. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. For example, siRNA, miRNA, shRNA, antisense RNA, messenger RNA (mRNA), single strand RNA genome, double strand RNA genome, RNA replicon, transfer RNA, ribosomal RNA and the like can be mentioned, with preference given to siRNA, miRNA, shRNA, mRNA, antisense RNA, and RNA replicon.

The nucleic acid used in the present invention is preferably purified by a method generally used by those of ordinary skill in the art.

The nucleic acid-encapsulated lipid nanoparticles produced by the method of the present invention can be administered in vivo for the purpose of, for example, prevention and/or treatment of diseases. Therefore, the nucleic acid to be used in the present invention is preferably one having a preventive and/or therapeutic activity against a given disease (prophylactic/therapeutic nucleic acid). Examples of such nucleic acid include nucleic acids and the like used for so-called gene therapy.

The nucleic acid-encapsulated lipid nanoparticles produced by the method of the present invention can be used as a drug delivery system for selectively delivering a nucleic acid and the like into a particular cell, and is useful for, for example, DNA vaccines by introducing antigen gene into dendritic cells, gene therapy drugs for tumor, nucleic acid pharmaceutical products that suppress expression of target genes by utilizing RNA interference, and the like. Therefore, the present invention provides a production method of a pharmaceutical composition.

The particle size of the lipid nanoparticles encapsulating the nucleic acid is not particularly limited, and is preferably 10 nm - 500 nm, more preferably 30 nm - 300 nm. The particle size can be measured by using a particle size distribution measuring device such as Zetasizer Nano (Malvern) or the like. The particle size of the lipid nanoparticles can be appropriately adjusted by the production method of the lipid nanoparticles.

The surface charge (zeta potential) of the lipid nanoparticles encapsulating the nucleic acid is not particularly limited and preferably -15 to +15 mV, more preferably -10 to +10 mV. In conventional transgene, particles electrically charged to have a plus surface potential have been mainly used. This is useful as a method for promoting electrostatic interactions with heparin sulfate on the negatively-charged cell surface to enhance uptake into cells. However, the positive surface charge may suppress, in the cell, release of nucleic acid from the carrier due to the interaction with a nucleic acid to be delivered or protein synthesis due to the interaction between mRNA and a nucleic acid to be delivered. This problem can be solved by adjusting the surface charge to fall within the above-mentioned range. The surface charge can be measured using a zeta potential measuring apparatus such as Zetasizer Nano and the like. The surface charge of the lipid nanoparticles can be adjusted by the composition of the constituent component of the lipid nanoparticles.

The step of contacting the lipid nanoparticles encapsulating the nucleic acid with the cell in vitro is specifically explained below.

The cells are suspended in a suitable medium several days before contact with the lipid nanoparticles, and cultured under appropriate conditions. At the time of contact with the lipid nanoparticles, the cells may or may not be in a proliferative phase.

The culture medium on contact may be a serum-containing medium or a serum-free medium, wherein the serum concentration of the medium is preferably not more than 30 wt%, more preferably not more than 20 wt%, since when the medium contains excess protein such as serum and the like, the contact between the lipid nanoparticles and the cell may be inhibited.

The cell density on contact is not particularly limited, and can be appropriately determined in consideration of the kind of the cell and the like. It is generally within the range of 1×10⁴ - 1×10⁷ cells/mL.

A suspension of the aforementioned lipid nanoparticles encapsulating the nucleic acid is added to the thus-prepared cells. The amount of the suspension to be added is not particularly limited, and can be appropriately determined in consideration of the cell number and the like. The concentration of the lipid nanoparticles to be contacted with the cells is not particularly limited as long as the desired introduction of the nucleic acid into the cells can be achieved. The lipid concentration is generally 1 - 300 nmol/mL, preferably 10 - 200 nmol/mL, and the concentration of the nucleic acid is generally 0.01 - 100 µg/mL, preferably 0.05 - 10 µg/mL.

After the aforementioned suspension is added to cells, the cells are cultured. The temperature, humidity and CO₂ concentration during culturing are appropriately determined in consideration of the kind of the cell. When the cell is derived from a mammal, generally, the temperature is about 37°C, humidity is about 95% and CO₂ concentration is about 5%. While the culture period can also be appropriately determined in consideration of the conditions such as the kind of the cell and the like, it is generally a range of 0.1 to 96 hr, preferably a range of 0.2 to 72 hr, more preferably a range of 0.5 to 48 hr. When the above-mentioned culture time is too short, the nucleic acid is not sufficiently introduced into the cells, and when the culture time is too long, the cells may become weak.

By the above-mentioned culture, the nucleic acid is introduced into cells. The culture is further continued preferably by exchanging the medium with a fresh medium, or adding a fresh medium to the medium. When the cell is a mammal-derived cell, the fresh medium preferably contains a serum or nutrition factor.

As mentioned above, a nucleic acid can be introduced into cells not only outside the body (in vitro) but also in the body (in vivo) by using lipid nanoparticles encapsulating the nucleic acid. That is, by administration of the lipid nanoparticles encapsulating the nucleic acid to a subject, the lipid nanoparticles reaches and contacts with the target cells, and the nucleic acid encapsulated in the lipid nanoparticles is introduced into the cells in vivo. The subject to which the lipid nanoparticles can be administered is not particularly limited and, for example, vertebrates such as mammals (e.g., human, monkey, mouse, rat, hamster, bovine etc.), birds (e.g., chicken, ostrich etc.), amphibia (e.g., frog etc.), fishes (e.g., zebrafish, rice-fish etc.) and the like, invertebrates such as insects (e.g., silk moth, moth, Drosophila etc.) and the like, plants and the like can be mentioned. The subject of administration of the lipid nanoparticles encapsulating the nucleic acid is preferably human or other mammal.

The kind of the target cell is not particularly limited, and a nucleic acid can be introduced into cells in various tissues (e.g., liver, kidney, pancreas, lung, spleen, heart, blood, muscle, bone, brain, stomach, small intestine, large intestine, skin, adipose tissue, lymph node, tumor, etc.) by using the lipid nanoparticles encapsulating the nucleic acid.

The administration method of the lipid nanoparticles into which a nucleic acid and/or a compound other than nucleic acid is introduced to a target (e.g., vertebrate, invertebrate and the like) is not particularly limited as long as the lipid nanoparticles reaches and contacts with the target cells, and the compound introduced into the lipid nanoparticles can be introduced into the cell, and an administration method known per se (e.g., oral administration, parenteral administration (e.g., intravenous administration, intramuscular administration, topical administration, transdermal administration, subcutaneous administration, intraperitoneal administration, spray etc.) etc.) can be appropriately selected in consideration of the kind of the compound to be introduced, the kind and the site of the target cell and the like. The dose of the lipid nanoparticles is not particularly limited as long as the introduction of the compound into the cells can be achieved, and can be appropriately selected in consideration of the kind of the subject of administration, administration method, the kind of the compound to be introduced, the kind and the site of the target cell and the like.

When the lipid nanoparticles encapsulating the nucleic acid are used as a nucleic acid-introducing agent, they can be formulated according to a conventional method.

When the nucleic acid-introducing agent is provided as a reagent for studies, the lipid nanoparticles encapsulating the nucleic acid may be provided as it is as the nucleic acid-introducing agent of the present invention, or the nucleic acid-introducing agent of the present invention may be provided as a sterile solution or suspension with, for example, water or other physiologically acceptable liquid (e.g., water-soluble solvent (e.g., malic acid buffer etc.), organic solvent (e.g., ethanol, methanol, DMSO, tert-butanol and the like), or a mixture of aqueous solvent and organic solvent etc.). The nucleic acid-introducing agent of the present invention may appropriately contain physiologically acceptable additive (e.g., excipient, vehicle, preservative, stabilizer, binder etc.), which are known per se.

When the nucleic acid-introducing agent is provide as a medicament, the lipid nanoparticles encapsulating the nucleic acid may be used as it is as the nucleic acid-introducing agent of the present invention or the nucleic acid-introducing agent of the present invention may be produced as an oral preparation (for example, tablet, capsule etc.) or a parenteral agent (for example, injection, spray etc.), preferably a parenteral agent (more preferably, injection) by blending with a pharmaceutically acceptable known additives such as carrier, flavor, excipient, vehicle, preservative, stabilizer, binder and the like in a conventionally-admitted unit dosage form required for practicing preparation formulation.

The nucleic acid-introducing agent of the present invention can be formulated into a preparation not only for adults but also for children.

### [Example]

The Examples of the present invention are explained in further detail in the following, but the present invention is not limited in any way by the Examples.

In the following Examples, the ionic lipids represented by the formula (1) or the formula (2) are shown by the names listed in the aforementioned Tables. Nucleic acid-encapsulated lipid nanoparticles may be referred to as "nucleic acid-encapsulated nanoparticles". The abbreviations used in the following Examples mean the following.
Chol: cholesterol
DMG-PEG2000: 1,2-dimyristoyl-rac-glycerol, methoxypolyethylene glycol (number average molecular weight (Mn) of PEG: 2000)
DOPC: 1,2-dioleoyl-sn-glycero-3-phosphocholine
DSPC: 1,2-distearoyl-sn-glycero-3-phosphocholine
DEPC: 1,2-dierucoyl-sn-glycero-3-phosphocholine
MES: 2-morpholinoethanesulfonic acid
PBS: phosphate buffered saline
DDW: deionized distilled water
DCM: dichloromethane
THF: tetrahydrofuran
IPA: 2-propanol
DHP: 3,4-dihydro-2H-pyran
PPTS: pyridinium p-toluenesulfonate
THP: 2-tetrahydropyranyl
DMAP: 4-dimethylaminopyridine
DMT-MM: 4-(4,6-dimethoxy-1,3,5-triazin-2-yl)-4-methylmorpholinium chloride n-hydrate
DSC: di(N-succinimidyl) carbonate
EDC: 1-(3-dimethylaminopropyl)-3-ethylcarbodiimide hydrochloride
TEA: triethylamine
TBAI: tetrabutylammonium iodide
DIPEA: N,N-diisopropylethylamine
NPM: 4-phenylmorpholine
MsCl: methanesulfonyl chloride
Ms: methanesulfonyl
pNPCl: 4-nitrophenyl chloroformate

### Example 1: Influence of buffer

SS-OP and MC3 were used as ionic lipids. When SS-OP was used as the ionic lipid, the lipid composition was SS-OP:DOPC:Chol:DMG-PEG2000=52.5:7.5:40:1.5 in molar ratio, and when MC3 was used as the ionic lipid, the lipid composition was MC3:DSPC:Chol:DMG-PEG2000=50:10:38.5:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 5) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 5) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 5), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipids 20 mM, 15 µL) was mixed with a buffer solution (nucleic acid concentration 1.5 µg/135 µL, 135 µL) of MES (pH 5), phthalic acid (pH 5), maleic acid (pH 5), succinic acid (pH 5), DL-malic acid (pH 5), DL-tartaric acid (pH 5), or citric acid (pH 5) containing mRNA encoding luciferase (CleanCap (registered trademark) FLuc mRNA - (L-7602)) while diluting under stirring conditions using a vortex mixer, and incubated at 37°C for 5 min using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, mRNA was encapsulated in all buffer solutions of MES, phthalic acid, maleic acid, succinic acid, DL-malic acid, DL-tartaric acid, and citric acid, and no difference in the encapsulation rate was observed (see Table 3, Fig. 1 and Fig. 2). The particle size is shown as Z-Ave (Z-average) and Number Mean (number average) (same in the following).

**[Table 3]**

| Sample | | | encapsulation rate (%) | Z-Ave (d.nm) | Number Mean (d.nm) | PdI |
|---|---|---|---|---|---|---|
| 1 | SS-OP | MES | 91.5 | 124.7 | 74.8 | 0.180 |
| 2 | | phthalic acid | 96.6 | 123.7 | 80.1 | 0.155 |
| 3 | | maleic acid | 97.1 | 124.9 | 74.7 | 0.152 |
| 4 | | succinic acid | 95.9 | 124.7 | 79.1 | 0.154 |
| 5 | | DL-malic acid | 94.3 | 126.0 | 75.4 | 0.138 |
| 6 | | DL-tartaric acid | 92.0 | 125.7 | 75.2 | 0.135 |
| 7 | | citric acid | 95.6 | 127.2 | 78.2 | 0.157 |
| 8 | MC3 | MES | 97.9 | 78.1 | 37.8 | 0.254 |
| 9 | | phthalic acid | 99.4 | 93.4 | 54.2 | 0.207 |
| 10 | | maleic acid | 100.0 | 89.3 | 38.6 | 0.261 |
| 11 | | DL-tartaric acid | 99.9 | 87.5 | 43.6 | 0.264 |
| 12 | | DL-malic acid | 98.7 | 89.2 | 40.9 | 0.300 |
| 13 | | succinic acid | 91.7 | 100.6 | 67.9 | 0.194 |
| 14 | | citric acid | 99.5 | 90.1 | 56.5 | 0.164 |

### Example 2: Influence of sucrose concentration and incubation temperature

SS-OP was used as ionic lipid. The lipid composition was SS-OP:DOPC:Chol:DMG-PEG2000=52.5:7.5:40:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution or DNase/RNase-Free Distilled Water was added to a sucrose final concentration of 0 mg/mL, 160 mg/mL or 320 mg/mL to prepare a particle solution.

The particle solution (total lipid 20 mM, 15 µL) stored at 4°C (hereinafter also referred to as liquid) or the particle solution frozen at -80°C and then thawed at 4°C (hereinafter also referred to as frozen-thawed particles) was mixed with a solution containing luciferase-encoding mRNA (CleanCap (registered trademark) FLuc mRNA - (L-7602)) (nucleic acid concentration 1.5 µg/135 µL, buffer condition MES (pH 5), 135 µL) while diluting under stirring conditions using a vortex mixer, and the liquid samples of each sucrose concentration were incubated for 5 min at 95°C, 75°C, 55°C, 35°C, and room temperature using an aluminum block incubator. Among them, the sample with a sucrose concentration of 160 mg/mL was added and incubated on ice for 5 min. The frozen and thawed particles were incubated for 5 min at 95°C, 35°C, and room temperature. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, mRNA was encapsulated at any incubation temperature, and no difference in the encapsulation rate was observed. In addition, mRNA was encapsulated at any sucrose concentration, and no difference in the encapsulation rate was observed. The particle size of the obtained nucleic acid-encapsulated nanoparticles was large when incubated at 95°C, and no difference in particle size was observed at other temperatures (75°C, 55°C, 35°C, or room temperature) (see Table 4, Fig. 3 and Fig. 4).

The nucleic acid-encapsulated nanoparticles obtained were applied to HeLa cells to evaluate the nucleic acid introduction efficiency. HeLa cells were seeded in D-MEM medium (high glucose) (containing L-glutamine and phenol red) (containing 10% FBS and 1% penicillin-streptomycin solution added) at 5×10³ cells/100 µL in a 96-well flat-bottom transparent plate. The obtained nucleic acid-encapsulated particles were added such that the nucleic acid concentration in the medium was 0.02 µg/4 µL. After 16 hr, the amount of luciferase protein introduced was evaluated using the amount of luminescence. 100 µL of Steady-Glo (registered trademark) was added, and the cells were lysed by shaking for 5 min, and the amount of luminescence was quantified using a GloMax 20/20 luminometer. As a result, the introduction of luciferase protein was confirmed at any incubation temperatures, and no difference in in vitro activity was observed. Furthermore, the frozen-thawed particles showed higher activity than the liquid (see Fig. 5).

**[Table 4]**

| Sample | production method | sucrose concentration | temperature (°C) | encapsulation rate (%) | Z-Ave (d.nm) | Number Mean (d.nm) | PdI |
|---|---|---|---|---|---|---|---|
| 1 | | 160 mg/mL | 95 | 90.2 | 149.9 | 116.1 | 0.091 |
| 2 | | | 75 | 90.5 | 136.8 | 97.45 | 0.106 |
| 3 | | | 55 | 93.3 | 134.8 | 91.51 | 0.124 |
| 4 | | | 35 | 92.4 | 132.4 | 92.97 | 0.117 |
| 5 | | | room temperature | 91.5 | 135.1 | 95.15 | 0.095 |
| 6 | | | on ice | 92.1 | 130.9 | 83.14 | 0.128 |
| 7 | | 0 mg/mL | 95 | 92.0 | 147.3 | 111.4 | 0.086 |
| 8 | liquid | | 75 | 93.7 | 136 | 85.81 | 0.107 |
| 9 | | | 55 | 92.7 | 132.5 | 100.9 | 0.104 |
| 10 | | | 35 | 93.0 | 133.2 | 102.8 | 0.067 |
| 11 | | | room temperature | 93.2 | 133.3 | 100.1 | 0.075 |
| 12 | | 320 mg/mL | 95 | 91.8 | 152.7 | 115 | 0.08 |
| 13 | | | 75 | 92.8 | 137.5 | 92.7 | 0.114 |
| 14 | | | 55 | 96.8 | 134.3 | 98.42 | 0.109 |
| 15 | | | 35 | 92.1 | 132.5 | 86.81 | 0.123 |
| 16 | | | room temperature | 96.1 | 133.9 | 102.3 | 0.069 |
| 17 | frozen-thawed particles | 160 mg/mL | 95 | 85.5 | 169.5 | 123.3 | 0.101 |
| 18 | | | 35 | 88.8 | 152.3 | 111.4 | 0.125 |
| 19 | | | room temperature | 86.1 | 150.1 | 102.2 | 0.119 |

### Example 3: Influence of incubation time

SS-OP was used as ionic lipid. The lipid composition was SS-OP:DOPC:Chol:DMG-PEG2000=52.5:7.5:40:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipid 20 mM, 15 µL) was mixed with a solution containing luciferase-encoding mRNA (CleanCap (registered trademark) FLuc mRNA - (L-7602)) (nucleic acid concentration 1.5 µg/135 µL, buffer condition MES (pH 6), 135 µL) while diluting under stirring conditions using a vortex mixer, and the mixture was incubated for 60 min, 30 min, 15 min, 10 min, 5 min or 1 min at room temperature using an aluminum block incubator. After incubation, 150 µL of PBS was added to the solution to obtain nucleic acid-encapsulated nanoparticles. Alternatively, for comparison, 150 µL of PBS was first added to the particle solution (total lipids 20 mM, 15 µL), and then mRNA encoding luciferase (same as above) was added to a final concentration of 1.5 µg/300 µL.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, mRNA was encapsulated at any incubation time, and no difference in the encapsulation rate was observed. When PBS was added to the particle solution first (PBS→mRNA), a low encapsulation rate was obtained (see Fig. 6 and Fig. 7).

The nucleic acid-encapsulated nanoparticles obtained were applied to HeLa cells to evaluate the nucleic acid introduction efficiency. HeLa cells were seeded in D-MEM medium (high glucose) (containing L-glutamine and phenol red) (containing 10% FBS and 1% penicillin-streptomycin solution added) at 5×10³ cells/100 µL in a 96-well flat-bottom transparent plate. The obtained nucleic acid-encapsulated particles were added such that the nucleic acid concentration in the medium was 0.02 µg/4 µL. Luciferin was added to the medium at 0.1 mM, and the amount of luciferase protein introduced was evaluated over time using the amount of luminescence. HeLa cells cultured in a medium containing nucleic acid-encapsulated nanoparticles and luciferin were placed in an incubator-type luminometer (Kronos), and the cumulative amount of luminescence was measured for 2 min every hour. As a result, the efficiency of nucleic acid introduction did not change depending on the incubation time. When PBS was added to the particle solution first (PBS→mRNA), a low nucleic acid introduction efficiency was obtained (see Fig. 8 and Fig. 9).

### Example 4: Influence of pH

SS-OP, SS-OC, MC3 or DODAP was used as ionic lipid. When SS-OP or SS-OC was used, DOPC, Chol and DMG-PEG2000 were used as other lipids, and when MC3 or DODAP was used, DSPC, Chol and DMG-PEG2000 were used as other lipids. The molar ratio of SS-OP or SS-OC:DOPC:Chol used was 52.5:7.5:40, and 1.5 mol% of DMG-PEG2000 was used relative to the total of SS-OP or SS-OC, DOPC and Chol. The molar ratio of MC3 or DODAP:DSPC:Chol:DMG-PEG2000 used was 50:10:38.5:1.5.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 5, pH 5.5, pH 6, pH 6.5 or pH 7) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 5, pH 5.5, pH 6, pH 6.5 or pH 7) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 5, pH 5.5, pH 6, pH 6.5 or pH 7), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipid 20 mM, 15 µL) was mixed with a solution containing luciferase-encoding mRNA (CleanCap (registered trademark) FLuc mRNA - (L-7602)) (nucleic acid concentration 1.5 µg/135 µL, buffer condition MES (pH 6), 135 µL) while diluting under stirring conditions using a vortex mixer, and the mixture of each pH and ionic lipid combination was incubated for 5 min at 37°C using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, SS-OP and MC3 were encapsulated up to pH 6, and the encapsulation rate decreased at pH 6.5 and pH 7. On the other hand, SS-OC and DODAP were encapsulated up to pH 5.5, and the encapsulation rate decreased at pH 6, pH 6.5, and pH 7 (see Fig. 10 and Fig. 11).

### Example 5: Influence of salt concentration

SS-OP and MC3 were used as ionic lipids. When SS-OP was used as the ionic lipid, the lipid composition was SS-OP:DOPC:Chol:DMG-PEG2000=52.5:7.5:40:1.5 in molar ratio, and when MC3 was used as the ionic lipid, the lipid composition was MC3:DSPC:Chol:DMG-PEG2000=50:10:38.5:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipid 20 mM, 15 µL) was mixed with a solution containing luciferase-encoding mRNA (CleanCap (registered trademark) FLuc mRNA - (L-7602)) (nucleic acid concentration 1.5 µg/135 µL, buffer condition MES (pH 6) containing 0 mM, 1 mM, 10 mM, 30 mM, 50 mM, 100 mM, 150 mM or 300 mM NaCl, 135 µL) while diluting under stirring conditions using a vortex mixer, and the mixture of each salt concentration and ionic lipid combination was incubated for 5 min at 37°C using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, the encapsulation rate of mRNA decreased with increasing salt concentration of the buffer (see Fig. 12 and Fig. 13).

### Example 6: Influence of mixing ratio and mixing mode

SS-OP was used as ionic lipid. The lipid composition was SS-OP:DOPC:Chol:DMG-PEG2000=52.5:7.5:40:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution or a solution obtained by diluting the particle solution with MES (pH 6) buffer (lipid concentration 300 nmol/15 µL, 300 nmol/75 µL, or 300 nmol/135 µL) and MES (pH 6) buffer containing luciferase-encoding mRNA (CleanCap (registered trademark) FLuc mRNA - (L-7602)) (nucleic acid concentration 3 ug/135 µL, 3 ug/75 µL, or 3 ug/15 µL) were mixed while diluting under stirring conditions using a vortex mixer or by tapping after pipetting, and incubated at 37°C for 5 min using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, a high encapsulation rate was obtained for all mixing ratios under stirring conditions using a vortex mixer; however, when mixing was performed by tapping after pipetting, the encapsulation rate decreased as the volume of the nucleic acid aqueous solution decreased (see Fig. 19, Fig. 20 and Fig. 21).

The nucleic acid-encapsulated nanoparticles obtained were applied to HeLa cells and the nucleic acid introduction efficiency was evaluated. HeLa cells were seeded in D-MEM medium (high glucose) (containing L-glutamine and phenol red) (containing 10% FBS and 1% penicillin-streptomycin solution) at 5×10³ cells/100 µL in a 96-well flat-bottom transparent plate. The nucleic acid-encapsulated particles obtained were added such that the nucleic acid concentration in the medium was 0.02 ug/4 µL. After 13 hr, the amount of luciferase protein introduced was evaluated using the amount of luminescence. 100 µL of Steady-Glo (registered trademark) was added, and the cells were lysed by shaking for 5 min, and the amount of luminescence was quantified using a GloMax 20/20 luminometer. As a result, no difference in in vitro activity was observed under stirring conditions using a vortex mixer, but when mixing was performed by tapping after pipetting and the volume of the aqueous nucleic acid solution was small, the in vitro activity decreased (see Fig. 21).

### Example 7: Influence of buffer

SS-OP and MC3 were used as ionic lipids. When SS-OP was used as the ionic lipid, the lipid composition was SS-OP:DOPC:Chol:DMG-PEG2000=52.5:7.5:40:1.5 in molar ratio, and when MC3 was used as the ionic lipid, the lipid composition was MC3:DSPC:Chol:DMG-PEG2000=50:10:38.5:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 5) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 5) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 5), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipids 20 mM, 15 µL) was mixed with a buffer solution (nucleic acid concentration 3 µg/135 µL, 135 µL) of MES (pH 5), phthalic acid (pH 5), maleic acid (pH 5), succinic acid (pH 5), DL-malic acid (pH 5), DL-tartaric acid (pH 5), or citric acid (pH 5) containing mRNA encoding luciferase (CleanCap (registered trademark) FLuc mRNA - (L-7602)) while diluting under stirring conditions using a vortex mixer, and incubated at 37°C for 5 min using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, mRNA was encapsulated in all buffer solutions of MES, phthalic acid, maleic acid, succinic acid, DL-malic acid, DL-tartaric acid, and citric acid, and no difference in the encapsulation rate was observed (see Fig. 22 and Fig. 23).

### Example 8: Influence of incubation temperature

SS-OP was used as ionic lipid. The lipid composition was SS-OP:DOPC:Chol:DMG-PEG2000=52.5:7.5:40:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution or DNase/RNase-Free Distilled Water was added to a sucrose final concentration of 0 mg/mL, 160 mg/mL or 320 mg/mL to prepare a particle solution.

The particle solution (total lipid 20 mM, 15 µL) was mixed with a solution containing luciferase-encoding mRNA (CleanCap (registered trademark) FLuc mRNA - (L-7602)) (nucleic acid concentration 3 µg/135 µL, buffer condition MES (pH 5), 135 µL) while diluting under stirring conditions using a vortex mixer, and the mixture was incubated for 5 min at 95°C, 75°C, 55°C or 37°C using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, mRNA was encapsulated at any incubation temperature, and no difference in the encapsulation rate was observed. The particle size of the obtained nucleic acid-encapsulated nanoparticles tended to increase with increasing incubation temperature (see Fig. 24 and Fig. 25).

The nucleic acid-encapsulated nanoparticles obtained were applied to HeLa cells to evaluate the nucleic acid introduction efficiency. HeLa cells were seeded in D-MEM medium (high glucose) (containing L-glutamine and phenol red) (containing 10% FBS and 1% penicillin-streptomycin solution added) at 5×10³ cells/100 µL in a 96-well flat-bottom transparent plate. The obtained nucleic acid-encapsulated particles were added such that the nucleic acid concentration in the medium was 0.02 µg/4 µL. After 13 hr, the amount of luciferase protein introduced was evaluated using the amount of luminescence. 100 µL of Steady-Glo (registered trademark) was added, and the cells were lysed by shaking for 5 min, and the amount of luminescence was quantified using a GloMax 20/20 luminometer. As a result, the introduction of luciferase protein was confirmed at any incubation temperatures, and no difference in in vitro activity was observed (see Fig. 26).

### Example 9: Influence of incubation time

SS-OP was used as ionic lipid. The lipid composition was SS-OP:DOPC:Chol:DMG-PEG2000=52.5:7.5:40:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipid 20 mM, 15 µL) was mixed with a solution containing luciferase-encoding mRNA (CleanCap (registered trademark) FLuc mRNA - (L-7602)) (nucleic acid concentration 3 µg/135 µL, buffer condition MES (pH 6), 135 µL) while diluting under stirring conditions using a vortex mixer, and the mixture was incubated for 60 min, 30 min, 15 min, 10 min, 5 min or 1 min at room temperature using an aluminum block incubator. After incubation, 150 µL of PBS was added to the solution to obtain nucleic acid-encapsulated nanoparticles. Alternatively, for comparison, 150 µL of PBS was first added to the particle solution (total lipids 20 mM, 15 pL), and then mRNA encoding luciferase (same as above) was added to a final concentration of 1.5 µg/300 µL.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, mRNA was encapsulated at any incubation time, and no difference in the encapsulation rate was observed. When PBS was added to the particle solution first (PBS→mRNA) , a low encapsulation rate was obtained (see Fig. 26 and Fig. 27).

The nucleic acid-encapsulated nanoparticles obtained were applied to HeLa cells to evaluate the nucleic acid introduction efficiency. HeLa cells were seeded in D-MEM medium (high glucose) (containing L-glutamine and phenol red) (containing 10% FBS and 1% penicillin-streptomycin solution added) at 5×10³ cells/100 µL in a 96-well flat-bottom transparent plate. The obtained nucleic acid-encapsulated particles were added such that the nucleic acid concentration in the medium was 0.02 µg/4 µL. After 13 hr, the amount of luciferase protein introduced was evaluated using the amount of luminescence. 100 µL of Steady-Glo (registered trademark) was added, and the cells were lysed by shaking for 5 min, and the amount of luminescence was quantified using a GloMax 20/20 luminometer. As a result, the introduction of luciferase protein was confirmed at any incubation time, and no difference in in vitro activity was observed. When PBS was added to the particle solution first (PBS→mRNA), a decrease in the in vitro activity was observed (see Fig. 29).

### Example 10: Influence of pH

SS-OP, SS-OC, MC3 or DODAP was used as ionic lipid. When SS-OP or SS-OC was used, DOPC, Chol and DMG-PEG2000 were used as other lipids, and when MC3 or DODAP was used, DSPC, Chol and DMG-PEG2000 were used as other lipids. The molar ratio of SS-OP or SS-OC:DOPC:Chol used was 52.5:7.5:40, and 1.5 mol% of DMG-PEG2000 was used relative to the total of SS-OP or SS-OC, DOPC and Chol. The molar ratio of MC3 or DODAP:DSPC:Chol:DMG-PEG2000 used was 50:10:38.5:1.5.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 5, pH 5.5, pH 6, pH 6.5 or pH 7) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 5, pH 5.5, pH 6, pH 6.5 or pH 7) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 5, pH 5.5, pH 6, pH 6.5 or pH 7), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipid 20 mM, 15 µL) was mixed with a solution containing luciferase-encoding mRNA (CleanCap (registered trademark) FLuc mRNA - (L-7602)) (nucleic acid concentration 3 µg/135 µL, buffer condition MES (pH 5, pH 5.5, pH 6, pH 6.5 or pH 7), 135 µL) while diluting under stirring conditions using a vortex mixer, and the mixture of each pH and ionic lipid combination was incubated for 5 min at 37°C using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, all of SS-OP, SS-OC, MC3 and DODAP were encapsulated up to pH 6, and the encapsulation rate decreased at pH 6.5 and pH 7 (see Fig. 30 and Fig. 31).

Among the obtained nucleic acid-encapsulated nanoparticles, the particles produced using SS-OP were applied to HeLa cells to evaluate the nucleic acid introduction efficiency. HeLa cells were seeded in D-MEM medium (high glucose) (containing L-glutamine and phenol red) (containing 10% FBS and 1% penicillin-streptomycin solution added) at 5×10³ cells/100 µL in a 96-well flat-bottom transparent plate. The obtained nucleic acid-encapsulated particles were added such that the nucleic acid concentration in the medium was 0.02 µg/4 µL. After 13 hr, the amount of luciferase protein introduced was evaluated using the amount of luminescence. 100 µL of Steady-Glo (registered trademark) was added, and the cells were lysed by shaking for 5 min, and the amount of luminescence was quantified using a GloMax 20/20 luminometer. As a result, the highest in vitro activity was observed at pH 6, the activity slightly decreasing at pH 6.5 and low activity was observed at pH 5, pH 5.5, and pH 7 (see Fig. 32).

Among the obtained nucleic acid-encapsulated nanoparticles, the particles produced using SS-OP were analyzed for Zeta potential. Zeta potential was measured by the electrophoretic light scattering method using Zetasizer (registered trademark) and using MES buffer (pH 5, pH 5.5, pH 6, pH 6.5 or pH 7). As a result, the particles produced at pH 5 and pH 5.5 showed higher values in a low pH environment compared to the particles produced at pH 6, pH 6.5 and pH 7 (see Fig. 33).

### Example 11: Influence of salt concentration

SS-OP and MC3 were used as ionic lipids. When SS-OP was used as the ionic lipid, the lipid composition was SS-OP:DOPC:Chol:DMG-PEG2000=52.5:7.5:40:1.5 in molar ratio, and when MC3 was used as the ionic lipid, the lipid composition was MC3:DSPC:Chol:DMG-PEG2000=50:10:38.5:1.5 in molar ratio.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipid 20 mM, 15 µL) was mixed with a solution containing luciferase-encoding mRNA (CleanCap (registered trademark) FLuc mRNA - (L-7602)) (nucleic acid concentration 3 µg/135 µL, buffer condition MES (pH 6) containing 0 mM, 1 mM, 10 mM, 30 mM, 50 mM, 100 mM, 150 mM or 300 mM NaCl, 135 µL) while diluting under stirring conditions using a vortex mixer, and the mixture of each salt concentration and ionic lipid combination was incubated for 5 min at 37°C using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, the encapsulation rate of mRNA tended to decrease with increasing salt concentration of the buffer (see Fig. 34 and Fig. 35).

### Example 12: Influence of ionic lipid species

LN-81 (compound 34 of Production Example described later) was used as ionic lipid. The lipid compositions were LN-81:DEPC:Chol:DMG-PEG2000=52.5:17.5:30:1 and LN-81:DEPC:Chol:DMG-PEG2000=60:5:35:1 in molar ratio.

2400 µL of acidic malic acid buffer (20 mM, pH 5.0) and 800 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 3:1, total flow rate of 4 mL/min, and syringe holder temperature: 25°C. After adding 3000 µL of MES buffer (pH 6.0) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6.0) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

The liquid or frozen-thawed particles were mixed with MES (pH 6) buffer (nucleic acid concentration 11.1 µg/180 µL, 180 µL) containing luciferase-encoding mRNA (CleanCap (registered trademark) FLuc mRNA - (L-7602)) while diluting under stirring conditions using a vortex mixer, and the mixture was incubated for 5 min at 37°C using an aluminum block incubator. After incubation, 100 µL of the solution was dispensed, 1010 µL of PBS was added thereto to obtain nucleic acid-encapsulated nanoparticles.

The particle size, polydispersity index (PdI), and mRNA encapsulation rate of the obtained nucleic acid-encapsulated nanoparticles were analyzed. The particle size and PdI were measured by the dynamic light scattering method using a Zetasizer (registered trademark). The mRNA encapsulation rate was measured by the Ribogreen (registered trademark) assay. As a result, mRNA was encapsulated (see Table 5).

**[Table 5]**

| Sample | | | production method | encapsulation rate (%) | Z-Ave (d.nm) | Number Mean (d.nm) | PdI |
|---|---|---|---|---|---|---|---|
| 1 | LN-81 | 52.5:17.5 :30:1 | liquid | 87 | 117 | 81 | 0.120 |
| 2 | | 60:5:35:1 | liquid | 94 | 92 | 58 | 0.115 |
| 3 | | | frozen-thawed particles | 93 | 111 | 71 | 0.116 |

### Comparative Example 1: Preparation method of freeze-dried product

SS-OP was used as the ionic lipid, and DOPC, Chol, and DMG-PEG2000 were used as other lipids. The molar ratio of SS-OP:DOPC:Chol used was 52.5:7.5:40, and 1.5 mol% of DMG-PEG2000 was used relative to the total of SS-OP, DOPC, and Chol.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). Sucrose solution was added to a final concentration of 160 mg/mL to prepare a particle solution. The obtained solution was placed in a mighty vial in an amount of 200 µL each, and freeze-dried to obtain a freeze-dried product.

The freeze-dried product was dissolved in a solution containing luciferase-encoding mRNA (CleanCap (registered trademark) FLuc mRNA - (L-7602)) (nucleic acid concentration 2 µg/200 µL, dissolved in DDW). The mixture was incubated for 5 min at 95°C using an aluminum block incubator. After incubation, 200 µL of PBS was added to the solution to obtain nucleic acid-encapsulated nanoparticles.

### Comparative Example 2: Preparation method of freeze-dried product

SS-OP was used as the ionic lipid, and DOPC, Chol, and DMG-PEG2000 were used as other lipids. The molar ratio of SS-OP:DOPC:Chol used was 52.5:7.5:40, and 1.5 mol% of DMG-PEG2000 was used relative to the total of SS-OP, DOPC, and Chol.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). Sucrose solution was added to a final concentration of 160 mg/mL to prepare a particle solution. The obtained solution was placed in a mighty vial in an amount of 200 µL each, and freeze-dried to obtain a freeze-dried product.

The freeze-dried product was dissolved in a solution containing luciferase-encoding mRNA (CleanCap (registered trademark) FLuc mRNA - (L-7602)) (nucleic acid concentration 4 µg/200 µL, dissolved in DDW). The mixture was incubated for 5 min at 95°C using an aluminum block incubator. After incubation, 200 µL of PBS was added to the solution to obtain nucleic acid-encapsulated nanoparticles.

### Comparative Example 3: Preparation method of freeze-dried product

SS-OP was used as the ionic lipid, and DOPC, Chol, and DMG-PEG2000 were used as other lipids. The molar ratio of SS-OP:DOPC:Chol used was 52.5:7.5:40, and 1.5 mol% of DMG-PEG2000 was used relative to the total of SS-OP, DOPC, and Chol.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 16 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). Sucrose solution was added to a final concentration of 160 mg/mL. The obtained solution was placed in a mighty vial in an amount of 200 µL each, and freeze-dried to obtain a freeze-dried product.

The freeze-dried product was dissolved in a solution containing luciferase-encoding mRNA (CleanCap (registered trademark) FLuc mRNA - (L-7602)) (nucleic acid concentration 4 µg/200 µL, dissolved in DDW). The mixture was incubated for 5 min at 75°C using an aluminum block incubator. After incubation, 200 µL of PBS was added to the solution to obtain nucleic acid-encapsulated nanoparticles.

### Experimental Example 1: In vitro activity

SS-OP was used as the ionic lipid, and DOPC, Chol, and DMG-PEG2000 were used as other lipids. The molar ratio of SS-OP:DOPC:Chol used was 52.5:7.5:40, and 1.5 mol% of DMG-PEG2000 was used relative to the total of SS-OP, DOPC, and Chol.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipid 20 mM, 15 µL) stored at 4°C (liquid) or the particle solution frozen at -80°C and then thawed at 4°C (frozen-thawed particles) was mixed with a solution containing luciferase-encoding mRNA (CleanCap (registered trademark) FLuc mRNA - (L-7602)) (nucleic acid concentration 1.5 µg/135 µL, buffer condition MES (pH 5), 135 µL) while diluting under stirring conditions using a vortex mixer, and the mixture was incubated for 5 min at 95°C using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles or nucleic acid-encapsulated nanoparticles prepared by freeze-dry technique in Comparative Example 1 were analyzed. The particle size and PdI were measured by the dynamic light scattering method using a Zetasizer (registered trademark). The mRNA encapsulation rate was measured by the Ribogreen (registered trademark) assay. As a result, the mRNA encapsulation rate in the liquid and in the frozen and thawed product was equivalent to that of nucleic acid-encapsulated nanoparticles produced by freeze-dry technique. In addition, the particle size of the nucleic acid-encapsulated nanoparticles was smaller than that of nucleic acid-encapsulated nanoparticles produced by the freeze-dry technique (see Fig. 14 and Fig. 15).

The obtained nucleic acid-encapsulated nanoparticles or the nucleic acid-encapsulated nanoparticles prepared by the freeze-dry technique in Comparative Example 1 were applied to HeLa cells and the nucleic acid transfer efficiency was evaluated. HeLa cells were seeded in D-MEM medium (high glucose) (containing L-glutamine and phenol red) (containing 10% FBS and 1% penicillin-streptomycin solution added) at 5×10³ cells/100 µL in a 96-well flat-bottom transparent plate. The obtained nucleic acid-encapsulated particles were added such that the nucleic acid concentration in the medium was 0.02 µg/4 µL. Luciferin was added to the medium at 0.1 mM, and the amount of luciferase protein introduced was evaluated over time using the amount of luminescence. HeLa cells cultured in a medium containing nucleic acid-encapsulated nanoparticles and luciferin were placed in an incubator-type luminometer (Kronos), and the cumulative amount of luminescence was measured for 2 min every hour. As a result, a higher nucleic acid introduction efficiency was obtained in the liquid and the frozen and thawed product, compared to the nucleic acid-encapsulated nanoparticles prepared by the freeze-dry technique (see Fig. 16 and Fig. 17).

### Experimental Example 2: In vivo activity

SS-OP was used as the ionic lipid, and DOPC, Chol, and DMG-PEG2000 were used as other lipids. The molar ratio of SS-OP:DOPC:Chol used was 52.5:7.5:40, and 1.5 mol% of DMG-PEG2000 was used relative to the total of SS-OP, DOPC, and Chol.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

The particle solution (total lipid 20 mM, 15 µL) stored at 4°C (liquid) or the particle solution frozen at -80°C and then thawed at 4°C (frozen-thawed particles) was mixed with a solution containing luciferase-encoding mRNA (CleanCap (registered trademark) FLuc mRNA - (L-7602)) (nucleic acid concentration 1.5 µg/135 µL, buffer condition MES (pH 5), 135 µL) while diluting under stirring conditions using a vortex mixer, and the mixture was incubated for 5 min at 95°C using an aluminum block incubator. After incubation, 150 µL of PBS was added to the solution to obtain nucleic acid-encapsulated nanoparticles.

The obtained nucleic acid-encapsulated nanoparticles or the nucleic acid-encapsulated nanoparticles prepared by the freeze-dry technique in Comparative Example 1 were applied to mouse (Balb/c, 6-week-old, female) and gene expression activity in the liver was evaluated using a GloMax 20/20 Luminometer. Each nucleic acid-encapsulated nanoparticle was administered at 0.05 mg/kg via the tail vein of the mice. Six hr after administration, the liver was removed and the expression level of luciferase protein was evaluated. As a result, higher gene expression activity in the liver was observed in the liquid and the frozen and thawed product compared to the nucleic acid-encapsulated nanoparticles prepared using the freeze-dry technique (see Fig. 18).

### Experimental Example 3: In vivo activity

SS-OP was used as the ionic lipid, and DOPC, Chol, and DMG-PEG2000 were used as other lipids. The molar ratio of SS-OP:DOPC:Chol used was 52.5:7.5:40, and 1.5 mol% of DMG-PEG2000 was used relative to the total of SS-OP, DOPC, and Chol.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

A particle solution (total lipid 20 mM, 15 µL) (liquid) was mixed with a solution containing luciferase-encoding mRNA (CleanCap (registered trademark) FLuc mRNA - (L-7602)) (nucleic acid concentration 3 µg/135 µL, buffer condition MES (pH 6), 135 µL) while diluting under stirring conditions using a vortex mixer, and the mixture was incubated for 5 min at 37°C using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles or nucleic acid-encapsulated nanoparticles prepared by freeze-dry technique in Comparative Example 2, Comparative Example 3 were analyzed. The particle size and PdI were measured by the dynamic light scattering method using a Zetasizer (registered trademark). The mRNA encapsulation rate was measured by the Ribogreen (registered trademark) assay. As a result, the mRNA encapsulation rate in the liquid was equivalent to that of nucleic acid-encapsulated nanoparticles produced by freeze-dry technique. In addition, the particle size of the nucleic acid-encapsulated nanoparticles was smaller than that of nucleic acid-encapsulated nanoparticles produced by the freeze-dry technique (see Fig. 36 and Fig. 37). In Fig. 36, Fig. 37 and Fig. 38, Comparative Example 2 in which the total flow rate during preparation of lipid nanoparticles was 1 mL/min, is shown as "freeze-dry (FR=1)", and Comparative Example 3, in which the total flow rate was 16 mL/min, is shown as "freeze-dry (FR=16)".

The obtained nucleic acid-encapsulated nanoparticles or the nucleic acid-encapsulated nanoparticles prepared by the freeze-dry technique in Comparative Example 2, Comparative Example 3 were applied to mouse (Balb/c, 6-week-old, female) and gene expression activity in the liver was evaluated using IVIS Imaging System. Each nucleic acid-encapsulated nanoparticle was administered at 0.1 mg/kg via the tail vein of the mice. Six hr after administration, images were taken and the expression level of luciferase protein was evaluated. As a result, higher gene expression activity in the liver was observed in the liquid compared to the nucleic acid-encapsulated nanoparticles prepared using the freeze-dry technique (see Fig. 38).

### Experimental Example 4: In vivo activity

SS-OP was used as the ionic lipid, and DOPC, Chol, and DMG-PEG2000 were used as other lipids. The molar ratio of SS-OP:DOPC:Chol used was 52.5:7.5:40, and 1.5 mol% of DMG-PEG2000 was used relative to the total of SS-OP, DOPC, and Chol.

2100 µL of acidic malic acid buffer (20 mM, pH 3.0) and 300 µL of lipid ethanol solution were weighed into a syringe. Using a NanoAssmblr (registered trademark) ultrafast nanomedicine production device (manufactured by Precision NanoSystems), LNP was prepared under the conditions of acidic buffer solution:lipid ethanol solution flow ratio of 7:1, total flow rate of 1 mL/min, and syringe holder temperature: 25°C. After adding 9000 µL of MES buffer (pH 6) to the recovered material, the obtained mixture was transferred to Amicon Ultra 15 and concentrated to about 1500 µL by ultrafiltration under centrifugal conditions (25°C, 1000 g, 5 min). The obtained concentrate was diluted to 15 mL with MES buffer (pH 6) and concentrated to about 500 µL by ultrafiltration again under centrifugal conditions (25°C, 1000 g, 5 min). The above operation was performed twice, and the entire mixture obtained by concentration was transferred to Amicon Ultra 4, and after further adding 3000 µL of MES buffer (pH 6), and the mixture was concentrated to about 100 µL by ultrafiltration under centrifugal conditions (25°C, 1000 g, 5 min). Sucrose solution was added to a final concentration of 160 mg/mL to prepare a particle solution.

A particle solution (total lipid 20 mM, 15 µL) (liquid) was mixed with a solution containing luciferase-encoding mRNA (CleanCap (registered trademark) FLuc mRNA - (L-7602)) (nucleic acid concentration 3 µg/135 µL, buffer condition MES (pH 6), 135 µL) while diluting under stirring conditions using a vortex mixer, and the mixture was incubated for 5 min at 37°C using an aluminum block incubator. After incubation, 150 µL of PBS was added to the solution to obtain nucleic acid-encapsulated nanoparticles.

The particle size, polydispersity index (PdI), and mRNA encapsulation rate of the obtained nucleic acid-encapsulated nanoparticles (liquid) or nucleic acid-encapsulated nanoparticles produced using a microfluidic channel (MF) (LNP prepared with the same lipid composition and using MES buffer (20 mM, pH 6.5) containing nucleic acid and an ethanol solution of lipid) were analyzed. The particle size and PdI were measured by the dynamic light scattering method using a Zetasizer (registered trademark). The mRNA encapsulation rate was measured by the Ribogreen (registered trademark) assay. As a result, the mRNA encapsulation rate of the liquid was equivalent to that of nucleic acid-encapsulated nanoparticles produced using a microfluidic channel (see Fig. 39).

The obtained nucleic acid-encapsulated nanoparticles (liquid) or the nucleic acid-encapsulated nanoparticles produced using a microfluidic channel (MF) were applied to mouse (Balb/c, 6-week-old, female) and gene expression activity in each organ (liver, heart, spleen, kidney and lung) was evaluated using IVIS Imaging System. Each nucleic acid-encapsulated nanoparticle was administered at 0.1 mg/kg via the tail vein of the mice. Six hr after administration and after anatomy, images were taken and the expression level of luciferase protein was evaluated. As a result, the liquid showed a similar expression distribution compared to the nucleic acid-encapsulated nanoparticles produced using a microfluidic channel (see Fig. 40 and Fig. 41).

### Experimental Example 5: In vitro activity of particles of Example 12

The nucleic acid-encapsulated nanoparticles obtained in Example 12 or the nucleic acid-encapsulated nanoparticles produced by the freeze-dry technique in Comparative Example 1 were applied to HeLa cells to evaluate the nucleic acid introduction efficiency. HeLa cells were seeded in D-MEM medium (high glucose) (containing L-glutamine and phenol red) (containing 10% FBS and 1% penicillin-streptomycin solution added) at 3×10⁴ cells/600 µL in a 48-well flat-bottom transparent plate. The next day, luciferin potassium was added to the medium, the obtained nucleic acid-encapsulated particles were added such that the nucleic acid concentration in the medium was 0.12 µg/60 µL, and the amount of luciferase protein introduced was evaluated for 24 hr using Kronos HT. As a result, compared to the nucleic acid-encapsulated nanoparticles produced by the freeze-dry technique, the liquid and frozen-thawed particles showed a higher nucleic acid introduction efficiency. Furthermore, when comparing the liquid with the frozen-thawed particles, the frozen-thawed particles had a higher nucleic acid introduction efficiency. In terms of composition, the liquid made with LN-81:DEPC:Chol:DMG-PEG2000=52.5:17.5:30:1 showed the highest nucleic acid introduction efficiency (see Fig. 42).

### Production of ionic lipid (2)

Table 6 shows the names and structures of the ionic lipids produced in the following Production Examples.

**[Table 6-1]**

| Production Example No. | name and structure (X, Y, Z correspond to the formula (2)) | correspondence 1 with parameters in formulas (2), (5) to (7) | correspondence 2 with parameters in formulas (2), (5) to (7) |
|---|---|---|---|
| 1 | 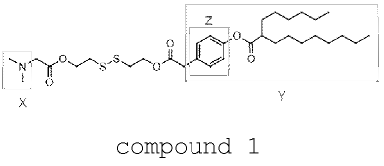 | R¹:methylene | |
| | | R²:methylene | k=1 l=0 |
| | | R^{x}:ethylene | m=0 |
| | | R^{y}:ethylene | n=0 |
| | | R⁷:1-hexylnonyl | t=0 |
| | | L¹:ester | u=0 |
| | | L²:ester | v=0 |
| | | L^{x}:ester | |
| 2 | 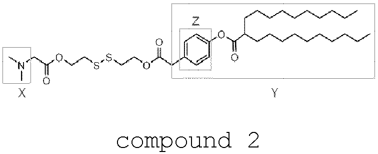 | R¹:methylene | |
| | | R²:methylene | k=1 |
| | | R^{x}:ethylene | l=0 |
| | | R^{y}:ethylene | m=0 |
| | | R⁷:1-decylundecyl | n=0 |
| | | | t=0 |
| | | L¹:ester | u=0 |
| | | L²:ester | v=0 |
| | | L^{x}:ester | |
| 3 | 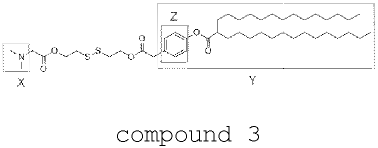 | R¹:methylene | |
| | | R²:methylene | k=1 |
| | | R^{x}:ethylene | l=0 |
| | | R^{y}:ethylene | m=0 |
| | | R⁷:1-tetradecyl-pentadecyl | n=0 |
| | | | t=0 |
| | | L¹:ester | u=0 |
| | | L²:ester | v=0 |
| | | L^{x}:ester | |
| 4 | 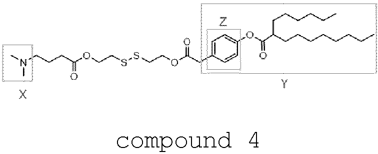 | R¹:trimethylene | |
| | | R²:methylene | k=1 |
| | | R^{x}:ethylene | l=0 |
| | | R^{y}:ethylene | m=0 |
| | | R⁷:1-hexylnonyl | n=0 |
| | | L¹:ester | t=0 |
| | | L²:ester | u=0 |
| | | L^{x}:ester | v=0 |

**[Table 6-2]**

| | | | |
|---|---|---|---|
| 5 | 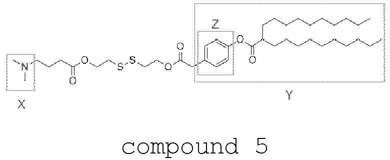 | R¹:trimethylene | k=1 |
| | | R²:methylene | l=0 |
| | | R^{x}: ethylene | m=0 |
| | | R^{y}:ethylene | n=0 |
| | | R⁷:1-decylundecyl | t=0 |
| | | | u=0 |
| | | L¹:ester | v=0 |
| | | L²:ester | |
| | | L^{x}:ester | |
| 6 | 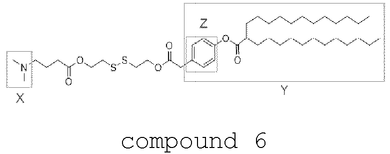 | R¹:trimethylene | k=1 |
| | | R²:methylene | l=0 |
| | | R^{x}:ethylene | m=0 |
| | | R^{y}:ethylene | n=0 |
| | | R⁷:1-dodecyltridecyl | t=0 |
| | | | u=0 |
| | | L¹:ester | v=0 |
| | | L²:ester | |
| | | L^{x}:ester | |
| 7 | 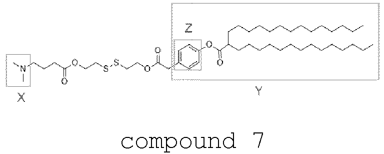 | R¹:trimethylene | k=1 |
| | | R²:methylene | l=0 |
| | | R^{x}: ethylene | m=0 |
| | | R^{y}:ethylene | n=0 |
| | | R⁷:1-tetradecyl- | t=0 |
| | | pentadecyl | u=0 |
| | | L¹:ester | v=0 |
| | | L²:ester | |
| | | L^{x}:ester | |
| 8 | 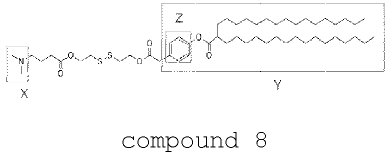 | R¹:trimethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:ester | v=0 |
| | | L²:ester | |
| | | L^{x}:ester | |
| 9 | 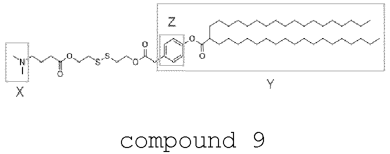 | R¹:trimethylene | k=1 |
| | | R²:methylene | l=0 |
| | | R^{x}:ethylene | m=0 |
| | | R^{y}:ethylene | n=0 |
| | | R⁷:1-octadecyl- | t=0 |
| | | nonadecyl | u=0 |
| | | L¹:ester | v=0 |
| | | L²:ester | |
| | | L^{x}:ester | |

**[Table 6-3]**

| | | | |
|---|---|---|---|
| 10 | 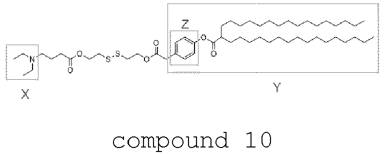 | R¹:trimethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:ester | v=0 |
| | | L²:ester | |
| | | L^{x}:ester | |
| 11 | 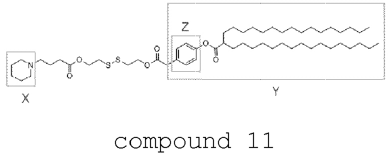 | R¹:trimethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:ester | v=0 |
| | | L²:ester | |
| | | L^{x}:ester | |
| 12 | 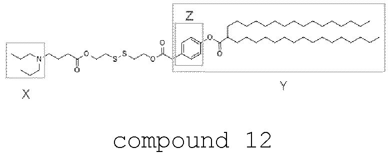 | R¹:trimethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:ester | v=0 |
| | | L²:ester | |
| | | L^{x}:ester | |
| 13 | 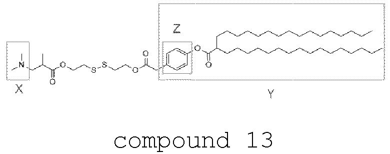 | R¹:isopropylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:ester | v=0 |
| | | L²:ester | |
| | | L^{x}:ester | |
| 14 | 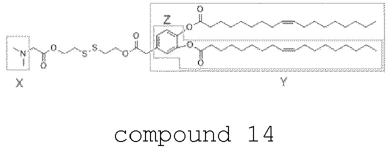 | R¹:methylene | k=1 |
| | | R²:methylene | l=0 |
| | | R^{x}:ethylene | m=0 |
| | | R^{y}:ethylene | n=0 |
| | | R⁷:heptadecenyl | t=0 |
| | | R⁸:octa-decenoyloxy | u=0 |
| | | | v=1 |
| | | L¹:ester | |
| | | L²:ester | |
| | | L^{x}:ester | |

**[Table 6-4]**

| | | | |
|---|---|---|---|
| 15 | 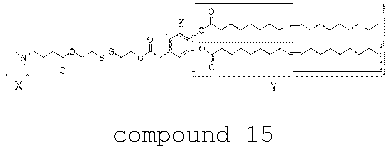 | R¹:trimethylene | k=1 |
| | | R²:methylene | l=0 |
| | | R^{x}: ethylene | m=0 |
| | | R^{y}:ethylene | n=0 |
| | | R⁷: heptadecenyl | t=0 |
| | | R⁸:octa-decenoyloxy | u=0 |
| | | | v=1 |
| | | L¹:ester | |
| | | L²:ester | |
| | | L^{x}:ester | |
| 16 | 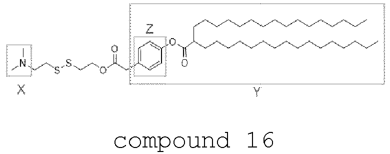 | R²:methylene | k=0 |
| | | R^{x}:ethylene | l=0 |
| | | R^{y}:ethylene | m=0 |
| | | R⁷:1-hexadecyl-heptadecyl | n=0 |
| | | | t=0 |
| | | L²:ester | u=0 |
| | | L^{x}:ester | v=0 |
| 17 | 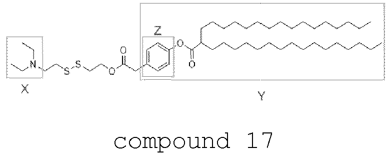 | R²:methylene | k=0 |
| | | R^{x}:ethylene | l=0 |
| | | R^{y}:ethylene | m=0 |
| | | R⁷:1-hexadecyl-heptadecyl | n=0 |
| | | | t=0 |
| | | L²:ester | u=0 |
| | | L^{x}:ester | v=0 |
| 18 | 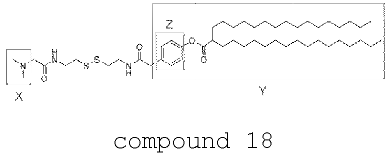 | R¹:methylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:amide | v=0 |
| | | L² :amide | |
| | | L^{x}:ester | |
| 19 | 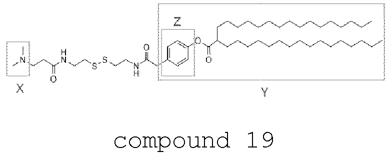 | R¹:ethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:amide | v=0 |
| | | L²:amide | |
| | | L^{x}:ester | |

**[Table 6-5]**

| | | | |
|---|---|---|---|
| 20 | 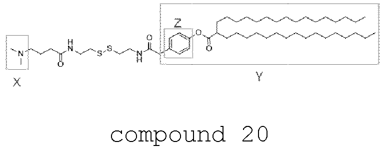 | R¹:trimethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:amide | v=0 |
| | | L²:amide | |
| | | L^{x}:ester | |
| 21 | 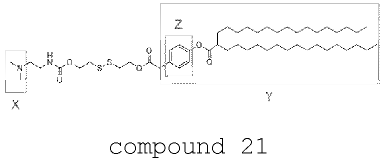 | R¹:ethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:carbamate | v=0 |
| | | L²:ester | |
| | | L^{x}:ester | |
| 22 | 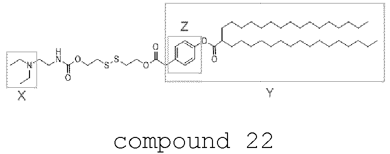 | R¹:ethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:carbamate | v=0 |
| | | L²:ester | |
| | | L^{x}:ester | |
| 23 | 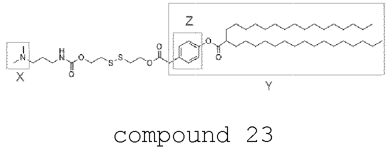 | R¹:trimethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:carbamate | v=0 |
| | | L²:ester | |
| | | L^{x}:ester | |
| 24 | 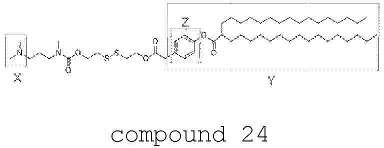 | R¹:trimethylene | k=1 |
| | | R²:methylene | l=0 |
| | | R^{x}:ethylene | m=0 |
| | | R^{y}:ethylene | n=0 |
| | | R⁷:1-hexadecyl- | t=0 |
| | | heptadecyl | u=0 |
| | | L¹:N-methylcarbamate | v=0 |
| | | | |
| | | L²:ester | |
| | | L^{x}:ester | |

**[Table 6-6]**

| | | | |
|---|---|---|---|
| 25 | 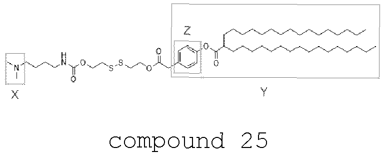 | R¹:tetra- | k=1 |
| | | methylene | l=0 |
| | | R²:methylene | m=0 |
| | | R^{x}:ethylene | n=0 |
| | | R^{y}:ethylene | t=0 |
| | | R⁷:1-hexadecyl-heptadecyl | u=0 |
| | | | v=0 |
| | | L¹:carbamate | |
| | | L²:ester | |
| | | L^{x}:ester | |
| 26 | 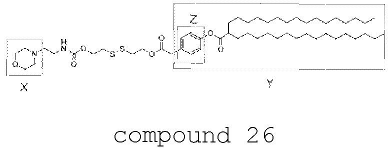 | R¹:ethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:carbamate | v=0 |
| | | L²:ester | |
| | | L^{x}:ester | |
| 27 | 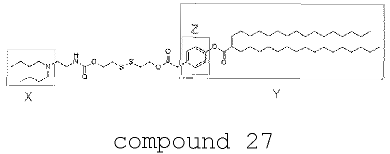 | R¹:ethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:carbamate | v=0 |
| | | L²:ester | |
| | | L^{x}:ester | |
| 28 | 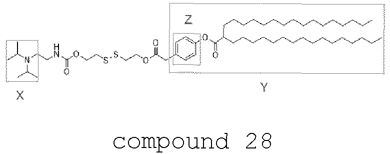 | R¹:ethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:carbamate | v=0 |
| | | L²:ester | |
| | | L^{x}:ester | |
| 29 | 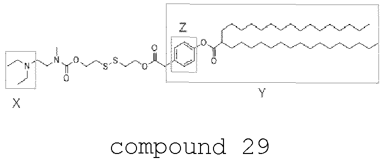 | R¹:ethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹ :N-methylcarbamate | v=0 |
| | | | |
| | | L²:ester | |
| | | L^{x}:ester | |

**[Table 6-7]**

| | | | |
|---|---|---|---|
| 30 | 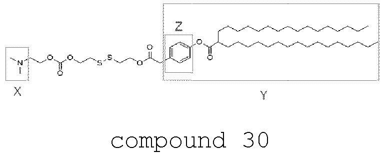 | R¹:ethylene | k=1 |
| | | R²:methylene | l=0 |
| | | R^{x}:ethylene | m=0 |
| | | R^{y}:ethylene | n=0 |
| | | R⁷:1-hexadecyl-heptadecyl | t=0 |
| | | | u=0 |
| | | L¹:carbonate | v=0 |
| | | L²:ester | |
| | | L^{x}:ester | |
| 31 | 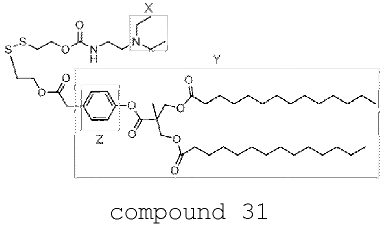 | R¹:ethylene | k=1 |
| | | R²:methylene | l=0 |
| | | R⁴:absent | m=1 |
| | | R⁵ :methyl | n=1 |
| | | R⁶:methylene | t=0 |
| | | R⁶':methylene | u=0 |
| | | R⁷:tridecyl | v=0 |
| | | R⁷''':tridecyl | |
| | | R^{x}:ethylene | |
| | | R^{y}:ethylene | |
| | | L¹:carbamate | |
| | | L²:ester | |
| | | L⁴:ester | |
| | | L⁴':ester | |
| | | L^{x}:ester | |
| 32 | 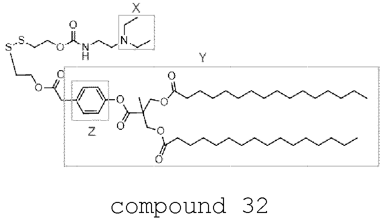 | R¹:ethylene | k=1 |
| | | R²:methylene | l=0 |
| | | R⁴:absent | m=1 |
| | | R⁵:methyl | n=1 |
| | | R⁶:methylene | t=0 |
| | | R⁶':methylene | u=0 |
| | | R⁷:pentadecyl | v=0 |
| | | R⁷''':pentadecyl | |
| | | R^{x}:ethylene | |
| | | R^{y}:ethylene | |
| | | L¹:carbamate | |
| | | L²:ester | |
| | | L⁴:ester | |
| | | L⁴':ester | |
| | | L^{x}:ester | |

**[Table 6-8]**

| | | | |
|---|---|---|---|
| 33 | 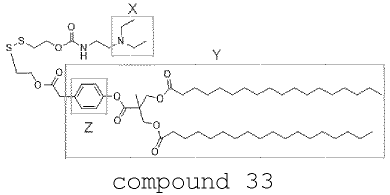 | R¹:ethylene | k=1 |
| | | R²:methylene | l=0 |
| | | R⁴:absent | m=1 |
| | | R⁵:methyl | n=1 |
| | | R⁶:methylene | t=0 |
| | | R⁶':methylene | u=0 |
| | | R⁷: heptadecyl | v=0 |
| | | R⁷''':heptadecyl | |
| | | R^{x}:ethylene | |
| | | R^{y}:ethylene | |
| | | L¹:carbamate | |
| | | L²:ester | |
| | | L⁴:ester | |
| | | L⁴':ester | |
| | | L^{x}:ester | |
| 34 | 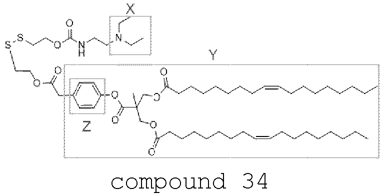 | R¹:ethylene | k=1 |
| | | R²:methylene | l=0 |
| | | R⁴:absent | m=1 |
| | | R⁵:methyl | n=1 |
| | | R⁶:methylene | t=0 |
| | | R⁶':methylene | u=0 |
| | | R⁷:heptadecenyl | v=0 |
| | | R⁷''':heptadecenyl | |
| | | R^{x}:ethylene | |
| | | R^{y}:ethylene | |
| | | L¹:carbamate | |
| | | L²:ester | |
| | | L⁴:ester | |
| | | L⁴':ester | |
| | | L^{x}:ester | |

**[Table 6-9]**

| | | | |
|---|---|---|---|
| 35 | 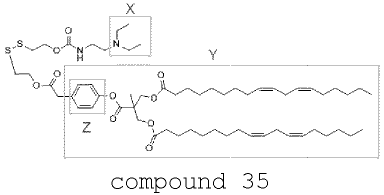 | R¹:ethylene | k=1 |
| | | R²:methylene | l=0 |
| | | R⁴:absent | m=1 |
| | | R⁵:methyl | n=1 |
| | | R⁶:methylene | t=0 |
| | | R⁶':methylene | u=0 |
| | | R⁷:hepta-decadienyl | v=0 |
| | | R⁷''':hepta-decadienyl | |
| | | R^{x}:ethylene | |
| | | R^{y}:ethylene | |
| | | L¹:carbamate | |
| | | L²:ester | |
| | | L⁴:ester | |
| | | L⁴':ester | |
| | | L^{x}:ester | |
| 36 | 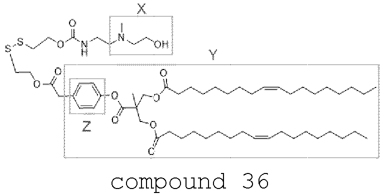 | R¹:ethylene | k=1 |
| | | R²:methylene | l=0 |
| | | R⁴:absent | m=1 |
| | | R⁵:methyl | n=1 |
| | | R⁶:methylene | t=0 |
| | | R⁶':methylene | u=0 |
| | | R⁷:heptadecenyl | v=0 |
| | | R⁷''':heptadecenyl | q=2 |
| | | R^{x}: ethylene | |
| | | R^{y}:ethylene | |
| | | R^{a}:methyl | |
| | | R^{b}:-(CH₂)₂-OH | |
| | | R^{c}:hydrogen | |
| | | L¹:carbamate | |
| | | L²:ester | |
| | | L⁴:ester | |
| | | L⁴':ester | |
| | | L^{x}:ester | |

**[Table 6-10]**

| | | | |
|---|---|---|---|
| 37 | 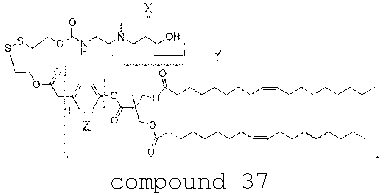 | R¹:ethylene | k=1 |
| | | R²:methylene | l=0 |
| | | R⁴:absent | m=1 |
| | | R⁵:methyl | n=1 |
| | | R⁶:methylene | t=0 |
| | | R⁶':methylene | u=0 |
| | | R⁷:heptadecenyl | v=0 |
| | | R⁷''':heptadecenyl | q=3 |
| | | R^{x}:ethylene | |
| | | R^{y}:ethylene | |
| | | R^{a}:methyl | |
| | | R^{b}:-(CH₂)₃-OH | |
| | | R^{c}:hydrogen | |
| | | L¹:carbamate | |
| | | L²:ester | |
| | | L⁴:ester | |
| | | L⁴':ester | |
| | | L^{x}:ester | |
| 38 | 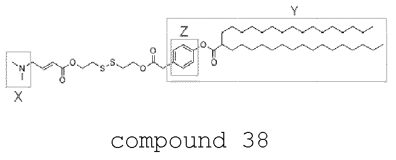 | R¹:propenylene | k=1 |
| | | R²:methylene | l=0 |
| | | R^{x}:ethylene | m=0 |
| | | R^{y}:ethylene | n=0 |
| | | L¹:ester | t=0 |
| | | L²:ester | u=0 |
| | | L^{x}:ester | v=0 |
| | | R⁷:1-hexadecyl-heptadecyl | |
| 39 | 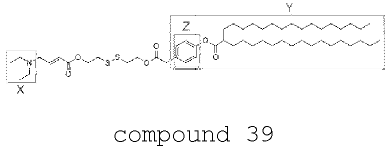 | R¹:propenylene | k=1 |
| | | R²:methylene | l=0 |
| | | R^{x}: ethylene | m=0 |
| | | R^{y}:ethylene | n=0 |
| | | L¹:ester | t=0 |
| | | L²:ester | u=0 |
| | | L^{x}:ester | v=0 |
| | | R⁷:1-hexadecyl-heptadecyl | |

**[Table 6-11]**

| | | | |
|---|---|---|---|
| 40 | 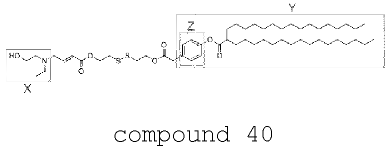 | R¹:propenylene | k=1 |
| | | R²:methylene | l=0 |
| | | R^{x}: ethylene | m=0 |
| | | R^{y}:ethylene | n=0 |
| | | R^{a}:ethyl | t=0 |
| | | R^{b}:-(CH₂)₂-OH | u=0 |
| | | R^{c}:hydrogen | v=0 |
| | | L¹:ester | q=2 |
| | | L²:ester | |
| | | L^{x}:ester | |
| | | R⁷:1-hexadecyl-heptadecyl | |
| | | | |
| 41 | 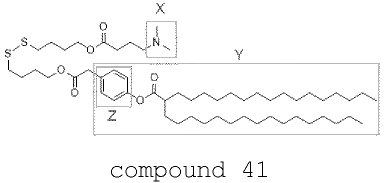 | R¹:trimethylene | k=1 |
| | | R²:methylene | l=0 |
| | | R^{x}:tetra- | m=0 |
| | | methylene | n=0 |
| | | R^{y}:tetra- | t=0 |
| | | methylene | u=0 |
| | | L¹:ester | v=0 |
| | | L²:ester | |
| | | L^{x}:ester | |
| | | R⁷:1-hexadecyl-heptadecyl | |
| | | | |
| 42 | 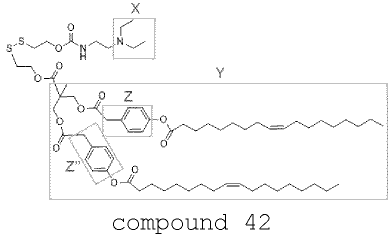 | R¹:ethylene | k=1 |
| | | R²:absent | l=1 |
| | | R³:methylene | m=0 |
| | | R⁷: heptadecenyl | n=0 |
| | | R⁷":heptadecenyl | t=1 |
| | | | u=0 |
| | | R^{x}:ethylene | v=0 |
| | | R^{y}:ethylene | |
| | | R^{e}':methylene | |
| | | S¹:methyl | |
| | | L¹:carbamate | |
| | | L²:ester | |
| | | L³:ester | |
| | | L^{a}:ester | |
| | | L^{b}:ester | |
| | | L^{x}:ester | |

**[Table 6-12]**

| | | | |
|---|---|---|---|
| 43 | 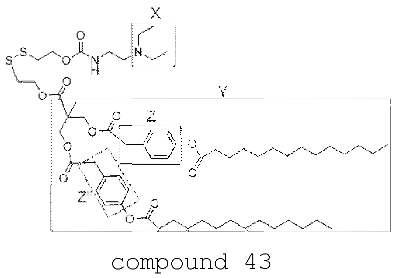 | R¹:ethylene | k=1 |
| | | R²:absent | l=1 |
| | | R³:methylene | m=0 |
| | | R⁷:tridecanyl | n=0 |
| | | R⁷''':tridecanyl | t=1 |
| | | R^{x}:ethylene | u=0 |
| | | R^{y}:ethylene | v=0 |
| | | R^{e}':methylene | |
| | | S¹:methyl | |
| | | L¹:carbamate | |
| | | L²:ester | |
| | | L³:ester | |
| | | L^{a}:ester | |
| | | L^{b}:ester | |
| | | L^{x}:ester | |
| 44 | 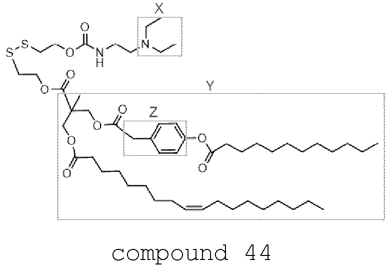 | R¹:ethylene | k=1 |
| | | R²: absent | l=1 |
| | | R³:methylene | m=0 |
| | | R⁷:undecyl | n=0 |
| | | R⁷":heptadecenyl | t=1 |
| | | | u=0 |
| | | R^{x}:ethylene | v=0 |
| | | R^{y}:ethylene | |
| | | R^{e}':methylene | |
| | | S¹:methyl | |
| | | L¹:carbamate | |
| | | L²:ester | |
| | | L³:ester | |
| | | L^{a}:ester | |
| | | L^{x}:ester | |

**[Table 6-13]**

| | | | |
|---|---|---|---|
| 45 | 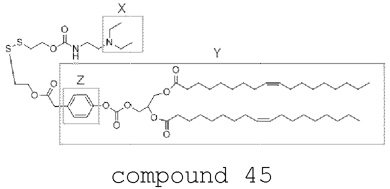 | R¹:ethylene | k=1 |
| | | R²:methylene | l=0 |
| | | R⁴:methylene | m=1 |
| | | R⁵:hydrogen | n=1 |
| | | R⁶:methylene | t=0 |
| | | R⁶':absent | u=0 |
| | | R⁷:heptadecenyl | v=0 |
| | | R⁷''':heptadecenyl | |
| | | R^{x}:ethylene | |
| | | R^{y}:ethylene | |
| | | L¹:carbamate | |
| | | L²:ester | |
| | | L⁴:ester | |
| | | L⁴':ester | |
| | | L^{x}:carbonate | |
| 46 | 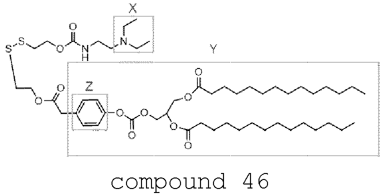 | R¹:ethylene | k=1 |
| | | R²:methylene | l=0 |
| | | R⁴:methylene | m=1 |
| | | R⁵:hydrogen | n=1 |
| | | R⁶:methylene | t=0 |
| | | R⁶':absent | u=0 |
| | | R⁷:tridecyl | v=0 |
| | | R⁷''':tridecyl | |
| | | R^{x}: ethylene | |
| | | R^{y}:ethylene | |
| | | L¹:carbamate | |
| | | L²:ester | |
| | | L⁴:ester | |
| | | L⁴':ester | |
| | | L^{x}:carbonate | |

### [Production Example 1] Synthesis of compound 1

Compound 1 was prepared by the following synthetic route, but the present invention is not limited to such synthetic route.

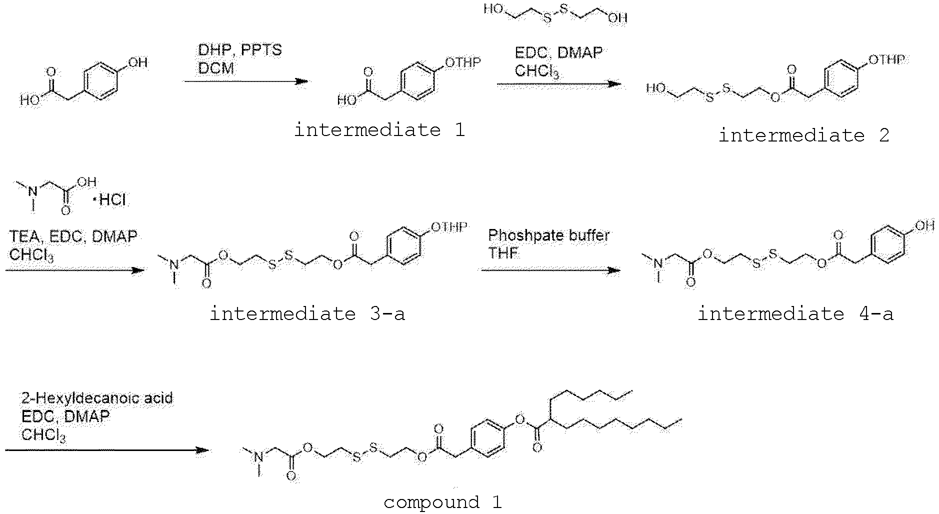

### <Synthesis of intermediate 1>

4-Hydroxyphenylacetic acid (75.0 g) and PPTS (12.4 g) were dissolved in 400 g of dichloromethane at room temperature, and the obtained solution was cooled to 10-20°C. A solution obtained by dissolving DHP (207 g) in 100 g of dichloromethane was added dropwise to the solution, and the reaction was performed at 25°C for 2 hr. The reaction solution was cooled to 10-20°C, and DMAP (30.1 g) was added to the solution to quench the reaction. 590 g of 2-propanol was added to the solution after the quenching, and the solution was cooled to 10-20°C. A solution of 98.8 g of 400 g/L aqueous sodium hydroxide solution and 307 g of ion-exchanged water were added dropwise to the solution after the cooling, and the reaction was performed at 25°C for 2 hr. The reaction solution was concentrated using an evaporator, and dichloromethane and 2-propanol were distilled off. The obtained concentrate was washed twice with 750 g of chloroform, and then 6N hydrochloric acid was added to obtain a solution with a pH of 5.0. The obtained solution was extracted twice with 750 g of chloroform, and the organic layer was dehydrated by adding 75.0 g of sodium sulfate. The sodium sulfate was removed by filtration, and the filtrate was concentrated in an evaporator to obtain 98.2 g of intermediate 1.

### <Synthesis of intermediate 2>

Intermediate 1 (10.2 g), bis(2-hydroxyethyl) disulfide (9.99 g), and DMAP (1.06 g) were dissolved in 153 g of chloroform at room temperature. EDC (12.4 g) was added to the obtained solution, and the mixture was allowed to react at 25°C for 2 hr. The reaction solution was washed with 102 g of 20 wt% brine, and then dehydrated by adding 5.1 g of sodium sulfate. After removing the sodium sulfate by filtration, the filtrate was concentrated in an evaporator, and the obtained crude product was purified with a column to obtain 9.01 g of intermediate 2.

### <Synthesis of intermediate 3-a>

Intermediate 2 (350 mg), dimethylglycine hydrochloride (144 mg), triethylamine (143 mg), and DMAP (23.0 mg) were dissolved in 5.25 mL of chloroform at room temperature. EDC (270 mg) was added to the obtained solution and the mixture was reacted at room temperature for 2 hr. The reaction solution was washed with 3.5 g of 0.5 M phosphate buffer (pH 4.0), 3.5 g of 7 wt% sodium bicarbonate water, and 3.5 g of 20 wt% brine in that order, and then 175 mg of sodium sulfate was added for dehydration. After removing the sodium sulfate by filtration, the filtrate was concentrated in an evaporator to obtain 378 mg of intermediate 3-a.

### <Synthesis of intermediate 4-a>

Intermediate 3-a (363 mg) was dissolved in 3.27 g of THF, and then 13.1 g of 0.5 M phosphate buffer (pH 2.0) was added and the reaction was performed at room temperature for 2 hr. 5.45 g of chloroform was added to the reaction solution for washing. 1N aqueous sodium hydroxide solution was added to the washed aqueous layer to adjust the pH to 6.0, and the aqueous layer after pH adjustment was extracted with 5.45 g of chloroform. The organic layer was dehydrated by adding 180 mg of sodium sulfate, and the sodium sulfate was removed by filtration. The filtrate was concentrated using an evaporator to obtain 187 mg of intermediate 4-a.

### <Synthesis of compound 1>

Intermediate 4-a (187 mg), 2-hexyldecanoic acid (128 mg), and DMAP (12.0 mg) were dissolved in 2.81 g of chloroform at room temperature. EDC (144 mg) was added to the obtained solution, and the reaction was performed at 25°C for 2 hr. The reaction solution was washed with 1.87 g of 20 wt% brine, and then 93.0 mg of sodium sulfate was added for dehydration. After removing the sodium sulfate by filtration, the filtrate was concentrated using an evaporator, and the obtained crude product was purified with a column to obtain 159 mg of compound 1.

### <¹H-NMR(600MHz, CDCl₃) of compound 1>

δ:0.86-0.90(m, 6H), 1.20-1.45(m, 22H), 1.69-1.75(m, 2H), 2.36(s, 6H), 2.52-2.59(m, 1H), 2.88-2.97(m, 4H), 3.19(s, 2H), 3.63(s, 2H), 4.34-4.42(m, 4H), 7.02-7.05(m, 2H), 7.27-7.30(m, 2H)

### [Production Example 2] Synthesis of compound 2

### <Synthesis of compound 2>

Using intermediate 4-a and 2-decyldodecanoic acid, compound 2 was synthesized by the same synthetic route as in Production Example 1.

### <¹H-NMR(600MHz, CDCl₃) of compound 2>

δ:0.88(t, 6H), 1.20-1.45(m, 34H), 1.69-1.75(m, 2H), 2.36(s, 6H), 2.52-2.59(m, 1H), 2.88-2.97(m, 4H), 3.19(s, 2H), 3.63(s, 2H), 4.34-4.42(m, 4H), 7.02-7.05(m, 2H), 7.27-7.30(m, 2H)

### [Production Example 3] Synthesis of compound 3

### <Synthesis of compound 3>

Using intermediate 4-a and 2-tetradecylhexadecanoic acid, compound 3 was synthesized by the same synthetic route as in Production Example 1.

### <¹H-NMR(600MHz, CDCl₃) of compound 3>

δ:0.88(t, 6H), 1.20-1.45(m, 50H), 1.69-1.75(m, 2H), 2.36(s, 6H), 2.52-2.59(m, 1H), 2.88-2.97(m, 4H), 3.19(s, 2H), 3.63(s, 2H), 4.34-4.42(m, 4H), 7.02-7.05(m, 2H), 7.27-7.30(m, 2H)

### [Production Example 4] Synthesis of compound 4

### <Synthesis of intermediate 3-b>

In the same manner as in Production Example 1 except that 4-(dimethylamino)butanoic acid hydrochloride was used, intermediate 3-b represented by the following formula was synthesized.

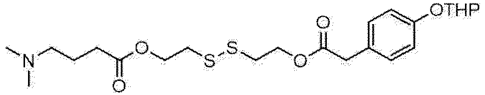

### <Synthesis of intermediate 4-b>

In the same manner as in Production Example 1 except that intermediate 3-b was used, intermediate 4-b represented by the following formula was synthesized.

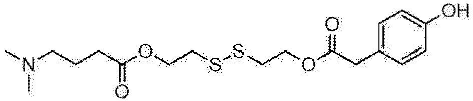

### <Synthesis of compound 4>

In the same manner as in Production Example 1 except that intermediate 4-b was used, compound 4 was synthesized.

### <¹H-NMR(600MHz, CDCl₃) of compound 4>

δ:0.86-0.90(m, 6H), 1.20-1.45(m, 22H), 1.69-1.82(m, 4H), 2.21(s, 6H), 2.28(t, 2H), 2.35(t, 2H), 2.52-2.59(m, 1H), 2.88-2.97(m, 4H), 3.63(s, 2H), 4.28-4.38(m, 4H), 7.02-7.05(m, 2H), 7.27-7.30(m, 2H)

### [Production Example 5] Synthesis of compound 5

### <Synthesis of compound 5>

Using intermediate 4-b and 2-decyldodecanoic acid, compound 5 was synthesized by the same synthetic route as in Production Example 1.

### <¹H-NMR(600MHz, CDCl₃) of compound 5>

δ:0.88(t, 6H), 1.20-1.45(m, 34H), 1.69-1.84(m, 4H), 2.21(s, 6H), 2.28(t, 2H), 2.35(t, 2H), 2.52-2.59(m, 1H), 2.88-2.97(m, 4H), 3.63(s, 2H), 4.28-4.38(m, 4H), 7.02-7.05(m, 2H), 7.27-7.30(m, 2H)

### [Production Example 6] Synthesis of compound 6

### <Synthesis of compound 6>

Using intermediate 4-b and 2-dodecyltetradecanoic acid, compound 6 was synthesized by the same synthetic route as in Production Example 1.

### <¹H-NMR(600MHz, CDCl₃) of compound 6>

δ:0.88(t, 6H), 1.20-1.45(m, 42H), 1.69-1.84(m, 4H), 2.21(s, 6H), 2.28(t, 2H), 2.35(t, 2H), 2.52-2.59(m, 1H), 2.88-2.97(m, 4H), 3.63(s, 2H), 4.28-4.38(m, 4H), 7.02-7.05(m, 2H), 7.27-7.30(m, 2H)

### [Production Example 7] Synthesis of compound 7

### <Synthesis of compound 7>

Using intermediate 4-b and 2-tetradecylhexadecanoic acid, compound 7 was synthesized by the same synthetic route as in Production Example 1.

### <¹H-NMR(600MHz, CDCl₃) of compound 7>

δ:0.88(t, 6H), 1.20-1.45(m, 50H), 1.69-1.84(m, 4H), 2.21(s, 6H), 2.28(t, 2H), 2.35(t, 2H), 2.52-2.59(m, 1H), 2.88-2.97(m, 4H), 3.63(s, 2H), 4.28-4.38(m, 4H), 7.02-7.05(m, 2H), 7.27-7.30(m, 2H)

### [Production Example 8] Synthesis of compound 8

### <Synthesis of compound 8>

Using intermediate 4-b and 2-hexadecyloctadecanoic acid, compound 8 was synthesized by the same synthetic route as in Production Example 1.

### <¹H-NMR(600MHz, CDCl₃) of compound 8>

δ:0.88(t, 6H), 1.20-1.45(m, 58H), 1.69-1.84(m, 4H), 2.21(s, 6H), 2.28(t, 2H), 2.35(t, 2H), 2.52-2.59(m, 1H), 2.88-2.97(m, 4H), 3.63(s, 2H), 4.28-4.38(m, 4H), 7.02-7.05(m, 2H), 7.27-7.30(m, 2H)

### [Production Example 9] Synthesis of compound 9

### <Synthesis of compound 9>

Using intermediate 4-b and 2-octadecyleicosanoic acid, compound 9 was synthesized by the same synthetic route as in Production Example 1.

### <¹H-NMR(600MHz, CDCl₃) of compound 9>

δ:0.88(t, 6H), 1.20-1.45(m, 66H), 1.69-1.84(m, 4H), 2.21(s, 6H), 2.28(t, 2H), 2.35(t, 2H), 2.52-2.59(m, 1H), 2.88-2.97(m, 4H), 3.63(s, 2H), 4.28-4.38(m, 4H), 7.02-7.05(m, 2H), 7.27-7.30(m, 2H)

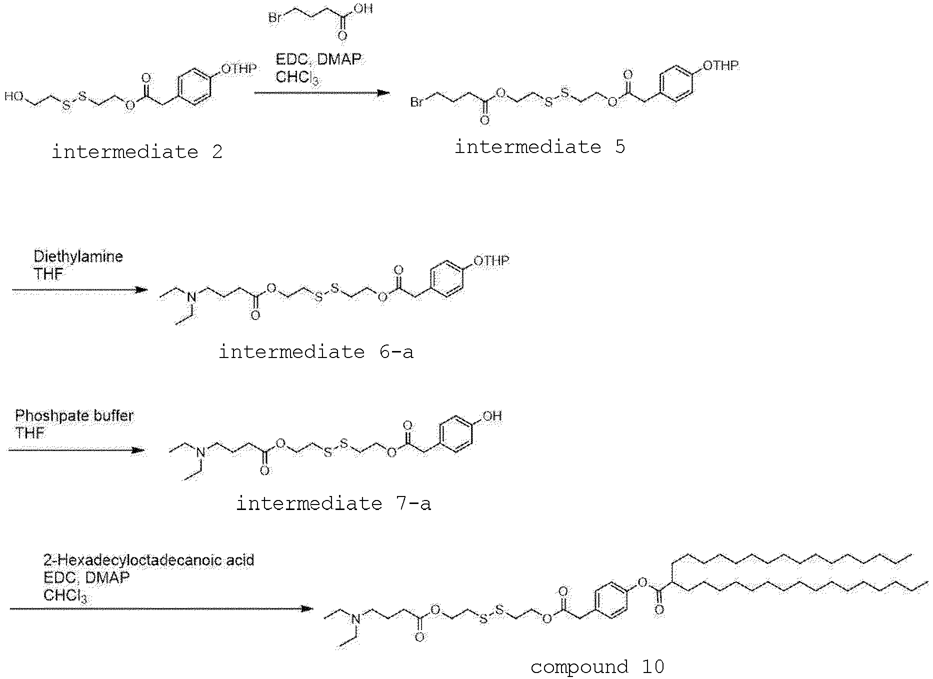

### [Production Example 10] Synthesis of compound 10

### <Synthesis of intermediate 5>

Intermediate 2 (5.10 g), 4-bromobutyric acid (2.52 g), and DMAP (335 mg) were dissolved in 76.5 g of chloroform at room temperature. EDC (3.94 g) was added to the obtained solution and the mixture was reacted at 25°C for 2 hr. The reaction solution was washed with 51.0 g of 20 wt% brine, and then 2.55 g of sodium sulfate was added for dehydration. After removing the sodium sulfate by filtration, the filtrate was concentrated in an evaporator to obtain 6.07 g of Intermediate 5.

### <Synthesis of intermediate 6-a>

Diethylamine (258 mg) was dissolved in 1.04 g of THF at room temperature. A solution obtained by dissolving intermediate 5 (230 mg) in 780 mg of THF was added dropwise to the solution, and the mixture was reacted at 50°C for 15 hr. After adding 3.45 g of chloroform to the reaction solution, the mixture was washed with 2.30 g of 0.5 M acetate buffer (pH 4.0), 2.30 g of 7 wt% sodium bicarbonate water, and 2.30 g of 20 wt% brine in that order. After adding 130 mg of sodium sulfate to dehydrate, the sodium sulfate was removed by filtration, and the filtrate was concentrated in an evaporator to obtain 130 mg of intermediate 6-a.

### <Synthesis of intermediate 7-a>

Using intermediate 6-a, intermediate 7-a was synthesized by a method similar to that for intermediate 4-a in Production Example 1.

### <Synthesis of compound 10>

Using intermediate 7-a and 2-hexadecyloctadecanoic acid, compound 10 was synthesized by a method similar to that for compound 1 in Production Example 1.

### <¹H-NMR(600MHz, CDCl₃) of compound 10>

δ:0.88(t, 6H), 1.00(t, 6H), 1.20-1.45(m, 58H), 1.69-1.84(m, 4H), 2.28-2.60(m, 9H), 2.88-2.97(m, 4H), 3.63(s, 2H), 4.28-4.38(m, 4H), 7.02-7.05(m, 2H), 7.27-7.30(m, 2H)

### [Production Example 11] Synthesis of compound 11

### <Synthesis of intermediate 6-b>

In the same manner as in Production Example 10 except that piperidine was used, intermediate 6-b represented by the following formula was synthesized.

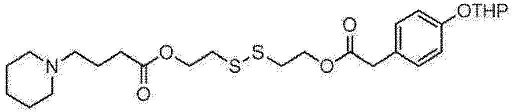

### <Synthesis of intermediate 7-b>

In the same manner as in Production Example 10 except that intermediate 6-b was used, intermediate 7-b represented by the following formula was synthesized.

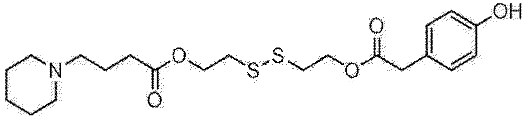

### <Synthesis of compound 11>

Using intermediate 7-b and 2-hexadecyloctadecanoic acid, compound 11 was synthesized by the same synthetic route as in Production Example 10.

### <¹H-NMR(600MHz, CDCl₃) of compound 11>

δ:0.88(t, 6H), 1.20-1.45(m, 60H), 1.45-1.60(m, 4H), 1.69-1.84(m, 4H), 2.20-2.50(m, 8H), 2.52-2.59(m, 1H), 2.88-2.97(m, 4H), 3.63(s, 2H), 4.28-4.38(m, 4H), 7.02-7.05(m, 2H), 7.27-7.30(m, 2H)

### [Production Example 12] Synthesis of compound 12

### <Synthesis of intermediate 6-c>

In the same manner as in Production Example 10 except that dipropylamine was used, intermediate 6-c represented by the following formula was synthesized.

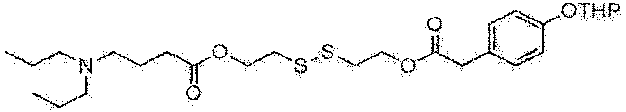

### <Synthesis of intermediate 7-c>

In the same manner as in Production Example 10 except that intermediate 6-c was used, intermediate 7-c represented by the following formula was synthesized.

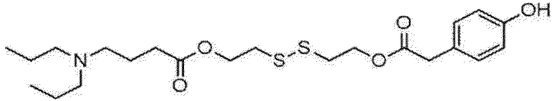

### <Synthesis of compound 12>

Using intermediate 7-c and 2-hexadecyloctadecanoic acid, compound 12 was synthesized by the same synthetic route as in Production Example 10.

### <¹H-NMR(600MHz, CDCl₃) of compound 12>

δ:0.86-0.92(m, 12H), 1.20-1.45(m, 62H), 1.69-1.84(m, 4H), 2.28-2.60(m, 9H), 2.88-2.97(m, 4H), 3.63(s, 2H), 4.28-4.38(m, 4H), 7.02-7.05(m, 2H), 7.27-7.30(m, 2H)

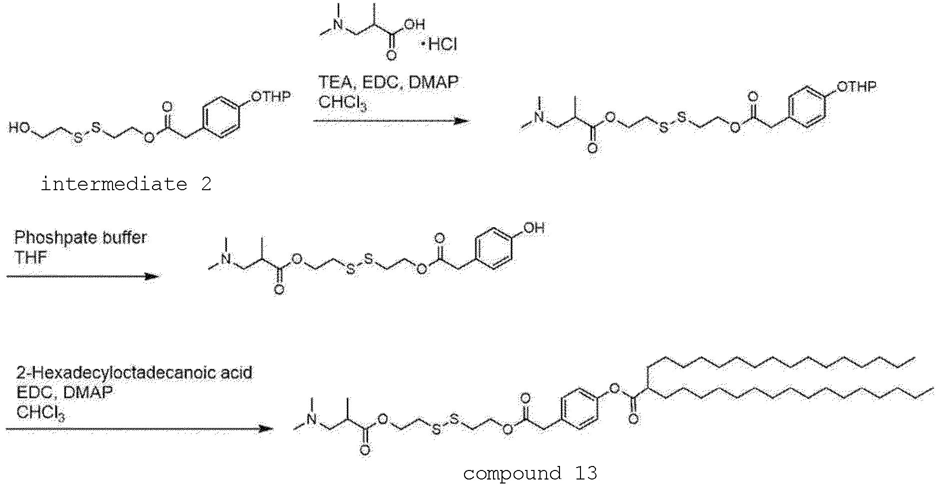

### [Production Example 13] Synthesis of compound 13

### <Synthesis of compound 13>

By a synthesis route similar to that in Production Example 8 except that intermediate 2 and 3-(dimethylamino)-2-methylpropionic acid hydrochloride were used as starting materials, compound 13 was synthesized.

### <¹H-NMR(600MHz, CDCl₃) of compound 13>

δ:0.88(t, 6H), 1.13(d, 3H), 1.20-1.45(m, 58H), 1.68-1.78(m, 2H), 2.18-2.22(m, 7H), 2.52-2.70(m, 3H), 2.88-2.97(m, 4H), 3.63(s, 2H), 4.28-4.38(m, 4H), 7.02-7.05(m, 2H), 7.27-7.30(m, 2H)

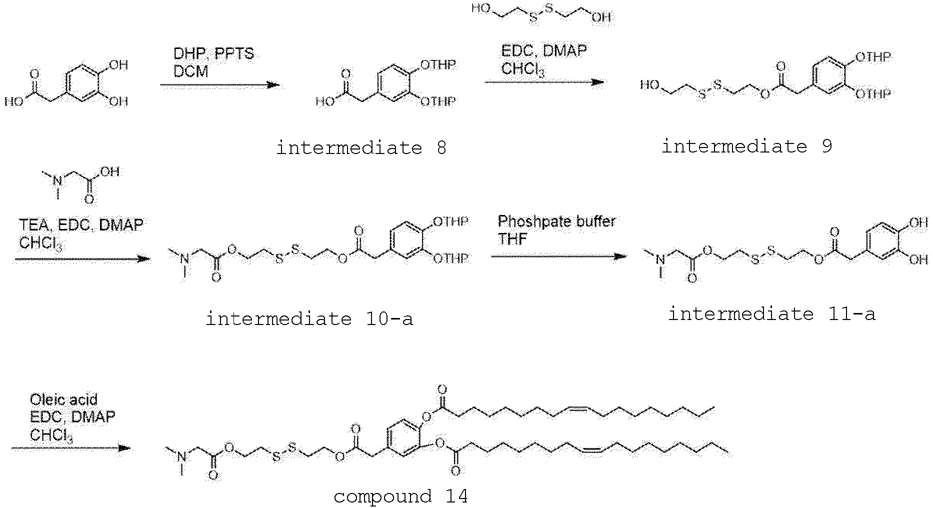

### [Production Example 14] Synthesis of compound 14

### <Synthesis of intermediate 8>

3,4-Dihydroxyphenylacetic acid (3.00 g) and PPTS (448 mg) were dissolved in 40.0 g of dichloromethane at room temperature, and the obtained solution was cooled to 20°C. DHP (7.50 g) was added dropwise to the solution, and the reaction was performed at room temperature for 2 hr. The reaction solution was cooled to 20°C, and DMAP (1.09 g) was added to the solution to quench the reaction. 47.0 g of 2-propanol was added to the solution after the quenching, and the solution was cooled to 10-20°C. 30.0 g of 1N aqueous sodium hydroxide solution was added dropwise to the solution after the cooling, and the reaction was performed at 25°C for 2 hr. The reaction solution was concentrated using an evaporator, and dichloromethane and 2-propanol were distilled off. The concentrate was washed with 30.0 g of chloroform, and then 6N hydrochloric acid was added to obtain a solution with a pH of 5.0. The solution was extracted with 30.0 g of chloroform, and the organic layer was dehydrated by adding 1.50 g of sodium sulfate. After removing the sodium sulfate by filtration, the filtrate was concentrated in an evaporator to obtain 4.98 g of intermediate 8.

### <Synthesis of intermediate 9>

Intermediate 8 (800 mg), bis(2-hydroxyethyl) disulfide (550 mg), and DMAP (58.0 mg) were dissolved in 12.0 g of chloroform at room temperature. EDC (684 mg) was added to the obtained solution, and the reaction was performed at 25°C for 2 hr. The reaction solution was washed with 8.00 g of 20 wt% brine, and then dehydrated by adding 400 mg of sodium sulfate. After removing sodium sulfate by filtration, the filtrate was concentrated using an evaporator, and the obtained crude product was purified with a column to obtain 530 mg of intermediate 9.

### <Synthesis of intermediate 10-a>

Intermediate 9 (525 mg), dimethylglycine hydrochloride (171 mg), triethylamine (169 mg), and DMAP (27.0 mg) were dissolved in 7.90 g of chloroform at room temperature. EDC (319 mg) was added to the obtained solution and the mixture was reacted at room temperature for 2 hr. The reaction solution was washed with 5.25 g of 0.5 M phosphate buffer (pH 4.0), 5.25 g of 7 wt% sodium bicarbonate water, and 5.25 g of 20 wt% brine, in that order, and then 250 mg of sodium sulfate was added for dehydration. After removing sodium sulfate by filtration, the filtrate was concentrated using an evaporator to obtain 558 mg of intermediate 10-a.

### <Synthesis of intermediate 11-a>

Intermediate 10-a (550 mg) was dissolved in 4.95 g of THF, and then 19.8 g of 0.5 M phosphate buffer (pH 2.0) was added and the mixture was reacted at room temperature for 2 hr. 8.25 g of chloroform was added to the reaction solution for washing. 1N aqueous sodium hydroxide solution was added to the washed aqueous layer to adjust the pH to 6.5, and the aqueous layer after pH adjustment was extracted with 8.25 g of chloroform. 300 mg of sodium sulfate was added to the organic layer for dehydration, and sodium sulfate was removed by filtration. The filtrate was concentrated in an evaporator to obtain 503 mg of intermediate 11-a.

### <Synthesis of compound 14>

Intermediate 11-a (190 mg), oleic acid (289 mg), and DMAP (24.0 mg) were dissolved in 2.85 g of chloroform at room temperature. EDC (234 mg) was added to the obtained solution and the mixture was reacted at 25°C for 2 hr. The reaction solution was washed with 1.90 g of 20 wt% brine, and then dehydrated by adding 100 mg of sodium sulfate. After removing sodium sulfate by filtration, the filtrate was concentrated in an evaporator and purified with a column to obtain 291 mg of compound 14.

### <¹H-NMR(600MHz, CDCl₃) of compound 14>

δ:0.88(t, 6H), 1.22-1.42(m, 40H), 1.69-1.83(m, 6H), 1.99-2.05(m, 8H), 2.36(s, 6H), 2.50-2.53(m, 4H), 2.88-2.97(m, 4H), 3.63(s, 2H), 4.34-4.42(m, 4H), 5.32-5.38(m, 4H), 7.10-7.17(m, 3H)

### [Production Example 15] Synthesis of compound 15

### <Synthesis of intermediate 10-b>

In the same manner as in Production Example 14 except that 4-(dimethylamino)butanoic acid hydrochloride was used, intermediate 10-b represented by the following formula was synthesized.

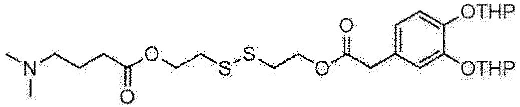

### <Synthesis of intermediate 11-b>

In the same manner as in Production Example 14 except that intermediate 10-b was used, intermediate 11-b represented by the following formula was synthesized.

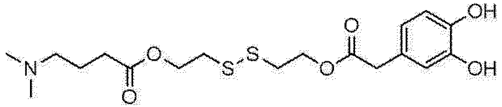

### <Synthesis of compound 15>

Using intermediate 11-b and oleic acid, compound 15 was synthesized by the same synthetic route as in Production Example 14.

### <¹H-NMR(600MHz, CDCl₃) of compound 15>

δ:0.88(t, 6H), 1.22-1.42(m, 40H), 1.69-1.83(m, 6H), 1.99-2.05(m, 8H), 2.26(s, 6H), 2.33-2.54(m, 4H), 2.50-2.53(m, 4H), 2.88-2.97(m, 4H), 3.63(s, 2H), 4.34-4.42(m, 4H), 5.32-5.38(m, 4H), 7.10-7.17(m, 3H)

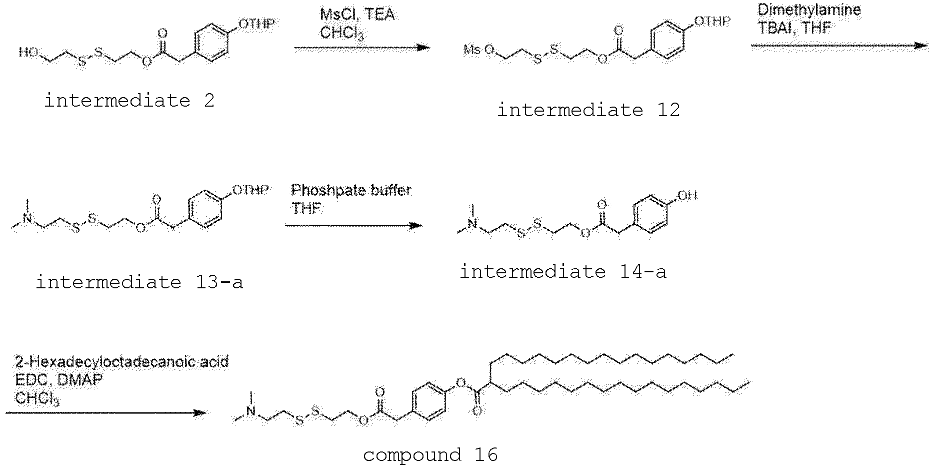

### [Production Example 16] Synthesis of compound 16

### <Synthesis of intermediate 12>

Intermediate 2 (5.00 g) and triethylamine (2.72 g) were dissolved in 125 g of chloroform at room temperature. Methanesulfonyl chloride (2.31 g) was added to the obtained solution and the mixture was reacted at 25°C for 3 hr. The reaction solution was washed with 75.0 g of 7 wt% sodium bicarbonate water, and 5.00 g of sodium sulfate was added for dehydration. After removing the sodium sulfate by filtration, the filtrate was concentrated in an evaporator and purified with a column to obtain 4.42 g of intermediate 12.

### <Synthesis of intermediate 13-a>

Intermediate 12 (600 mg) was dissolved in 5.40 g of dimethylamine (2.0 mol/L in THF), and TBAI (49.0 mg) was added and the mixture was reacted at 25°C for 20 hr. The reaction solution was added with 9.00 g of chloroform, washed with 6.00 g of 0.5 M acetate buffer (pH 4.0), and dehydrated by adding 300 mg of sodium sulfate. After removing the sodium sulfate by filtration, the filtrate was concentrated using an evaporator to obtain 470 mg of intermediate 13-a.

<Synthesis of intermediate 14-a>

In the same manner as in intermediate 4-a of Production Example 1 except that intermediate 13-a was used, intermediate 14-a was synthesized.

### <Synthesis of compound 16>

Using intermediate 14-a and 2-hexadecyloctadecanoic acid, compound 16 was synthesized by a method similar to that for compound 1 in Production Example 1.

### <¹H-NMR(600MHz, CDCl₃) of compound 16>

δ:0.88(t, 6H), 1.20-1.45(m, 58H), 1.69-1.84(m, 2H), 2.25(s, 6H), 2.52-2.60(m, 3H), 2.80(t, 2H), 2.90(t, 2H), 3.63(s, 2H), 4.35(t, 2H), 7.02-7.05(m, 2H), 7.27-7.30(m, 2H)

### [Production Example 17] Synthesis of compound 17

### <Synthesis of intermediate 13-b>

4.20 g of THF was added to intermediate 12 (600 mg) and diethylamine (779 mg) and dissolved at room temperature. TBAI (49.0 mg) was added to the obtained solution and the mixture was reacted at 25°C for 3 hr. 9.00 g of chloroform was added to the reaction solution, which was washed with 6.00 g of 0.5 M acetate buffer (pH 4.0), and 300 mg of sodium sulfate was added for dehydration. After removing sodium sulfate by filtration, the filtrate was concentrated using an evaporator to obtain 473 mg of intermediate 13-b represented by the following formula.

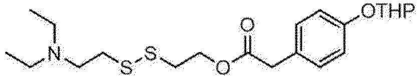

### <Synthesis of intermediate 14-b>

In the same manner as in Production Example 16 except that intermediate 13-b was used, intermediate 14-b represented by the following formula was synthesized.

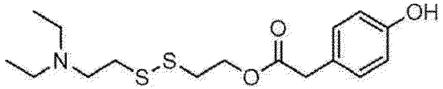

### <Synthesis of compound 17>

Using intermediate 14-b and 2-hexadecyloctadecanoic acid, compound 17 was synthesized by the same synthetic route as in Production Example 16.

### <¹H-NMR(600MHz, CDCl₃) of compound 17>

δ:0.88(t, 6H), 1.03(t, 6H), 1.20-1.45(m, 58H), 1.69-1.84(m, 2H), 2.53-2.57(m, 5H), 2.75-2.80(m, 4H), 2.91(t, 2H), 3.63(s, 2H), 4.35(t, 2H), 7.02-7.05(m, 2H), 7.27-7.30(m, 2H)

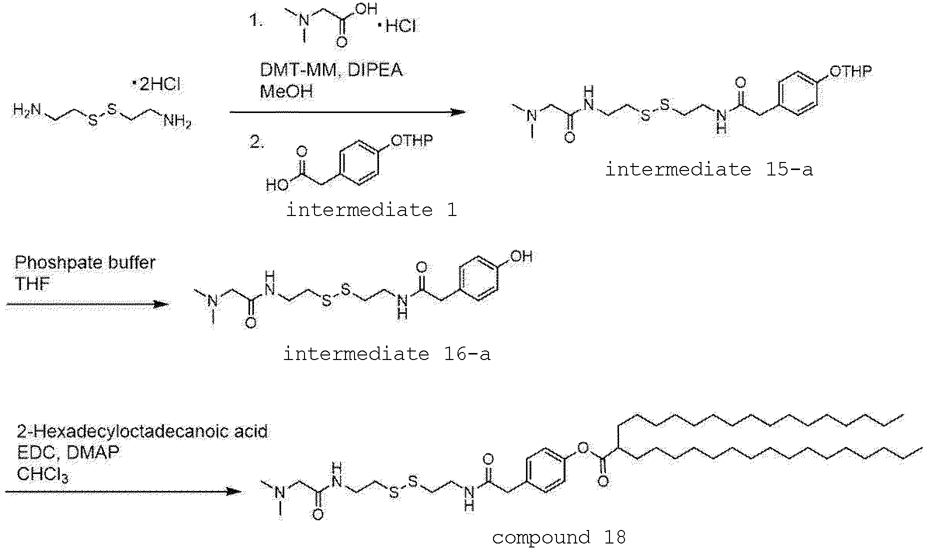

### [Production Example 18] Synthesis of compound 18

### <Synthesis of intermediate 15-a>

Cystamine dihydrochloride (227 mg), DIPEA (391 mg) and dimethylglycine hydrochloride (141 mg) were dissolved in 4.54 g of methanol at room temperature. DMT-MM (837 mg) was added to the obtained solution and the mixture was reacted at 25°C for 2 hr. Intermediate 1 (239 mg) was then added to the reaction solution and the mixture was reacted at 25°C for 2 hr. After concentrating the reaction solution, 3.40 g of chloroform was added, and the solution was washed with 2.27 g of 0.5 M phosphate buffer (pH 6.0), 2.27 g of 7 wt% sodium bicarbonate water, and 2.27 g of 20 wt% brine in that order. 100 mg of sodium sulfate was added to the obtained solution to dehydrate it, and sodium sulfate was removed by filtration. The filtrate was concentrated in an evaporator to obtain 748 mg of intermediate 15-a as a crude product.

### <Synthesis of intermediate 16-a>

In the same manner as in intermediate 4-a of Production Example 1 except that a crude product of intermediate 15-a was used, intermediate 16-a was synthesized.

### <Synthesis of compound 18>

Using intermediate 16-a and 2-hexadecyloctadecanoic acid, compound 18 was synthesized by the same synthetic route as in Production Example 1.

### <¹H-NMR(600MHz, CDCl₃) of compound 18>

δ:0.88(t, 6H), 1.20-1.45(m, 58H), 1.69-1.84(m, 2H), 2.30(s, 6H), 2.52-2.59(m, 1H), 2.76-2.82(m, 4H), 2.95(s, 2H), 3.50-3.60(m, 6H), 6.34(s, 1H), 7.02-7.05(m, 2H), 7.27-7.30(m, 2H), 7.50(s, 1H)

### [Production Example 19] Synthesis of compound 19

### <Synthesis of intermediate 15-b>

In the same manner as in Production Example 18 except that 3-(dimethylamino)propionic acid hydrochloride was used, intermediate 15-b represented by the following formula was synthesized.

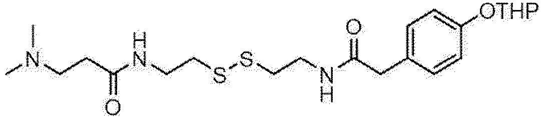

### <Synthesis of intermediate 16-b>

In the same manner as in Production Example 18 except that intermediate 15-b was used, intermediate 16-b represented by the following formula was synthesized.

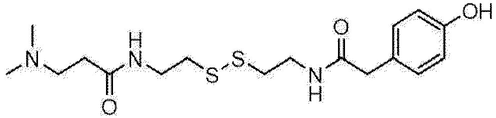

### <Synthesis of compound 19>

Using intermediate 16-b and 2-hexadecyloctadecanoic acid, compound 19 was synthesized by the same synthetic route as in Production Example 18.

### <¹H-NMR(600MHz, CDCl₃) of compound 19>

δ:0.88(t, 6H), 1.20-1.45(m, 58H), 1.69-1.84(m, 2H), 2.28(s, 6H), 2.33-2.38(m, 2H), 2.52-2.59(m, 3H), 2.76-2.82(m, 4H), 3.50-3.60(m, 6H), 6.34(s, 1H), 7.02-7.05(m, 2H), 7.27-7.30(m, 2H), 7.50(s, 1H)

### [Production Example 20] Synthesis of compound 20

### <Synthesis of intermediate 15-c>

In the same manner as in Production Example 18 except that 4-(dimethylamino)butanoic acid hydrochloride was used, intermediate 15-c represented by the following formula was synthesized.

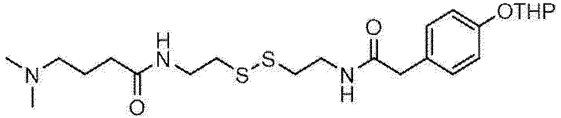

### <Synthesis of intermediate 16-c>

In the same manner as in Production Example 18 except that intermediate 15-c was used, intermediate 16-c represented by the following formula was synthesized.

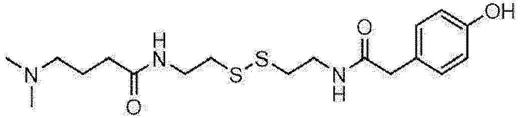

### <Synthesis of compound 20>

Using intermediate 16-c and 2-hexadecyloctadecanoic acid, compound 20 was synthesized by the same synthetic route as in Production Example 18.

### <¹H-NMR(600MHz, CDCl₃) of compound 20>

δ:0.88(t, 6H), 1.20-1.45(m, 58H), 1.69-1.80(m, 4H), 2.25-2.38(m, 8H), 2.52-2.59(m, 1H), 2.76-2.82(m, 4H), 3.50-3.60(m, 6H), 6.34(s, 1H), 7.02-7.05(m, 2H), 7.27-7.30(m, 2H), 7.50(s, 1H)

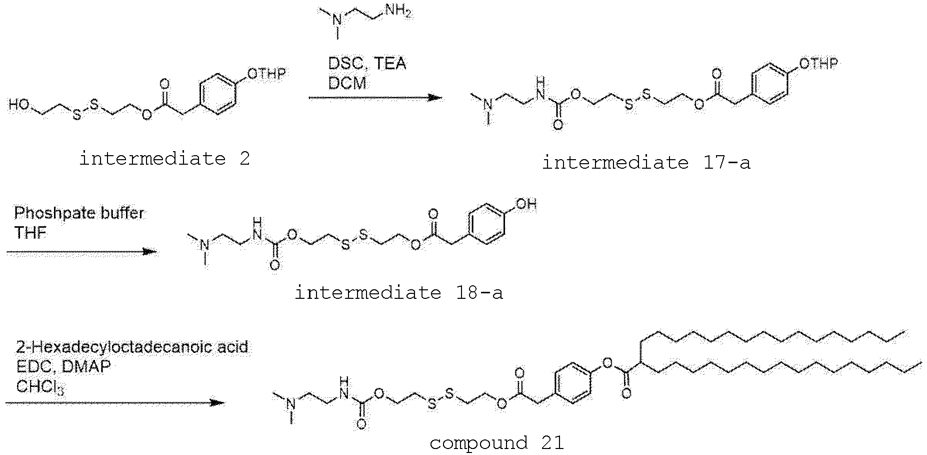

### [Production Example 21] Synthesis of compound 21

### <Synthesis of intermediate 17-a>

7.10 g of dichloromethane was added to intermediate 2 (393 mg) and triethylamine (235 mg) and dissolved at room temperature. DSC (541 mg) was added to the obtained solution and the mixture was reacted at 25°C for 2 hr. After removing the DSC remaining in the reaction solution by filtration, N,N-dimethylethylenediamine (112 mg) was added to the filtrate and the mixture was reacted at 25°C for 1 hr. After the reaction solution was washed with 5.90 g of 0.5 M acetate buffer (pH 4.0), 5.90 g of 7 wt% sodium bicarbonate water, and 5.90 g of 20 wt% brine in that order, 200 mg of sodium sulfate was added for dehydration. After removing the sodium sulfate by filtration, the filtrate was concentrated in an evaporator to obtain 472 mg of intermediate 17-a.

### <Synthesis of intermediate 18-a>

In the same manner as in intermediate 4-a of Production Example 1 except that intermediate 17-a was used, intermediate 18-a was synthesized.

### <Synthesis of compound 21>

Using intermediate 18-a and 2-hexadecyloctadecanoic acid, compound 21 was synthesized by a method similar to that for compound 1 in Production Example 1.

### <¹H-NMR(600MHz, CDCl₃) of compound 21>

δ:0.88(t, 6H), 1.20-1.45(m, 58H), 1.69-1.84(m, 2H), 2.22(s, 6H), 2.38-2.42(m, 2H), 2.52-2.59(m, 1H), 2.88-2.97(m, 4H), 3.22-3.26(m, 2H), 3.63(s, 2H), 4.28-4.38(m, 4H), 5.26(s, 1H), 7.02-7.05(m, 2H), 7.27-7.30(m, 2H)

### [Production Example 22] Synthesis of compound 22

### <Synthesis of intermediate 17-b>

In the same manner as in Production Example 21 except that N,N-diethylethylenediamine was used, intermediate 17-b represented by the following formula was synthesized.

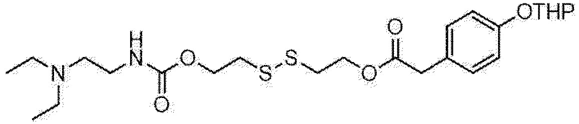

### <Synthesis of intermediate 18-b>

In the same manner as in Production Example 21 except that intermediate 17-b was used, intermediate 18-b represented by the following formula was synthesized.

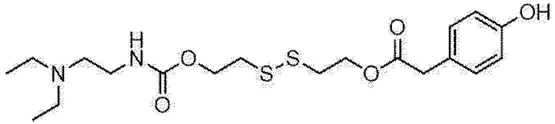

### <Synthesis of compound 22>

Using intermediate 18-b and 2-hexadecyloctadecanoic acid, compound 22 was synthesized by the same synthetic route as in Production Example 21.

### <¹H-NMR(600MHz, CDCl₃) of compound 22>

δ:0.88(t, 6H), 1.00(t, 6H), 1.20-1.45(m, 58H), 1.69-1.84(m, 2H), 2.47-2.57(m, 7H), 2.88-2.97(m, 4H), 3.22-3.26(m, 2H), 3.63(s, 2H), 4.28-4.38(m, 4H), 5.28(s, 1H), 7.02-7.05(m, 2H), 7.27-7.30(m, 2H)

### [Production Example 23] Synthesis of compound 23

### <Synthesis of intermediate 17-c>

In the same manner as in Production Example 21 except that N,N-dimethyl-1,3-propanediamine was used, intermediate 17-c represented by the following formula was synthesized.

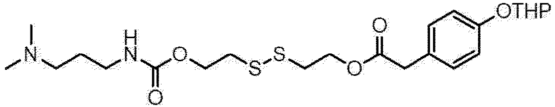

### <Synthesis of intermediate 18-c>

In the same manner as in Production Example 21 except that intermediate 17-c was used, intermediate 18-c represented by the following formula was synthesized.

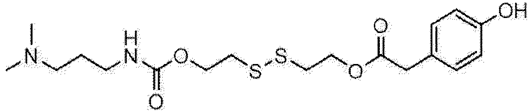

### <Synthesis of compound 23>

Using intermediate 18-c and 2-hexadecyloctadecanoic acid, compound 23 was synthesized by the same synthetic route as in Production Example 21.

### <¹H-NMR(600MHz, CDCl₃) of compound 23>

δ:0.88(t, 6H), 1.20-1.45(m, 58H), 1.69-1.84(m, 4H), 2.24(s, 6H), 2.38-2.42(m, 2H), 2.52-2.59(m, 1H), 2.88-2.97(m, 4H), 3.22-3.26(m, 2H), 3.63(s, 2H), 4.28-4.38(m, 4H), 5.70(s, 1H), 7.02-7.05(m, 2H), 7.27-7.30(m, 2H)

### [Production Example 24] Synthesis of compound 24

### <Synthesis of intermediate 17-d>

In the same manner as in Production Example 21 except that N,N,N'-trimethyl-1,3-propanediamine was used, intermediate 17-d represented by the following formula was synthesized.

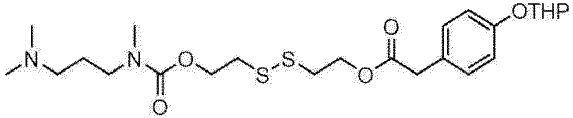

### <Synthesis of intermediate 18-d>

In the same manner as in Production Example 21 except that intermediate 17-d was used, intermediate 18-d represented by the following formula was synthesized.

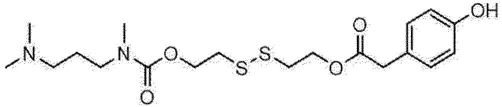

### <Synthesis of compound 24>

Using intermediate 18-d and 2-hexadecyloctadecanoic acid, compound 24 was synthesized by the same synthetic route as in Production Example 21.

### <¹H-NMR(600MHz, CDCl₃) of compound 24>

δ:0.88(t, 6H), 1.20-1.45(m, 58H), 1.69-1.84(m, 4H), 2.24(s, 6H), 2.38-2.42(m, 2H), 2.52-2.59(m, 1H), 2.88-2.97(m, 7H), 3.22-3.26(m, 2H), 3.63(s, 2H), 4.28-4.38(m, 4H), 7.02-7.05(m, 2H), 7.27-7.30(m, 2H)

### [Production Example 25] Synthesis of compound 25

### <Synthesis of intermediate 17-e>

In the same manner as in Production Example 21 except that N,N-dimethyl-1,4-butanediamine was used, intermediate 17-e represented by the following formula was synthesized.

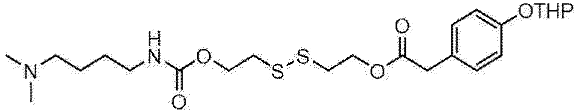

### <Synthesis of intermediate 18-e>

In the same manner as in Production Example 21 except that intermediate 17-e was used, intermediate 18-e represented by the following formula was synthesized.

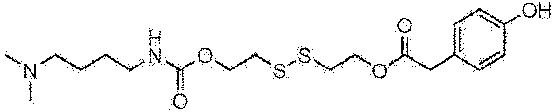

### <Synthesis of compound 25>

Using intermediate 18-e and 2-hexadecyloctadecanoic acid, compound 25 was synthesized by the same synthetic route as in Production Example 21.

### <¹H-NMR(600MHz, CDCl₃) of compound 25>

δ:0.88(t, 6H), 1.20-1.45(m, 58H), 1.50-1.62(m, 4H), 1.69-1.84(m, 2H), 2.20-2.42(m, 8H), 2.52-2.59(m, 1H), 2.88-2.97(m, 4H), 3.22-3.26(m, 2H), 3.63(s, 2H), 4.28-4.38(m, 4H), 5.70(s, 1H), 7.02-7.05(m, 2H), 7.27-7.30(m, 2H)

### [Production Example 26] Synthesis of compound 26

### <Synthesis of intermediate 17-f>

In the same manner as in Production Example 21 except that 4-(2-aminoethyl)morpholine was used, intermediate 17-f represented by the following formula was synthesized.

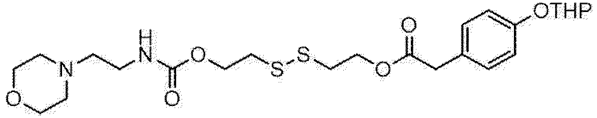

### <Synthesis of intermediate 18-f>

In the same manner as in Production Example 21 except that intermediate 17-f was used, intermediate 18-f represented by the following formula was synthesized.

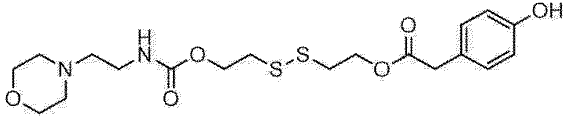

### <Synthesis of compound 26>

Using intermediate 18-f and 2-hexadecyloctadecanoic acid, compound 26 was synthesized by the same synthetic route as in Production Example 21.

### <¹H-NMR(600MHz, CDCl₃) of compound 26>

δ:0.88(t, 6H), 1.20-1.45(m, 58H), 1.69-1.84(m, 2H), 2.40-2.46(m, 6H), 2.52-2.59(m, 1H), 2.88-2.97(m, 4H), 3.22-3.26(m, 2H), 3.63(s, 2H), 4.28-4.38(m, 4H), 5.26(s, 1H), 7.02-7.05(m, 2H), 7.27-7.30(m, 2H)

### [Production Example 27] Synthesis of compound 27

### <Synthesis of intermediate 17-g>

In the same manner as in Production Example 21 except that N,N-dibutylethylenediamine was used, intermediate 17-g represented by the following formula was synthesized.

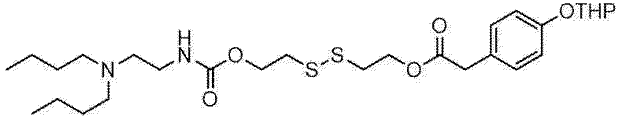

### <Synthesis of intermediate 18-g>

In the same manner as in Production Example 21 except that intermediate 17-g was used, intermediate 18-g represented by the following formula was synthesized.

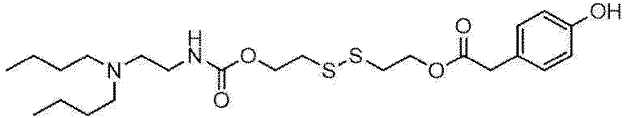

### <Synthesis of compound 27>

Using intermediate 18-g and 2-hexadecyloctadecanoic acid, compound 27 was synthesized by the same synthetic route as in Production Example 21.

### <¹H-NMR(600MHz, CDCl₃) of compound 27>

δ:0.87-0.93(m, 12H), 1.20-1.45(m, 66H), 1.69-1.84(m, 2H), 2.39-2.57(m, 7H), 2.88-2.97(m, 4H), 3.22-3.26(m, 2H), 3.63(s, 2H), 4.28-4.38(m, 4H), 5.28(s, 1H), 7.02-7.05(m, 2H), 7.27-7.30(m, 2H)

### [Production Example 28] Synthesis of compound 28

### <Synthesis of intermediate 17-h>

In the same manner as in Production Example 21 except that N,N-diisopropylethylenediamine was used, intermediate 17-h represented by the following formula was synthesized.

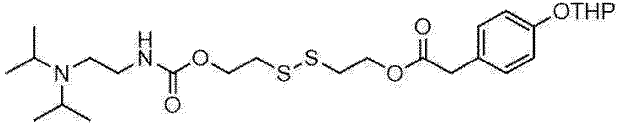

### <Synthesis of intermediate 18-h>

In the same manner as in Production Example 21 except that intermediate 17-h was used, intermediate 18-h represented by the following formula was synthesized.

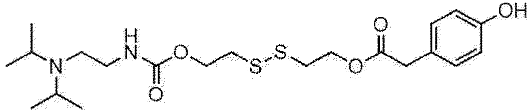

### <Synthesis of compound 28>

Using intermediate 18-h and 2-hexadecyloctadecanoic acid, compound 28 was synthesized by the same synthetic route as in Production Example 21.

### <¹H-NMR(600MHz, CDCl₃) of compound 28>

δ:0.88(t, 6H), 0.95-1.03(d, 12H), 1.20-1.45(m, 58H), 1.60-1.84(m, 6H), 2.52-2.59(m, 1H), 2.88-3.00(m, 6H), 3.22-3.26(m, 2H), 3.63(s, 2H), 4.28-4.38(m, 4H), 5.28(s, 1H), 7.02-7.05(m, 2H), 7.27-7.30(m, 2H)

### [Production Example 29] Synthesis of compound 29

### <Synthesis of intermediate 17-i>

In the same manner as in Production Example 21 except that N,N-diethyl-N'-methylethylenediamine was used, intermediate 17-i represented by the following formula was synthesized.

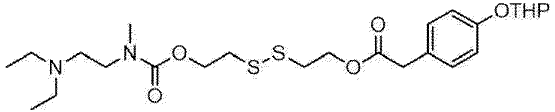

### <Synthesis of intermediate 18-i>

In the same manner as in Production Example 21 except that intermediate 17-i was used, intermediate 18-i represented by the following formula was synthesized.

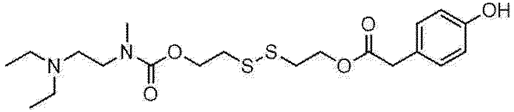

### <Synthesis of compound 29>

Using intermediate 18-i and 2-hexadecyloctadecanoic acid, compound 29 was synthesized by the same synthetic route as in Production Example 21.

### <¹H-NMR(600MHz, CDCl₃) of compound 29>

δ:0.88(t, 6H), 1.00(t, 6H), 1.20-1.45(m, 58H), 1.69-1.84(m, 2H), 2.47-2.57(m, 7H), 2.88-2.97(m, 7H), 3.22-3.26(m, 2H), 3.63(s, 2H), 4.28-4.38(m, 4H), 5.28(s, 1H), 7.02-7.05(m, 2H), 7.27-7.30(m, 2H)

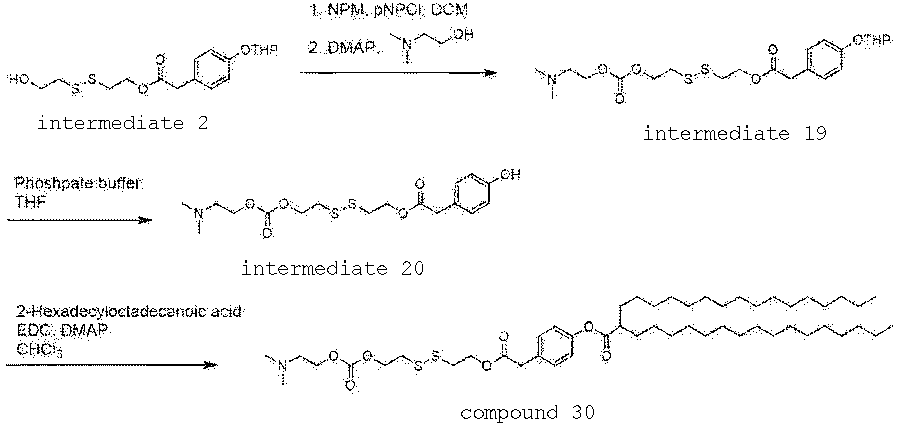

### [Production Example 30] Synthesis of compound 30

### <Synthesis of intermediate 19>

5.10 g of dichloromethane was added to intermediate 2 (510 mg) and NPM (670 mg) and dissolved at room temperature. pNPCl (690 mg) was added to the obtained solution and the mixture was reacted at 25°C for 2 hr. DMAP (33.0 mg) and 2-(dimethylamino)ethanol (976 mg) were added to this reaction solution and the mixture was reacted at 25°C for 6 hr. After the reaction, the solution was washed with 5.10 g of 0.5 M acetate buffer (pH 4.0), 5.10 g of 7 wt% sodium bicarbonate water, and 5.10 g of 20 wt% brine, in that order, and then dehydrated by adding 250 mg of sodium sulfate. After removing the sodium sulfate by filtration, the filtrate was concentrated in an evaporator to obtain 287 mg of intermediate 19.

### <Synthesis of intermediate 20>

In the same manner as in intermediate 4-a of Production Example 1 except that intermediate 19 was used, intermediate 20 was synthesized.

### <Synthesis of compound 30>

Using intermediate 20 and 2-hexadecyloctadecanoic acid, compound 30 was synthesized by a method similar to that for compound 1 in Production Example 1.

### <¹H-NMR(600MHz, CDCl₃) of compound 30>

δ:0.88(t, 6H), 1.20-1.45(m, 58H), 1.69-1.84(m, 2H), 2.28(s, 6H), 2.58-2.61(m, 3H), 2.91-2.94(m, 4H), 3.63(s, 2H), 4.23(t, 2H), 4.32-4.38(m, 4H), 7.02-7.05(m, 2H), 7.27-7.30(m, 2H)

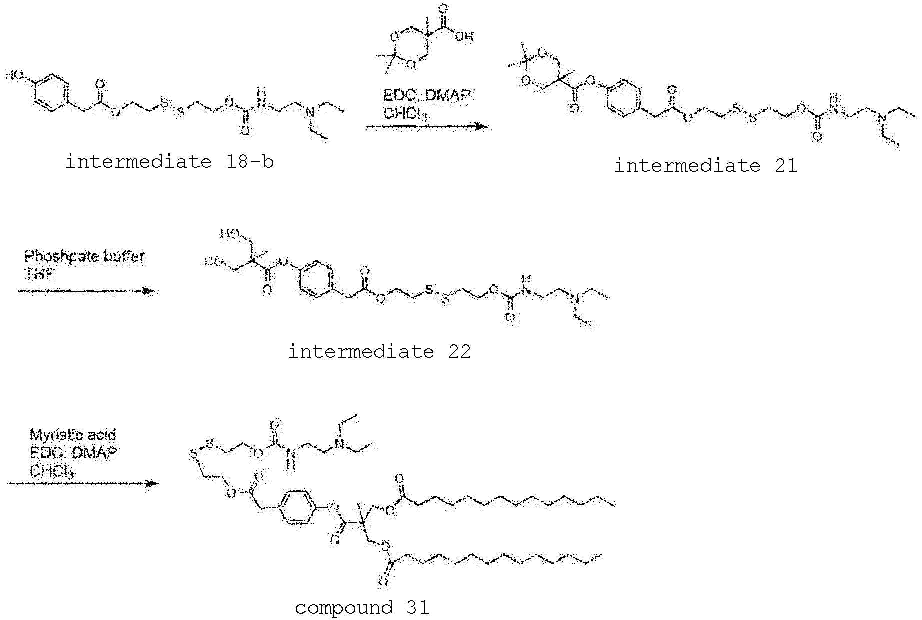

### [Production Example 31] Synthesis of compound 31

### <Synthesis of intermediate 21>

Intermediate 18-b (1.00 g), 2,2,5-trimethyl-1,3-dioxane-5-carboxylic acid (445 mg) and DMAP (57.0 mg) were dissolved in 15.0 g of chloroform at room temperature. EDC (668 mg) was added to the obtained solution and the mixture was reacted at 25°C for 2 hr. The reaction solution was washed with 10.0 g of 20 wt% brine, and then dehydrated by adding 500 mg of sodium sulfate. After removing the sodium sulfate by filtration, the filtrate was concentrated in an evaporator and purified with a column to obtain 1.25 g of intermediate 21.

### <Synthesis of intermediate 22>

Intermediate 21 (1.25 g) was dissolved in 11.3 g of THF, and then 45.0 g of 0.5 M phosphate buffer (pH 2.0) was added and the mixture was reacted at 40°C for 22 hr. 18.8 g of chloroform was added to the reaction solution for washing. After washing, 400 g/L aqueous sodium hydroxide solution was added to the aqueous layer, and the pH was adjusted to 6.0, and the aqueous layer was extracted with 18.8 g of chloroform. 600 mg of sodium sulfate was added to the extracted organic layer for dehydration, and sodium sulfate was removed by filtration. The filtrate was concentrated in an evaporator to obtain 908 mg of intermediate 22.

### <Synthesis of compound 31>

Intermediate 22 (200 mg), myristic acid (175 mg), and DMAP (18.0 mg) were dissolved in 3.00 g of chloroform at room temperature. EDC (175 mg) was added to the obtained solution and the mixture was reacted at 25°C for 2 hr. The reaction solution was concentrated using an evaporator and purified with a column to obtain 106 mg of compound 31.

### <¹H-NMR(600MHz, CDCl₃) of compound 31>

δ:0.89(t, 6H), 1.04(t, 6H), 1.25-1.35(m, 40H), 1.42(s, 3H), 1.55-1.65(m, 4H), 2.36(t, 4H), 2.52-2.58(m, 6H), 2.92-2.96(m, 4H), 3.22-3.26(m, 2H), 3.66(s, 2H), 4.30-4.40(m, 8H), 5.28(s, 1H), 7.02-7.05(m, 2H), 7.27-7.30(m, 2H)

### [Production Example 32] Synthesis of compound 32

### <Synthesis of compound 32>

Using intermediate 22 and palmitic acid, compound 32 was synthesized by the same synthetic route as in Production Example 31.

### <¹H-NMR(600MHz, CDCl₃) of compound 32>

δ:0.89(t, 6H), 1.04(t, 6H), 1.25-1.35(m, 48H), 1.42(s, 3H), 1.55-1.65(m, 4H), 2.36(t, 4H), 2.52-2.58(m, 6H), 2.92-2.96(m, 4H), 3.22-3.26(m, 2H), 3.66(s, 2H), 4.30-4.40(m, 8H), 5.28(s, 1H), 7.02-7.05(m, 2H), 7.27-7.30(m, 2H)

### [Production Example 33] Synthesis of compound 33

### <Synthesis of compound 33>

Using intermediate 22 and stearic acid, compound 33 was synthesized by the same synthetic route as in Production Example 31.

### <¹H-NMR(600MHz, CDCl₃) of compound 33>

δ:0.89(t, 6H), 1.04(t, 6H), 1.25-1.35(m, 56H), 1.42(s, 3H), 1.55-1.65(m, 4H), 2.36(t, 4H), 2.52-2.58(m, 6H), 2.92-2.96(m, 4H), 3.22-3.26(m, 2H), 3.66(s, 2H), 4.30-4.40(m, 8H), 5.28(s, 1H), 7.02-7.05(m, 2H), 7.27-7.30(m, 2H)

### [Production Example 34] Synthesis of compound 34

### <Synthesis of compound 34>

Using intermediate 22 and oleic acid, compound 34 was synthesized by the same synthetic route as in Production Example 31.

### <¹H-NMR(600MHz, CDCl₃) of compound 34>

δ:0.89(t, 6H), 1.04(t, 6H), 1.25-1.38(m, 40H), 1.42(s, 3H), 1.55-1.65(m, 4H), 1.99-2.05(m, 8H), 2.36(t, 4H), 2.52-2.58(m, 6H), 2.92-2.96(m, 4H), 3.22-3.26(m, 2H), 3.66(s, 2H), 4.30-4.40(m, 8H), 5.32-5.38(m, 5H), 7.02-7.05(m, 2H), 7.27-7.30(m, 2H)

### [Production Example 35] Synthesis of compound 35

### <Synthesis of compound 35>

Using intermediate 22 and linoleic acid, compound 35 was synthesized by the same synthetic route as in Production Example 31.

### <¹H-NMR(600MHz, CDCl₃) of compound 35>

δ:0.89(t, 6H), 1.04(t, 6H), 1.25-1.38(m, 28H), 1.42(s, 3H), 1.55-1.65(m, 4H), 1.99-2.05(m, 8H), 2.36(t, 4H), 2.52-2.58(m, 6H), 2.75-2.78(m, 4H), 2.92-2.96(m, 4H), 3.22-3.26(m, 2H), 3.66(s, 2H), 4.30-4.40(m, 8H), 5.32-5.38(m, 9H), 7.02-7.05(m, 2H), 7.27-7.30(m, 2H)

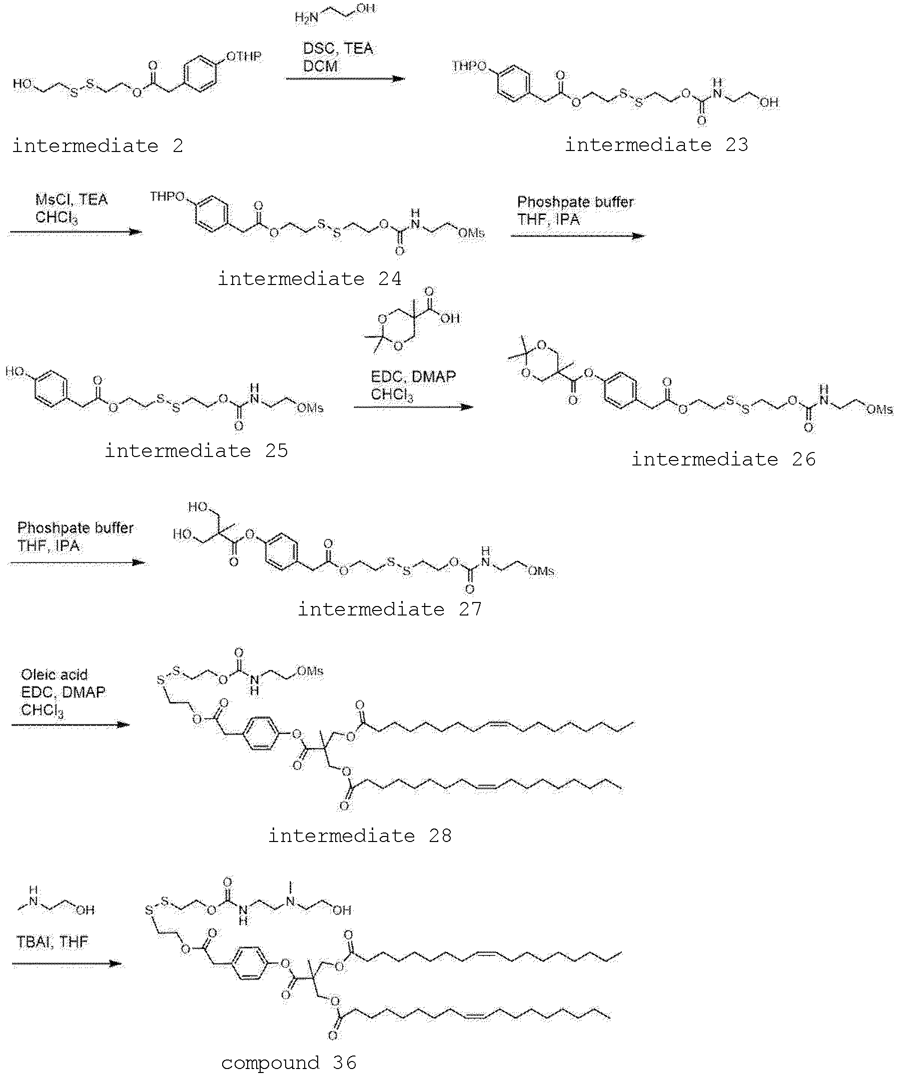

### [Production Example 36] Synthesis of compound 36

### <Synthesis of intermediate 23>

Intermediate 2 (2.0 g) and triethylamine (1.20 g) were added with 36.0 g of dichloromethane and dissolved at room temperature. DSC (2.75 g) was added to the obtained solution and the mixture was reacted at 25°C for 2 hr. After removing the DSC remaining in the reaction solution by filtration, ethanolamine (394 mg) was added to the filtrate and the mixture was reacted at 25°C for 1 hr. The solution after the reaction was washed with 30.0 g of 0.5 M acetate buffer (pH 4.0), and then 1.00 g of sodium sulfate was added to dehydrate it. After removing the sodium sulfate by filtration, the filtrate was concentrated using an evaporator to obtain 1.41 g of intermediate 23.

### <Synthesis of intermediate 24>

Intermediate 23 (1.35 g) and triethylamine (594 mg) were dissolved in 20.0 g of chloroform at room temperature. Methanesulfonyl chloride (505 mg) was added to the obtained solution and the mixture was reacted at room temperature for 3 hr. The reaction solution was washed with 15.0 g of 7 wt% sodium bicarbonate water, and 500 mg of sodium sulfate was added for dehydration. After removing the sodium sulfate by filtration, the filtrate was concentrated in an evaporator to obtain 1.34 g of intermediate 24.

### <Synthesis of intermediate 25>

Intermediate 2 (1.31 g) was dissolved in a mixed solvent of 11.8 g of THF and 13.1 g of IPA, and then 47.2 g of 0.5 M phosphate buffer (pH 2.0) was added and the mixture was reacted at 40°C for 2 hr. The reaction solution was washed with 19.7 g of chloroform. After washing, 400 g/L aqueous sodium hydroxide solution was added to the aqueous layer to neutralize it, and then 19.7 g of chloroform was added to the aqueous layer for extraction. 3.0 g of sodium sulfate was added to the extracted organic layer to dehydrate it, and sodium sulfate was removed by filtration. The filtrate was concentrated in an evaporator to obtain 939 mg of intermediate 25.

### <Synthesis of intermediate 26>

Intermediate 25 (930 mg), 2,2,5-trimethyl-1,3-dioxane-5-carboxylic acid (393 mg) and DMAP (50.0 mg) were dissolved in 15 g of chloroform at room temperature. EDC (590 mg) was added to the obtained solution and the mixture was reacted at 25°C for 2 hr. The reaction solution was washed with 9.30 g of 20 wt% brine, and then 500 mg of sodium sulfate was added to dehydrate it. After sodium sulfate was removed by filtration, the filtrate was concentrated in an evaporator and purified with a column to obtain 1.06 g of intermediate 26.

### <Synthesis of intermediate 27>

Intermediate 26 (1.00 g) was dissolved in a mixed solvent of 9.00 g THF and 9.00 g IPA, and then 40.0 g of 0.5 M phosphate buffer (pH 2.0) was added and the mixture was reacted at 40°C for 2 hr. 15.0 g of chloroform was added to the reaction solution for washing. After washing, 400 g/L aqueous sodium hydroxide solution was added to the aqueous layer to neutralize it, and 15.0 g of chloroform was added to the aqueous layer for extraction. 500 mg of sodium sulfate was added to the organic layer for dehydration, and sodium sulfate was removed by filtration. The filtrate was concentrated in an evaporator to obtain 785 mg of intermediate 27.

### <Synthesis of intermediate 28>

Intermediate 27 (770 mg), oleic acid (802 mg), and DMAP (66.0 mg) were dissolved in 11.6 g of chloroform at room temperature. EDC (648 mg) was added to the obtained solution and the mixture was reacted at 25°C for 2 hr. The reaction solution was washed with 8.00 g of 20 wt% brine, and then 2.0 g of sodium sulfate was added for dehydration. After removing the sodium sulfate by filtration, the filtrate was concentrated in an evaporator and purified with a column to obtain 1.15 g of intermediate 28.

### <Synthesis of compound 36>

Intermediate 28 (250 mg) was dissolved in 2.50 g of THF, and 2-(methylamino)ethanol (137 mg) and TBAI (17.0 mg) were added and the mixture was reacted at 40°C for 8 hr. 3.75 g of chloroform was added to the reaction solution, which was washed in the order of 2.50 g of 0.5 M acetate buffer (pH 4.0), 2.50 g of 7 wt% sodium bicarbonate water, and 2.50 g of 20 wt% brine. The filtrate was concentrated in an evaporator and purified with a column to obtain 79 mg of compound 36.

### <¹H-NMR(600MHz, CDCl₃) of compound 36>

δ:0.89(t, 6H), 1.25-1.38(m, 40H), 1.42(s, 3H), 1.55-1.65(m, 4H), 1.99-2.05(m, 8H), 2.30-2.40(m, 7H), 2.52-2.58(m, 4H), 2.92-2.96(m, 4H), 3.22-3.26(m, 2H), 3.58-3.63(m, 4H), 4.30-4.40(m, 8H), 5.32-5.38(m, 5H), 7.02-7.05(m, 2H), 7.27-7.30(m, 2H)

### [Production Example 37] Synthesis of compound 37

### <Synthesis of compound 37>

Using intermediate 28 and 3-(methylamino)-1-propanol, compound 37 was synthesized by the same synthetic route as in Production Example 36.

### <¹H-NMR(600MHz, CDCl₃) of compound 37>

δ:0.89(t, 6H), 1.25-1.38(m, 40H), 1.42(s, 3H), 1.55-1.72(m, 6H), 1.99-2.05(m, 8H), 2.30-2.40(m, 7H), 2.52-2.58(m, 4H), 2.92-2.96(m, 4H), 3.22-3.26(m, 2H), 3.58-3.63(m, 4H), 4.30-4.40(m, 8H), 5.32-5.38(m, 5H), 7.02-7.05(m, 2H), 7.27-7.30(m, 2H)

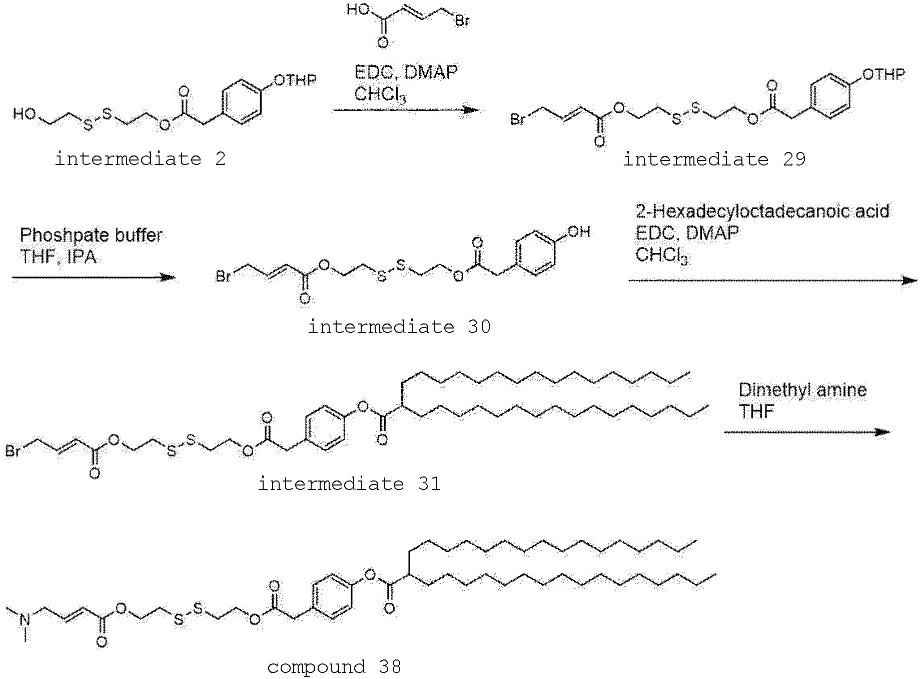

### [Production Example 38] Synthesis of compound 38

### <Synthesis of intermediate 29>

Intermediate 2 (2.00 g), (E)-4-bromocrotonic acid (974 mg), and DMAP (131 mg) were dissolved in 30.0 g of chloroform at room temperature. EDC (1.54 g) was added to the obtained solution and the mixture was reacted at 25°C for 2 hr. The reaction solution was washed with 9.0 mL of 20 wt% brine, and then 2.0 g of sodium sulfate was added for dehydration. After removing the sodium sulfate by filtration, the filtrate was concentrated in an evaporator to obtain 2.26 g of Intermediate 29.

### <Synthesis of intermediate 30>

Intermediate 29 (2.25 g) was dissolved in a mixed solvent of 20.23 g of THF and 22.5 g of IPA, and then 81.0 g of 0.5 M phosphate buffer (pH 2.0) was added and the mixture was reacted at 40°C for 2 hr. The reaction solution was washed with 34.0 g of chloroform. After washing, 400 g/L aqueous sodium hydroxide solution was added to the aqueous layer to neutralize it, and then 34.0 g of chloroform was added to the aqueous layer for extraction. 1.00 g of sodium sulfate was added to the extracted organic layer to dehydrate it, and the sodium sulfate was removed by filtration. The filtrate was concentrated using an evaporator to obtain 1.51 g of intermediate 30.

### <Synthesis of intermediate 31>

Intermediate 30 (1.40 g), 2-hexadecyloctadecanoic acid (1.80 g), and DMAP (79.0 mg) were dissolved in 21.0 g of chloroform at room temperature. EDC (925 mg) was added to the obtained solution and the mixture was reacted at 25°C for 2 hr. The reaction solution was concentrated using an evaporator to obtain 2.98 g of a crude product of intermediate 31.

### <Synthesis of compound 38>

500 mg of the crude product of intermediate 31 and 2.30 g of dimethylamine (2.0 mol/L in THF) were dissolved in 5.00 g of THF, and potassium iodide (0.12 mmol) was added and the mixture was reacted at 25°C for 10 hr. After removing insoluble matter by filtration, 7.50 g of chloroform was added to the filtrate, the mixture was then washed with 5.00 g of 0.5 M acetate buffer (pH 4.0), 5.00 g of 7 wt% sodium bicarbonate water, and 5.00 g of 20 wt% brine, in that order. After adding 250 mg of sodium sulfate for dehydration, the sodium sulfate was removed by filtration. The filtrate was concentrated using an evaporator and purified with a column to obtain 270 mg of compound 38.

### <¹H-NMR(600MHz, CDCl₃) of compound 38>

δ:0.88(t, 6H), 1.20-1.45(m, 58H), 1.69-1.84(m, 2H), 2.25(s, 6H), 2.52-2.59(m, 1H), 2.88-2.97(m, 4H), 3.07(t, 2H), 3.63(s, 2H), 4.28-4.38(m, 4H), 5.97-6.00(m, 1H), 6.95-7.05(m, 3H), 7.27-7.30(m, 2H)

### [Production Example 39] Synthesis of compound 39

### <Synthesis of compound 39>

In the same manner as in Production Example 38 except that diethylamine was used, compound 39 was synthesized.

### <¹H-NMR(600MHz, CDCl₃) of compound 39>

δ:0.88(t, 6H), 1.03(t, 6H), 1.20-1.45(m, 58H), 1.69-1.84(m, 2H), 2.50-2.59(m, 5H), 2.88-2.97(m, 4H), 3.23(t, 2H), 3.63(s, 2H), 4.28-4.38(m, 4H), 5.97-6.00(m, 1H), 6.95-7.05(m, 3H), 7.27-7.30(m, 2H)

### [Production Example 40] Synthesis of compound 40

### <Synthesis of compound 40>

In the same manner as in Production Example 38 except that 2-(ethylamino)ethanol was used, compound 40 was synthesized.

### <¹H-NMR(600MHz, CDCl₃) of compound 40>

δ:0.88(t, 6H), 1.03(t, 3H), 1.20-1.45(m, 58H), 1.69-1.84(m, 2H), 2.50-2.59(m, 5H), 2.88-2.97(m, 4H), 3.23(t, 2H), 3.55-3.58(m, 2H), 3.63(s, 2H), 4.28-4.38(m, 4H), 5.97-6.00(m, 1H), 6.95-7.05(m, 3H), 7.27-7.30(m, 2H)

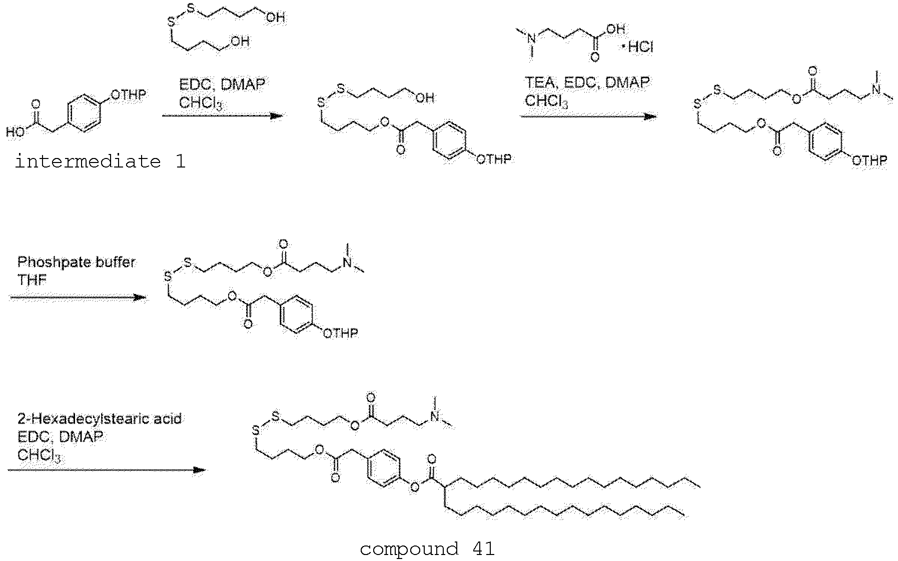

### [Production Example 41] Synthesis of compound 41

### <Synthesis of compound 41>

By a synthesis route similar to that in Production Example 1 except that intermediate 1, 4,4'-dithiobisbutan-1-ol and 4-(dimethylamino)butanoic acid hydrochloride were used as starting materials, compound 41 was synthesized.

### <¹H-NMR(600MHz, CDCl₃) of compound 41>

δ:0.88(t, 6H), 1.20-1.50(m, 64H), 1.69-1.84(m, 4H), 2.21(s, 6H), 2.28(t, 2H), 2.35(t, 2H), 2.52-2.59(m, 1H), 2.59-2.70(m, 4H), 3.63(s, 2H), 4.22-4.33(m, 4H), 7.02-7.05(m, 2H), 7.27-7.30(m, 2H)

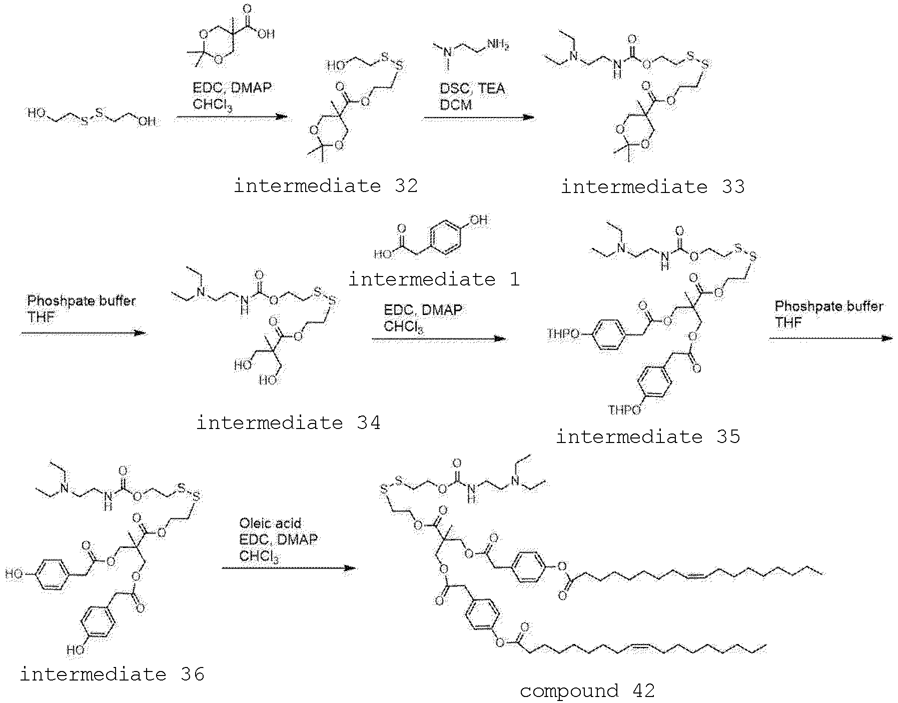

### [Production Example 42] Synthesis of compound 42

### <Synthesis of intermediate 32>

Bis(2-hydroxyethyl) disulfide (33.2 g), 2,2,5-trimethyl-1,3-dioxane-5-carboxylic acid (25.0 g) and DMAP (3.50 g) were dissolved in 250 g of chloroform at room temperature. EDC (41.0 g) was added to the obtained solution and the mixture was reacted at 25°C for 2 hr. The reaction solution was washed with 9.0 mL of 20 wt% brine, and then dehydrated by adding 25.0 g of sodium sulfate. After removing the sodium sulfate by filtration, the filtrate was concentrated in an evaporator and purified with a column to obtain 21.8 g of Intermediate 32.

### <Synthesis of intermediate 33>

180 g of dichloromethane was added to intermediate 32 (10.0 g) and triethylamine (7.17 g) and dissolved at room temperature. DSC (16.5 g) was added to the obtained solution and the mixture was reacted at 25°C for 2 hr. After removing the DSC remaining in the reaction solution by filtration, N,N-diethylethylenediamine (5.00 g) was added to the filtrate and the mixture was reacted at 25°C for 1 hr. After the reaction, the solution was washed with 150 g of 0.5 M acetate buffer (pH 4.0), 150 g of 7 wt% sodium bicarbonate water, and 150 g of 20 wt% brine, in that order, and then 5.00 g of sodium sulfate was added for dehydration. After removing the sodium sulfate by filtration, the filtrate was concentrated in an evaporator to obtain 13.1 g of intermediate 33.

### <Synthesis of intermediate 34>

Intermediate 33 (13.0 g) was dissolved in 49.0 g of THF, and then 195 g of 0.5 M phosphate buffer (pH 2.0) was added and the mixture was reacted at 40°C for 3 hr. 120 g of chloroform was added to the reaction solution for washing. After washing, 400 g/L aqueous sodium hydroxide solution was added to the aqueous layer, and the pH was adjusted to 7.0, and the aqueous layer was extracted with 195 g of chloroform. 6.50 g of sodium sulfate was added to the extracted organic layer for dehydration, and sodium sulfate was removed by filtration. The filtrate was concentrated in an evaporator to obtain 5.01 g of intermediate 34.

### <Synthesis of intermediate 35>

Intermediate 34 (5.00 g), intermediate 1 (6.01 g) and DMAP (592 mg) were dissolved in 75.0 g of chloroform at room temperature. EDC (5.81 g) was added to the obtained solution and the mixture was reacted at 25°C for 2 hr. The reaction solution was washed with 50.0 g of 20 wt% brine, and then 2.50 g of sodium sulfate was added for dehydration. After removing the sodium sulfate by filtration, the filtrate was concentrated in an evaporator to obtain 6.25 g of a crude product of intermediate 35.

### <Synthesis of intermediate 36>

6.25 g of the crude product of intermediate 35 was dissolved in 56.3 g of THF, and then 225 g of 0.5 M phosphate buffer (pH 2.0) was added and the mixture was reacted at 25°C for 2 hr. 94.0 g of chloroform was added to the reaction solution for washing. After washing, 400 g/L aqueous sodium hydroxide solution was added to the aqueous layer, and the pH was adjusted to 6.0, and the aqueous layer was extracted with 94.0 g of chloroform. 3.00 g of sodium sulfate was added to the extracted organic layer to dehydrate it, and sodium sulfate was removed by filtration. The filtrate was concentrated in an evaporator to obtain 3.7 g of intermediate 36.

### <Synthesis of compound 42>

Intermediate 36 (500 mg), oleic acid (436 mg) and DMAP (36.0 mg) were dissolved in 7.50 g of chloroform at room temperature. EDC (352 mg) was added to the obtained solution and the mixture was reacted at 25°C for 2 hr. The reaction solution was washed with 5.00 g of 20 wt% brine, and then dehydrated by adding 250 mg of sodium sulfate. After removing the sodium sulfate by filtration, the filtrate was concentrated in an evaporator and purified with a column to obtain 453 mg of compound 42.

### <¹H-NMR(600MHz, CDCl₃) of compound 42>

δ:0.88(t, 6H), 1.04(t, 6H), 1.25-1.38(m, 40H), 1.42(s, 3H), 1.55-1.65(m, 4H), 1.99-2.05(m, 8H), 2.36(t, 4H), 2.52-2.58(m, 6H), 2.92-2.96(m, 4H), 3.22-3.26(m, 2H), 3.66(s, 4H), 4.30-4.40(m, 8H), 5.32-5.38(m, 5H), 7.02-7.05(m, 4H), 7.27-7.30(m, 4H)

### [Production Example 43] Synthesis of compound 43

### <Synthesis of compound 43>

Using intermediate 36 and myristic acid, compound 43 was synthesized by the same synthetic route as in Production Example 42.

### <¹H-NMR(600MHz, CDCl₃) of compound 43>

δ:0.89(t, 6H), 1.04(t, 6H), 1.25-1.35(m, 40H), 1.42(s, 3H), 1.55-1.65(m, 4H), 2.36(t, 4H), 2.52-2.58(m, 6H), 2.92-2.96(m, 4H), 3.22-3.26(m, 2H), 3.66(s, 4H), 4.30-4.40(m, 8H), 5.32(s, 1H), 7.02-7.05(m, 4H), 7.27-7.30(m, 4H)

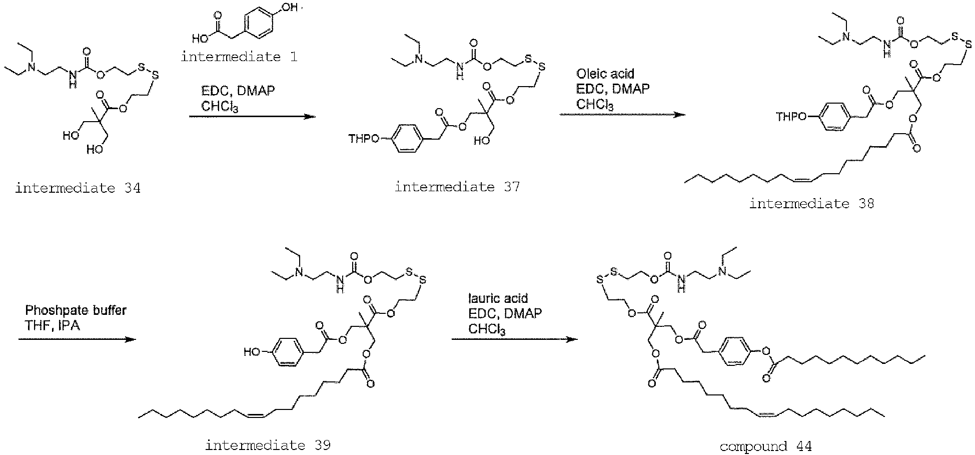

### [Production Example 44] Synthesis of compound 44

### <Synthesis of intermediate 37>

Intermediate 34 (1.60 g), intermediate 1 (4.19 g) and DMAP (165 mg) were dissolved in 24.0 g of chloroform at room temperature. EDC (1.95 g) was added to the obtained solution and the mixture was reacted at 25°C for 2 hr. The reaction solution was washed with 16.0 g of 20 wt% brine, and then 500 mg of sodium sulfate was added for dehydration. After removing the sodium sulfate by filtration, the filtrate was concentrated in an evaporator and purified with a column to obtain 1.85 g of intermediate 37.

### <Synthesis of intermediate 38>

Intermediate 37 (450 mg), oleic acid (241 mg) and DMAP (19.0 mg) were dissolved in 6.75 g of chloroform at room temperature. EDC (223 mg) was added to the obtained solution and the mixture was reacted at 25°C for 2 hr. The reaction solution was washed with 4.5 g of 20 wt% brine, and then 2.0 g of sodium sulfate was added for dehydration. After removing the sodium sulfate by filtration, the filtrate was concentrated in an evaporator to obtain 670 mg of a crude product of intermediate 38.

### <Synthesis of intermediate 39>

The crude product of intermediate 38 (670 mg) was dissolved in a mixed solvent of 4.00 g THF and 4.00 g IPA, and then 16.2 g of 0.5 M phosphate buffer (pH 2.0) was added and the mixture was reacted at 40°C for 3 hr. After adding 13.5 g of chloroform to the reaction solution for extraction, the organic layer was washed with 9.00 g of 0.5 M phosphate buffer (pH 6.5). After washing, 300 mg of sodium sulfate was added to the organic layer for dehydration, and sodium sulfate was removed by filtration. The filtrate was concentrated in an evaporator and purified with a column to obtain 240 mg of intermediate 39.

### <Synthesis of compound 44>

Intermediate 39 (150 mg), lauric acid (41.0 mg) and DMAP (5.0 mg) were dissolved in 2.25 g of chloroform at room temperature. EDC (53.0 mg) was added to the obtained solution and the mixture was reacted at 25°C for 2 hr. The reaction solution was washed with 1.50 g of 20 wt% brine, and then 50.0 mg of sodium sulfate was added for dehydration. After removing the sodium sulfate by filtration, the filtrate was concentrated in an evaporator and purified with a column to obtain 116 mg of compound 44.

### <¹H-NMR(600MHz, CDCl₃) of compound 44>

δ:0.80-0.92(m, 6H), 1.04(t, 6H), 1.25-1.38(m, 36H), 1.42(s, 3H), 1.55-1.65(m, 4H), 1.99-2.05(m, 4H), 2.36(t, 2H), 2.52-2.58(m, 6H), 2.92-2.96(m, 4H), 3.22-3.26(m, 2H), 3.66(s, 4H), 4.30-4.40(m, 8H), 5.32-5.36(m, 3H), 7.02-7.05(m, 2H), 7.27-7.30(m, 2H)

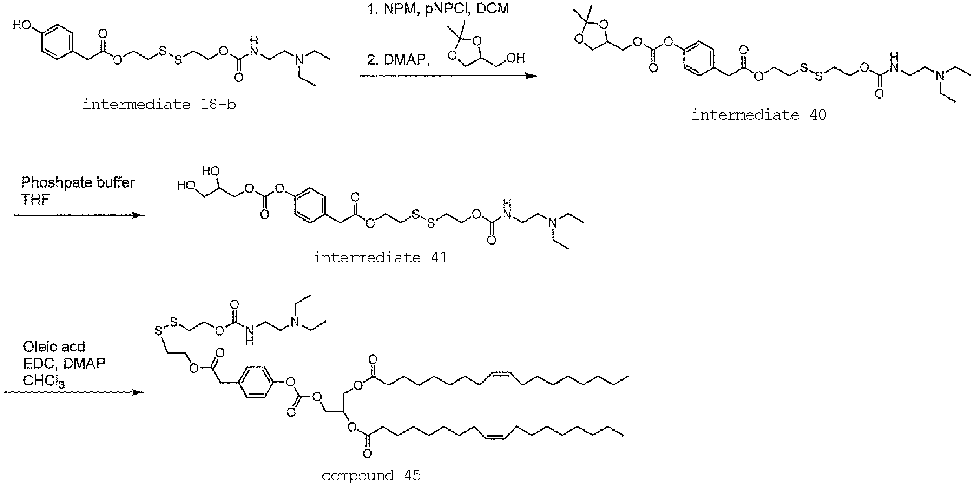

### [Production Example 45] Synthesis of compound 45

### <Synthesis of intermediate 40>

Intermediate 18-b (3.00 g) and NPM (3.41 g) were added with 30.0 g of dichloromethane and dissolved at room temperature. pNPCl (3.51 g) was added to the obtained solution and the mixture was reacted at 25°C for 10 hr. DMAP (170 mg) and 1,2-isopropylidene glycol (7.37 g) were added to this reaction solution and reacted at 25°C for 8 hr. The reaction solution was washed with 30.0 g of 7 wt% sodium bicarbonate water and 30.0 g of 20 wt% brine in that order, and then 9.00 g of sodium sulfate was added for dehydration. After removing the sodium sulfate by filtration, the filtrate was concentrated in an evaporator and purified with a column to obtain 2.16 g of intermediate 40.

### <Synthesis of intermediate 41>

Intermediate 40 (2.16 g) was dissolved in 11.3 g of THF, and then 45.0 g of 0.5 M phosphate buffer (pH 2.0) was added and the mixture was reacted at 40°C for 2 hr. 19.0 g of chloroform was added to the reaction solution for washing. After washing, 400 g/L aqueous sodium hydroxide solution was added to the aqueous layer, and the pH was adjusted to 6.0, and the aqueous layer was extracted with 19.0 g of chloroform. 1.20 g of sodium sulfate was added to the extracted organic layer to dehydrate it, and sodium sulfate was removed by filtration. The filtrate was concentrated using an evaporator to obtain 850 mg of intermediate 41.

### <Synthesis of compound 45>

By a synthesis route similar to that in Production Example 42 except that intermediate 41 and oleic acid were used, compound 45 was synthesized.

### <¹H-NMR(600MHz, CDCl₃) of compound 45>

δ:0.89(t, 6H), 1.04(t, 6H), 1.25-1.38(m, 40H), 1.42(s, 3H), 1.55-1.65(m, 4H), 1.99-2.05(m, 8H), 2.36(t, 4H), 2.52-2.58(m, 6H), 2.92-2.96(m, 4H), 3.22-3.26(m, 2H), 3.66(s, 2H), 4.30-4.50(m, 8H), 5.28-5.38(m, 6H), 6.97-7.00(m, 2H), 7.27-7.30(m, 2H)

### [Production Example 46] Synthesis of compound 46

### <Synthesis of compound 46>

By a synthesis route similar to that in Production Example 42 except that intermediate 41 and myristic acid were used, compound 46 was synthesized.

### <¹H-NMR(600MHz, CDCl₃) of compound 46>

δ:0.89(t, 6H), 1.04(t, 6H), 1.25-1.35(m, 32H), 1.42(s, 3H), 1.55-1.65(m, 4H), 2.36(t, 4H), 2.52-2.58(m, 6H), 2.92-2.96(m, 4H), 3.22-3.26(m, 2H), 3.66(s, 2H), 4.30-4.50(m, 8H), 5.28-5.31(m, 2H), 6.97-7.00(m, 2H), 7.27-7.30(m, 2H)

### [Comparative Example] Synthesis of O-Ph-P4C2

The title compound was synthesized by the synthesis route described in Production Example 3 of Patent Literature 3.

### <¹H-NMR(600MHz, CDCl₃) of O-Ph-P4C2>

δ0.88(t, 6H), 1.22-1.42(m, 46H), 1.54-1.76(m, 12H), 1.94-2.03(m, 12H), 2.52-2.56(m, 4H), 2.62-2.66(m, 4H), 2.80-2.89(m, 8H), 3.59(s, 4H), 4.13(t, 4H), 5.34-5.37(m, 4H), 7.02-7.05(m, 4H), 7.27-7.30(m, 4H)

### [Industrial Applicability]

According to the present invention, since nucleic acid can be intracellularly introduced with high efficiency, the present invention is useful for nucleic acid medicaments, gene therapy and biochemical experiments.

This application is based on patent application No. 2022-051913 filed in Japan, the contents of which are encompassed in full herein.

## Claims

1. A method for producing nucleic acid-encapsulated lipid nanoparticles, comprising the following steps:
a) a step of preparing a suspension of lipid nanoparticles not containing a nucleic acid, by mixing an alcohol solution comprising ionic lipid, sterol and PEG lipid with an acidic buffer having a buffering action at pH 1 to 6.5, and
b) a step of mixing, without lyophilization, the suspension of the lipid nanoparticles obtained in step a with an aqueous solution comprising a nucleic acid and optionally containing 0 to 25 v/v% alcohol, and optionally incubating the mixture at 0 to 95°C for 0 to 60 min to obtain nucleic acid-encapsulated lipid nanoparticles.

2. The production method according to claim 1, comprising the following step c after step b:
c) a step of exchanging an external aqueous phase of the obtained nucleic acid-encapsulated lipid nanoparticles with a neutral buffer by dialysis, ultrafiltration or dilution.

3. The production method according to claim 1 or 2, wherein step a further comprises a step of freezing the lipid nanoparticles not containing a nucleic acid at -80 to 0°C and then thawing the lipid nanoparticles at 0 to 95°C.

4. The production method according to any one of claims 1 to 3, wherein step a further comprises, after preparation of the suspension of the lipid nanoparticles, exchanging the external aqueous phase with another acidic buffer having a buffering action at pH 1 to 6.5 by dialysis, ultrafiltration or dilution.

5. The production method according to any one of claims 1 to 4, wherein the alcohol solution further comprises phospholipid in step a.

6. The production method according to any one of claims 1 to 5, wherein the ionic lipid is a compound represented by the formula (1): 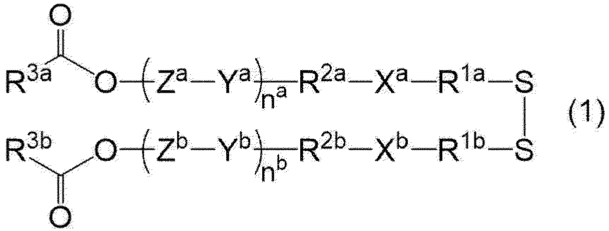 (in the formula (1),
R^{1a} and R^{1b} are each independently an alkylene group having 1 - 6 carbon atoms,
X^{a} and X^{b} are each independently a non-cyclic alkyl tertiary amino group having 1 - 6 carbon atoms and one tertiary amino group, or a cyclic alkylene tertiary amino group having 2 - 5 carbon atoms and 1 - 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 - 16 carbon atoms and at least one aromatic ring, and optionally having a hetero atom,
n^{a} and n^{b} are each independently 0 or 1, and
R^{3a} and R^{3b} are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 - 40 carbon atoms, an alkyl group having a cyclopropane ring and having 3 - 40 carbon atoms, or a group represented by the formula (3):
R⁹-O-CO-(CH₂)a- (3)
(in the formula (3),
R⁹ is an aliphatic hydrocarbon group having 2 - 20 carbon atoms, and
a is an integer of 2 to 10)).

7. The production method according to any one of claims 1 to 5, wherein the ionic lipid is a compound represented by the formula (2): 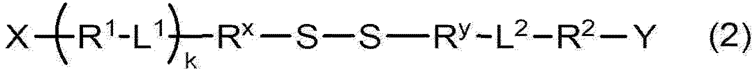 wherein
X is a nitrogen-containing aliphatic group containing one or more tertiary nitrogens,
R¹ is an aliphatic hydrocarbon group having not more than 8 carbon atoms,
L¹ is an ester bond, an amide bond, a carbamate bond, an N-alkylcarbamate bond, a carbonate bond or a urea bond,
k is 0 or 1,
R^{x} and R^{y} are each independently an alkylene group having 2 - 5 carbon atoms,
L² is an ester bond, an amide bond, a carbamate bond, a carbonate bond, an ether bond or a urea bond,
R² is an alkylene group having not more than 8 carbon atoms, or absent, and
Y is a group which (i) contains one or more divalent groups derived from an aromatic compound optionally having a hetero atom, (ii) contains a group containing at least one selected from the group consisting of an ester bond and a carbonate bond on the aromatic ring of the divalent group, and (iii) contains at least one selected from the group consisting of an aliphatic hydrocarbon group having 10 - 37 carbon atoms, a liposoluble vitamin residue, and a residue of a sterol derivative.

8. A method for introducing a nucleic acid into a cell, comprising a step of contacting nucleic acid-encapsulated lipid nanoparticles produced by the method according to any one of claims 1 to 7 with the cell in vitro.

9. A method for introducing a nucleic acid into a target cell, comprising a step of administering nucleic acid-encapsulated lipid nanoparticles produced by the method according to any one of claims 1 to 7 to a living organism.

10. A method for producing a pharmaceutical composition, comprising the method according to any one of claims 1 to 7.
